# EUROPEAN PATENT SPECIFICATION

(11) **EP 2 896 621 B1**
(45) Date of publication and mention of the grant of the patent: **25.04.2018**
(21) Application number: 13837441.8
(22) Date of filing: 10.09.2013
(51) Int. Cl.: C07D 471/04, C07D 519/00, H01L 51/50

(54) **NOVEL COMPOUND, MATERIAL FOR ORGANIC ELECTROLUMINESCENCE ELEMENT, ORGANIC ELECTROLUMINESCENCE ELEMENT, AND ELECTRONIC DEVICE**
NEUARTIGE VERBINDUNG, MATERIAL FÜR EIN ORGANISCHES ELEKTROLUMINESZENZELEMENT, ORGANISCHES ELEKTROLUMINESZENZELEMENT UND ELEKTRONISCHE VORRICHTUNG
NOUVEAU COMPOSÉ, MATÉRIAU POUR DISPOSITIF ÉLECTROLUMINESCENT ORGANIQUE, DISPOSITIF ÉLECTROLUMINESCENT ORGANIQUE ET DISPOSITIF ÉLECTRONIQUE

(30) Priority: 12.09.2012 JP 2012201028
(43) Date of publication of application: 22.07.2015
(73) Proprietor: Idemitsu Kosan Co., Ltd, Tokyo 100-8321 (JP)
(72) Inventor: MIZUTANI, Sayaka, Sodegaura-shi Chiba 299-0293 (JP); SADO, Takayasu, Sodegaura-shi Chiba 299-0293 (JP)
(74) Representative: Gille Hrabal
(86) International application number: PCT/JP2013/074431
(87) International publication number: WO 2014/042163

(56) References cited:
- WO-A1-2006/128800
- WO-A1-2010/064871
- WO-A1-2012/111462
- WO-A1-2012/115034
- WO-A2-2010/075411
- JP-A- 2004 107 263
- JP-A- 2007 077 094
- JP-A- 2008 222 624
- JP-A- 2010 034 548
- KR-A- 20120 072 785
- KR-A- 20120 096 383

## Description

### TECHNICAL FIELD

The present invention relates to a novel compound, a material for an organic electroluminescence device, an organic electroluminescence device, and an electronic device.

### BACKGROUND ART

An organic electroluminescence device (hereinafter, occasionally abbreviated as organic EL device) using an organic substance is highly expected to be used as an inexpensive solid-emitting full-color display device having a large area and has been variously developed.

In general, the organic EL device includes a pair of opposite electrodes and an emitting layer interposed between the pair of electrodes. When an electrical field is applied to the opposite electrodes of the organic EL device, electrons are injected from a cathode and holes are injected from an anode. The injected electrons and holes are recombined in the emitting layer to form excitons. Energy generated when the excitons in an excited state are returned to a ground state is irradiated as light. The organic EL device emits light in accordance with such a principle.

A typical organic EL device exhibits a higher drive voltage than an inorganic light-emitting diode. Moreover, since properties of the organic EL device are considerably deteriorated, the organic EL device is not in practical use. Although the organic EL device has been gradually improved in recent years, reduction of the voltage is further demanded.

Patent Literature 1 discloses an organic EL device containing a benzazole compound.

### CITATION LIST

### PATENT LITERATURES

Patent Literature 1: US Patent No. 5,645,948
Patent Literature 2: JP-A-2010-34548
Patent Literature 3: International Publication No. WO2006/128800
Further reference may be made to WO2012/111462 and KR2012/072785.

### SUMMARY OF THE INVENTION

### PROBLEMS TO BE SOLVED BY THE INVENTION

An organic EL device to be driven at a further lower voltage than the organic EL device containing a nitrogen-containing heterocyclic derivative of Patent Literature 1 is required.

An object of the invention is to provide a compound, a material for an organic electroluminescence device, and an organic electroluminescence device which are capable of decreasing a drive voltage. Another object of the invention is to provide an electronic device including the organic electroluminescence device.

### MEANS FOR SOLVING THE PROBLEMS

Our invention provides a compound as described in our claim 1, as well as some advantageous embodiments as described in claims 2 to 16.

Meanwhile, our invention provides a use of the compound in an organic compound layer of an organic electroluminescence device, as described in claim 17.

Our invention provides a use of the compound in an electron transporting layer of an organic electroluminescence device, as described in claim 18.

Our invention provides a material for an organic electroluminescence device, as described in claim 19.

Besides, our invention further provides an organic electroluminescence device according to claim 20 as well as its dependent claims 21 to 37.

At last but not least, our invention provides an electronic device as described in claim 38.

According to the above aspects of the invention, the compound and the organic electroluminescence device which are capable of decreasing the drive voltage can be provided.

### DESCRIPTION OF EMBODIMENT(S)

An exemplary embodiment of the invention will be described in detail below.

### Compound

A compound according to the exemplary embodiment is represented by a formula (1) below.

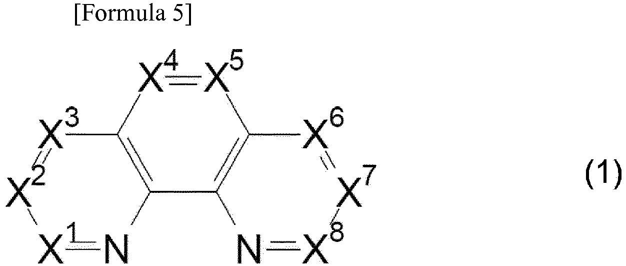

In the formula (1), X¹ to X⁸ each independently represent a carbon atom to be bonded to a group represented by a formula (2) below, CR^{X} or a nitrogen atom. In the formula (1), at least one of X¹ to X⁸ is a carbon atom to be bonded to the group represented by the formula (2). In the formula (1), R^{X} is each independently selected from the group consisting of a hydrogen atom, halogen atom, cyano group, nitro group, substituted or unsubstituted hydroxyl group, substituted or unsubstituted carboxyl group, substituted or unsubstituted sulfonyl group, substituted or unsubstituted boryl group, substituted or unsubstituted phosphino group, substituted or unsubstituted mercapto group, substituted or unsubstituted acyl group, substituted or unsubstituted amino group, substituted or unsubstituted silyl group, substituted or unsubstituted alkyl group having 1 to 30 carbon atoms, substituted or unsubstituted alkenyl group having 2 to 30 carbon atoms, substituted or unsubstituted alkynyl group having 2 to 30 carbon atoms, substituted or unsubstituted aralkyl group having 6 to 30 carbon atoms, substituted or unsubstituted aryl group having 6 to 40 ring carbon atoms, and substituted or unsubstituted heteroaryl group having 5 to 40 ring atoms. In the formula (1), R^{X} of adjacent ones of CR^{X} in X¹ to X⁸ may be bonded to each other to form a cyclic structure or may not be bonded. For instance, R^{X} of CR^{X} in X¹ may be bonded to R^{X} of CR^{X} in X² adjacent to X¹ to form a saturated or unsaturated cyclic structure.

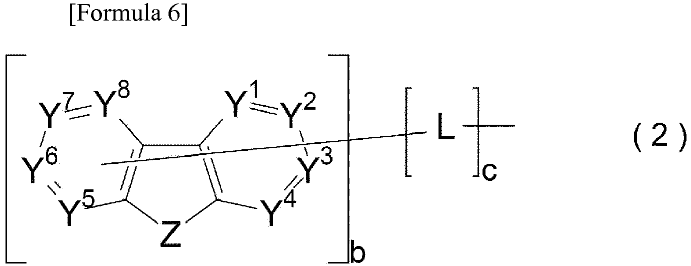

In the formula (2), b is an integer of 1 to 5, preferably 1 to 3, further preferably 1 to 2.

In the formula (2), c is an integer of 1 to 8, preferably 1 to 5, further preferably 1 to 3.

In the formula (2), Z represents an oxygen atom, sulfur atom or silicon atom. In the formula (2), when b is an integer of 2 to 5, a plurality of Z are mutually the same or different. In the formula (2), when Z is a silicon atom, R⁹ and R¹⁰ are bonded to the silicon atom. R⁹ and R¹⁰ each independently represent the same as R^{X} in the formula (1). R⁹ and R¹⁰ may be bonded to the structure represented by the formula (1). However, in the formula (2), when Z is a silicon atom, R⁹ and R¹⁰ do not form a cyclic structure by mutual bonding.

In the formula (2), L is selected from a single bond and a linking group. The linking group represents a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms. The polyvalent heteroaryl group having 5 to 40 ring atoms in L of the formula (2) includes a substituted or unsubstituted polyvalent group derived from a phenanthroline ring represented by the formula (1). "Polyvalent" means having valence of 2 or more. When c is an integer of 2 to 8 in the formula (2), a plurality of L are mutually the same or different. L is preferably a single bond or phenylene, more preferably phenylene rather than a single bond.

In the formula (2), Y¹ to Y⁸ each independently represent a nitrogen atom, CR^{Y} or a carbon atom to be bonded to L. Y⁴ and Y⁵ are preferably carbon atoms to be bonded to L. Alternatively, Y² and Y⁷ are preferably carbon atoms to be bonded to L.

R^{Y} represents the same as R^{X} in the formula (1). The heteroaryl group having 5 to 40 ring atoms in R^{Y} includes a substituted or unsubstituted phenanthrolyl group derived from the phenanthroline ring represented by the formula (1). In the formula (2), adjacent ones of R^{Y} may be bonded to each other to form a cyclic structure or may not be bonded.

The formula (2) in which a substituted or unsubstituted polyvalent group derived from the phenanthroline ring represented by the formula (1) is included in L is exemplarily represented by a formula (2-1) below. In the formula (2-1), A1 represents the structure represented by the formula (1).

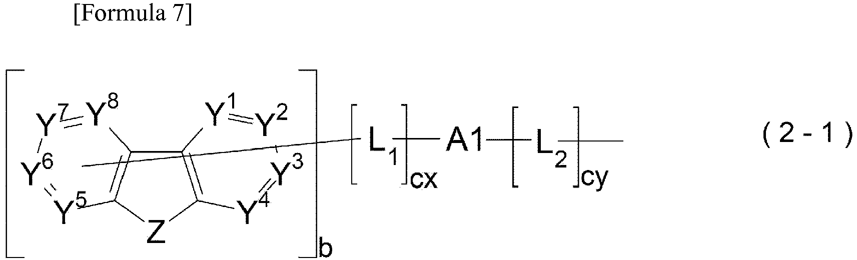

In the formula (2-1), cx is an integer of 0 to 7, cy is an integer of 0 to 7 and 0≤cx+cy≤7. In the formula (2-1), L₁ and L₂ each independently represent the same as L in the formula (2). In the formulae (2-1), X¹ to X⁸ of Al, Y¹ to Y⁸, Z and b respectively represent the same as X¹ to X⁸, Y¹ to Y⁸, Z and b in the formulae (1) and (2).

The formula (2) in which R^{Y} of CR^{Y} is a substituted or unsubstituted phenanthrolyl group derived from the phenanthroline ring represented by the formula (1) is exemplarily represented by a formula (1-1) below. In the formula (1-1), A2 and A3 each independently represent the structure represented by the formula (1).

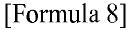

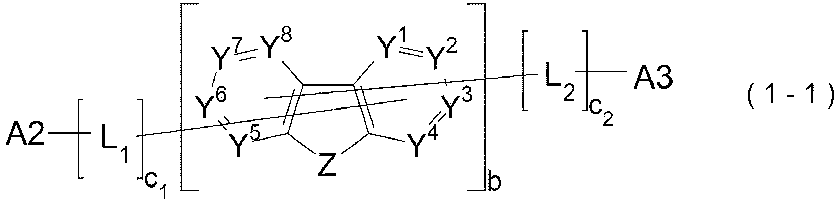

In the formula (1-1), c₁ is an integer of 1 to 8 and c₂ is an integer of 1 to 8. In the formula (1-1), L₁ and L₂ each independently represent the same as L in the formula (2). In the formula (1-1), X¹ to X⁸ in A2 and A3, Y¹ to Y⁸, Z and b respectively represent the same as X¹ to X⁸, Y¹ to Y⁸, Z and b in the formulae (1) and (2).

The formula (2) in which a substituted or unsubstituted polyvalent group derived from the phenanthroline ring represented by the formula (1) is included in L and in which R^{Y} of CR^{Y} is the substituted or unsubstituted phenanthrolyl group derived from the phenanthroline ring represented by the formula (1) is exemplarily represented by a formula (1-2) below. In the formula (1-2), A1, A2 and A3 each independently represent the structure represented by the formula (1).

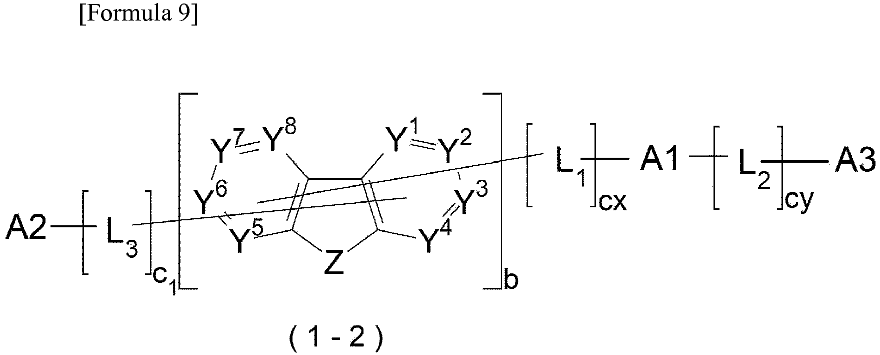

In the formula (1-2), X¹ to X⁸ in A1, A2 and A3, Y¹ to Y⁸, Z, L₁ to L₃, b, cx, cy and c₁ respectively represent the same as X¹ to X⁸, Y¹ to Y⁸, Z L₁ to L₃, b, cx, cy and c₁ in the formulae (1), (2), (1-1) and (2-1).

The formula (1) in which two or more of X¹ to X⁸ are carbon atoms to be bonded to the group represented by the formula (2) is exemplarily represented by a formula (1-3) below, in which two of X¹ to X⁸ are carbon atoms to be bonded to the group represented by the formula (2). In the formula (1-3), A1 represents the structure represented by the formula (1).

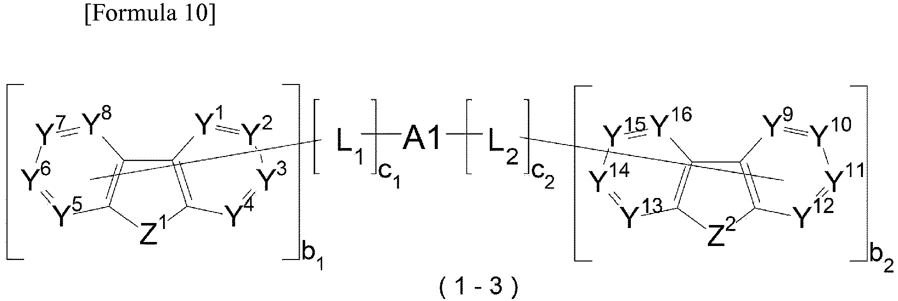

In the formula (1-3), Y⁹ to Y¹⁶ respectively independently represent the same as Y¹ to Y⁸ in the formula (2). In the formula (1-3), L₁ and L₂ each independently represent the same as L in the formula (2). In the formula (1-3), b₁ and b₂ are each independently an integer of 1 to 5 and c₁ and c₂ are each independently an integer of 1 to 8. In the formula (1-3), Z¹ and Z² each independently represent the same as Z in the formula (2). In the formula (1-3), X¹ to X⁸ in A1 and Y¹ to Y⁸ respectively represent the same as X¹ to X⁸ and Y¹ to Y⁸ in the formulae (1) and (2).

When X¹ or X⁸ of the formula (1) is a carbon atom to be bonded to the group represented by the formula (2) in which b is 1, Z is an oxygen atom, Y⁴ or Y⁵ is a carbon atom to be bonded to L, and c is 2, one of two L, which is near the phenanthroline ring represented by the formula (1), is a divalent group other than an anthracene group. Specifically, the compound represented by the formula (1) is represented by a formula (1-x). In the formula (1-x), provided that the two L are defined as L₁ and L₂, X¹ or X⁸ is a carbon atom bonded to L₂ while Y⁴ or Y⁵ is a carbon atom to be bonded to L₁. L₂ near the phenanthroline ring is selected from a single bond and a linking group. The linking group represents a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms (except for a divalent anthracene group), or a substituted or unsubstituted polyvalent heteroaryl group having 5 to 40 ring atoms. In the formula (1-x), L₁ represents the same as L of the formula (2). In the formula (1-x), X¹ to X⁸ and Y¹ to Y⁸ respectively represent the same as X¹ to X⁸ and Y¹ to Y⁸ in the formulae (1) and (2).

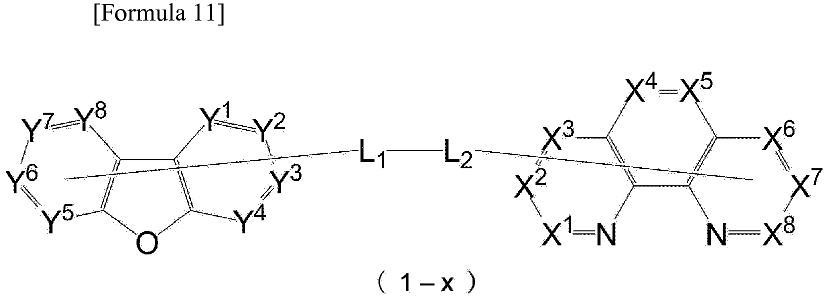

In the formula (1), when two of X¹ to X⁸ are carbon atoms to be bonded to the group represented by the formula (2) in which b and c are 1, Z is a sulfur atom, Y⁴ or Y⁵ is a carbon atom to be bonded to L, and L is a p-phenylene group, L is bonded to one of X¹, X², X⁴, X⁵, X⁷ and X⁸.

In other words, when the compound represented by the formula (1) is represented by a formula (1-4) below, A1 represents the structure represented by the formula (1) in the formula (1-4). In the formula (1-4), X¹, X², X⁴, X⁵, X⁷ and X⁸ in A1 and Y¹ to Y⁸ respectively represent the same as X¹, X², X⁴, X⁵, X⁷, X⁸ and Y¹ to Y⁸ in the formulae (1) and (2). In the formula (1-4), Y⁹ to Y¹⁶ each independently represent a nitrogen atom, CR^{Y} or a carbon atom to be bonded to L. In the formula (1-4), R^{Y} represents the same as R^{Y} of the formula (2). In the formula (1-4), when Y⁴ or Y⁵ is a carbon atom to be bonded to a p-phenylene group as L and Y¹² or Y¹³ is a carbon atom to be bonded to a p-phenylene group as the other L, X³ and X⁶ in A1 are each a nitrogen atom or CR^{X}.

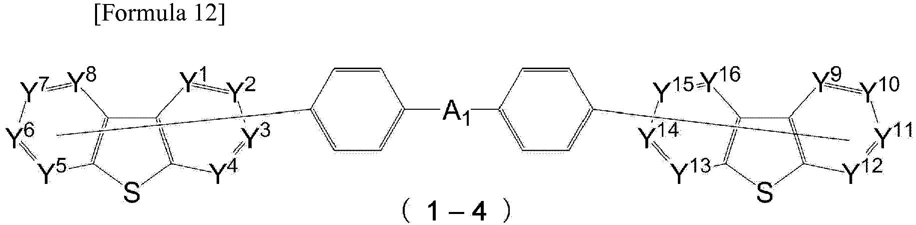

In the formula (1), when X¹ or X⁸ is a carbon atom to be bonded to the group represented by the formula (2) in which b and c are 1, Z is an oxygen atom or sulfur atom, Y³ is a carbon atom to be bonded to L, and L is a p-phenylene group, R^{Y} in Y⁴ is a group other than a phenyl group.

Alternatively, in the formula (1), when X¹ or X⁸ is a carbon atom to be bonded to the group represented by the formula (2) in which b and c are 1, Z is an oxygen atom or sulfur atom, Y⁶ is a carbon atom to be bonded to L, and L is a p-phenylene group, R^{Y} in Y⁵ is a group other than a phenyl group.

In other words, when the compound represented by the formula (1) is represented by a formula (1-5-1) below, in the formula (2), Y³ is a carbon atom to be bonded to a p-phenylene group as L, and Y⁴ is CR^{Y}. R^{Y} represents the same as R^{Y} described in the formula (2). It should be noted that R^{Y} is a group other than a phenyl group. In the formula (1-5-1), Z is an oxygen atom or a sulfur atom. In the formula (1-5-1), Y¹ to Y², Y³ to Y⁸ and X¹ to X⁸ respectively represent the same as Y¹ to Y², Y³ to Y⁸ and X¹ to X⁸ in the formulae (1) and (2).

Alternatively, when the compound represented by the formula (1) is represented by a formula (1-5-2) below, in the formula (2), Y⁶ is a carbon atom to be bonded to a p-phenylene group as L, Y⁵ is CR^{Y}. R^{Y} represents the same as R^{Y} described in the formula (2). It should be noted that R^{Y} is a group other than a phenyl group. In the formula (1-5-2), Z is an oxygen atom or a sulfur atom. In the formula (1-5-2), Y¹ to Y⁴, Y⁷ to Y⁸ and X¹ to X⁸ respectively represent the same as Y¹ to Y⁴, Y⁷ to Y⁸ and X¹ to X⁸ in the formulae (1) and (2).

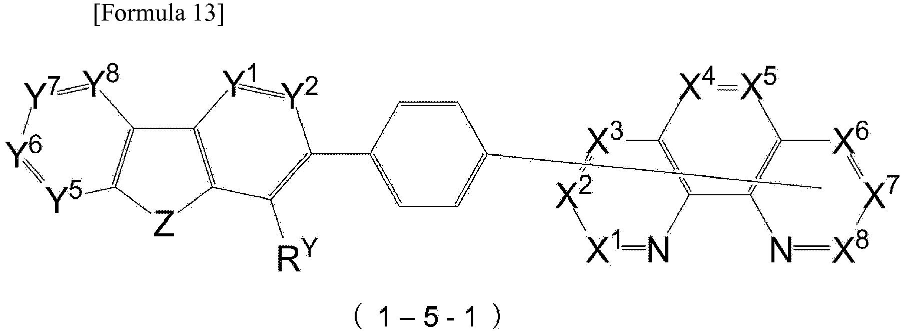

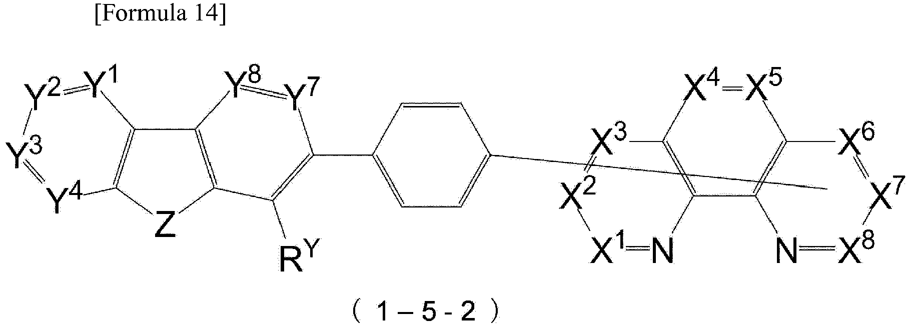

When X¹ or X⁸ of the formula (1) is a carbon atom to be bonded to the group represented by the formula (2) in which Z is a silicon atom, Y³ is a carbon atom to be bonded to L, L is a single bond, and R^{Y} of Y⁶ is bonded by a single bond to the phenanthrolyl group (the heteroaryl group having 5 to 40 ring atoms), R^{Y} of Y⁶ is bonded to the phenanthrolyl group at a position thereof other than a 2-position.

In other words, when the compound represented by the formula (1) is represented by a formula (1-6) below, in the formula (2), Y³ is a carbon atom to be bonded to X¹ or X⁸, Y⁶ is CR^{Y} in which R^{Y} is the phenanthrolyl group (the heteroaryl group having 5 to 40 ring atoms) which is shown on the left side of the formula (1-6) and one of X¹⁰ to X¹⁵ of the phenanthrolyl group is bonded to a carbon atom (C) of Y⁶. In the formula (1-6), the rest of X¹ to X¹⁶ except for the carbon atom to be bonded to the group represented by the formula (2) are each independently CR^{X} or a nitrogen atom. R^{X} represents the same as R^{X} in the formula (1). In the formula (1-6), Y¹, Y², Y⁴, Y⁵, Y⁷, Y⁸, R⁹ and R¹⁰ independently represent the same as Y¹, Y², Y⁴, Y⁵, Y⁷, Y⁸, R⁹ and R¹⁰ in the formula (2).

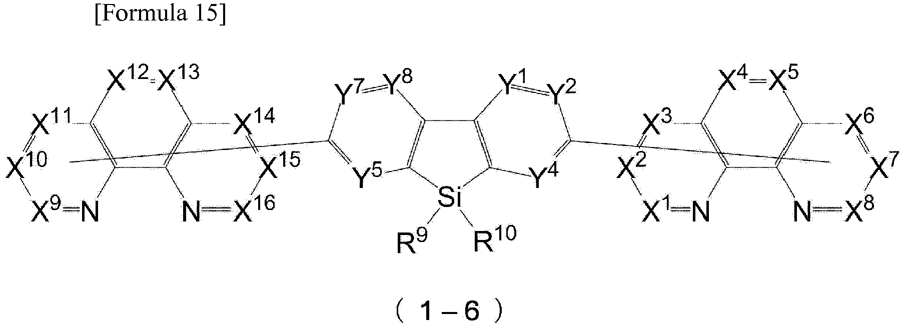

In the formula (1), when X⁴ or X⁵ is a carbon atom to be bonded to the group represented by the formula (2) in which Y² is a carbon atom to be bonded to L, L is a single bond and Z is an oxygen atom, R^{Y} in Y⁷ is a group other than a pyrenyl group.

Alternatively, in the formula (1), when X⁴ or X⁵ is a carbon atom to be bonded to the group represented by the formula (2) in which Y⁷ is a carbon atom to be bonded to L, and L is a single bond and Z is an oxygen atom, R^{Y} in Y² is a group other than a pyrenyl group.

In other words, when the compound represented by the formula (1) is represented by a formula (1-xx-1) below, in the formula (2), Y² is a carbon atom to be bonded to X¹ or X⁸, Y⁷ is CR^{Y}. R^{Y} represents the same as R^{Y} described in the formula (2). It should be noted that R^{Y} is a group other than a pyrenyl group. In the formula (1-xx-1), Y¹, Y⁴ to Y⁶, Y⁸ and X¹ to X⁸ respectively represent the same as Y¹, Y⁴ to Y⁶, Y⁸ and X¹ to X⁸ in the formulae (1) and (2).

Alternatively, when the compound represented by the formula (1) is represented by a formula (1-xx-2) below, in the formula (2), Y⁷ is a carbon atom to be bonded to X¹ or X⁸, Y² is CR^{Y}. R^{Y} represents the same as R^{Y} described in the formula (2). It should be noted that R^{Y} is a group other than a pyrenyl group. In the formula (1-xx-2), Y¹, Y³ to Y⁴, Y⁵, Y⁸ and X¹ to X⁸ respectively represent the same as Y¹, Y³ to Y⁴, Y⁵, Y⁸ and X¹ to X⁸ in the formulae (1) and (2).

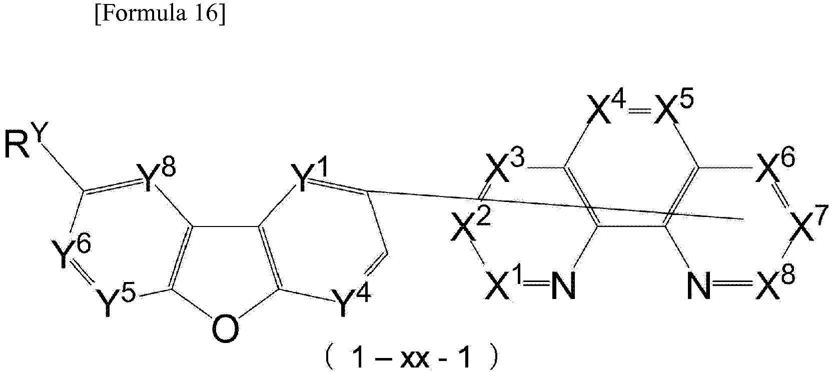

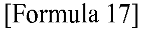

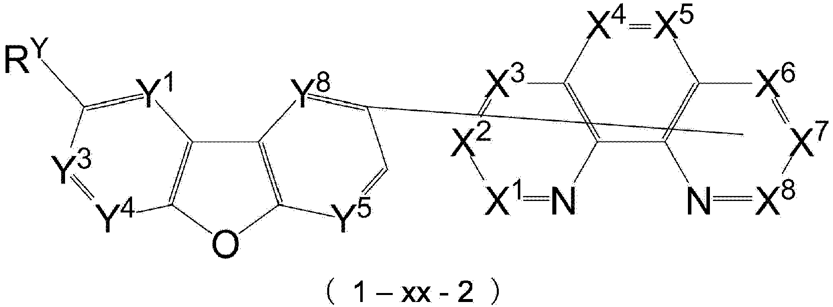

In the compound according to the exemplary embodiment, X¹ or X⁸ in the formula (1) is preferably a carbon atom to be bonded to the group represented by the formula (2). Specifically, the compound according to the exemplary embodiment is preferably a compound represented by a formula (1-7) below.

In the formula (1-7), Y¹ to Y⁸, Z, L, b and c respectively represent the same as Y¹ to Y⁸, Z, L, b and c in the formula (2). In the formula (1-7), X² to X⁸ each independently represent CR^{X} or a nitrogen atom in which R^{X} represents the same as R^{X} in the formula (1).

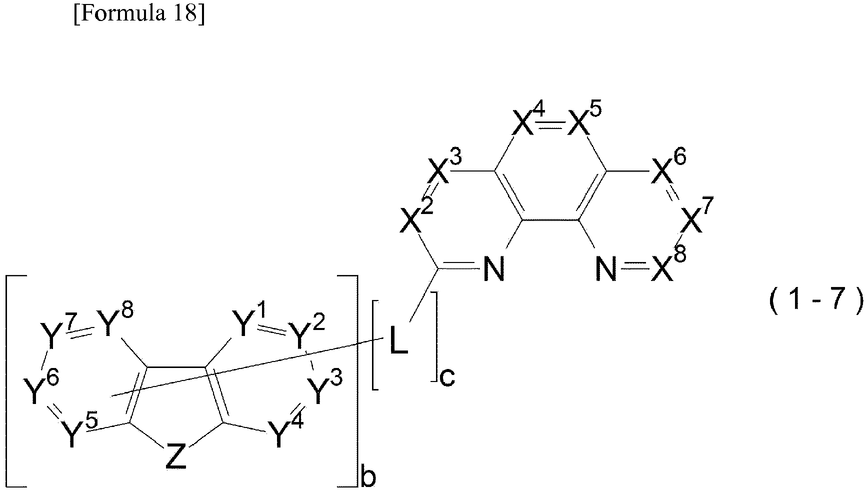

Unlike in the compound represented by the formula (1-7), X⁸ instead of X¹ may be a carbon atom to be bonded to the group represented by the formula (2).

In the compound according to the exemplary embodiment, X¹ and X⁸ in the formula (1) are preferably carbon atoms to be bonded to the group represented by the formula (2). Specifically, the compound according to the exemplary embodiment is preferably a compound represented by a formula (1-8) below.

In the formula (1-8), X² to X⁷ each independently represent CR^{X} or a nitrogen atom in which R^{X} represents the same as R^{X} in the formula (1). In the formula (1-8), Y¹ to Y¹⁶ each independently represent a nitrogen atom, CR^{Y} or a carbon atom to be bonded to L, in which R^{Y} represents the same as R^{Y} in the formula (2). In the formula (1-8), Z¹ and Z² each independently represent the same as Z in the formula (2). In the formula (1-8), L₁ and L₂ each independently represent the same as L in the formula (2). In the formula (1-8), b₁ and b₂ are each independently an integer of 1 to 5 and c₁ and c₂ are each independently an integer of 1 to 8.

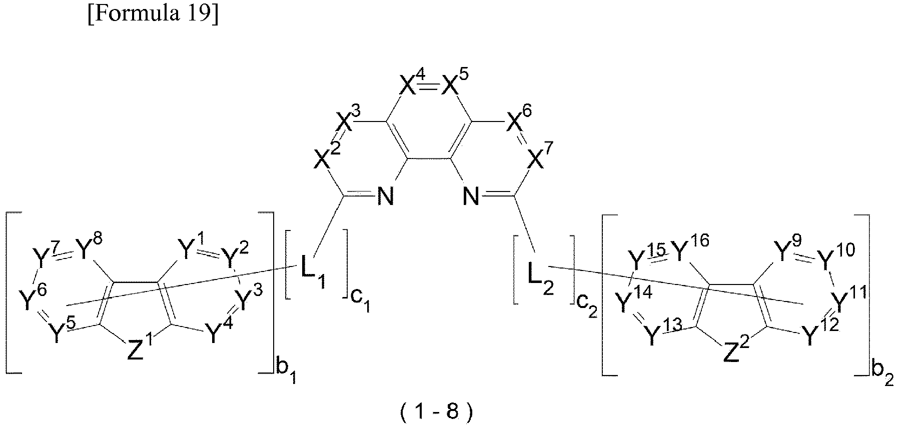

In the compound according to the exemplary embodiment, X³ and X⁶ in the formula (1) are preferably carbon atoms to be bonded to the group represented by the formula (2). Specifically, the compound according to the exemplary embodiment is preferably a compound represented by a formula (1-9) below.

In the formula (1-9), X¹, X², X⁴, X⁵, X⁷ and X⁸ each independently represent CR^{X} or a nitrogen atom in which R^{X} represents the same as R^{X} in the formula (1). In the formula (1-9), Y¹ to Y¹⁶ , Z¹, Z², L₁, L₂, b₁, b₂, c₁ and c₂ respectively represent the same as Y¹ to Y¹⁶, Z¹, Z², L₁, L₂, b₁, b₂, c₁ and C₂ in the formula (1-8).

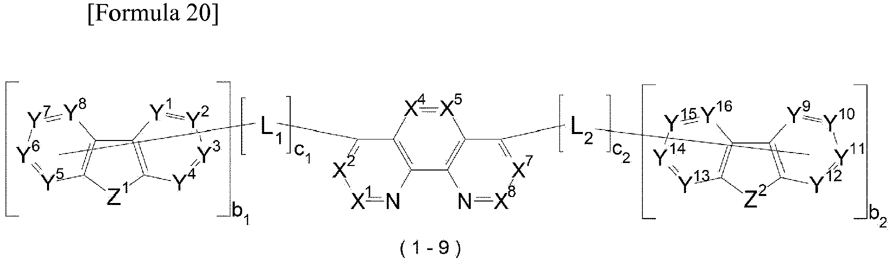

In the compound according to the exemplary embodiment, X² or X⁷ in the formula (1) is preferably a carbon atom to be bonded to the group represented by the formula (2). Specifically, the compound according to the exemplary embodiment is preferably a compound represented by a formula (1-10) below.

In the formula (1-10), X¹ and X³ to X⁸ each independently represent CR^{X} or a nitrogen atom in which R^{X} represents the same as R^{X} in the formula (1). In the formula (1-10), Y¹ to Y⁸, Z, L, b and c respectively represent the same as Y¹ to Y⁸, Z, L, b and c in the formula (2).

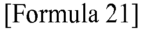

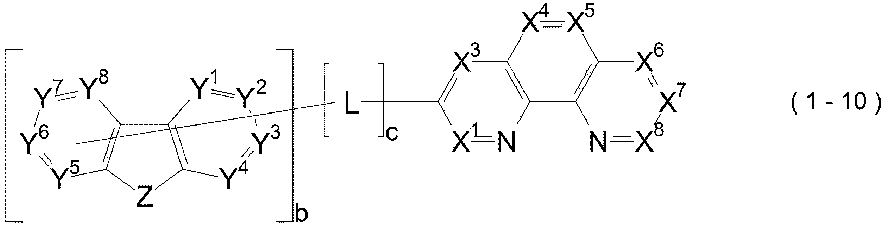

Unlike in the compound represented by the formula (1-10), X⁷ instead of X² may be a carbon atom to be bonded to the group represented by the formula (2).

In the compound according to the exemplary embodiment, X³ or X⁶ in the formula (1) is preferably a carbon atom to be bonded to the group represented by the formula (2). Specifically, the compound according to the exemplary embodiment is preferably a compound represented by a formula (1-11) below.

In the formula (1-11), X¹, X² and X⁴ to X⁸ each independently represent CR^{X} or a nitrogen atom in which R^{X} represents the same as R^{X} in the formula (1). In the formula (1-11), Y¹ to Y⁸, Z, L, b and c respectively represent the same as Y¹ to Y⁸, Z, L, b and c in the formula (2).

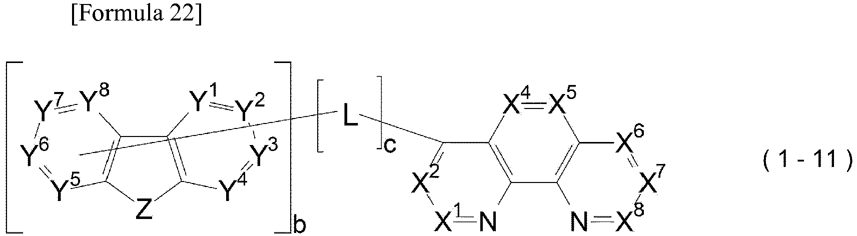

Unlike in the compound represented by the formula (1-11), X⁶ instead of X³ may be a carbon atom to be bonded to the group represented by the formula (2).

In the compound according to the exemplary embodiment, X⁴ or X⁵ in the formula (1) is preferably a carbon atom to be bonded to the group represented by the formula (2). Specifically, the compound according to the exemplary embodiment is preferably a compound represented by a formula (1-12) below.

In the formula (1-12), X¹ to X³ and X⁵ to X⁸ each independently represent CR^{X} or a nitrogen atom in which R^{X} represents the same as R^{X} in the formula (1). In the formula (1-12), Y¹ to Y⁸, Z, L, b and c respectively represent the same as Y¹ to Y⁸, Z, L, b and c in the formula (2).

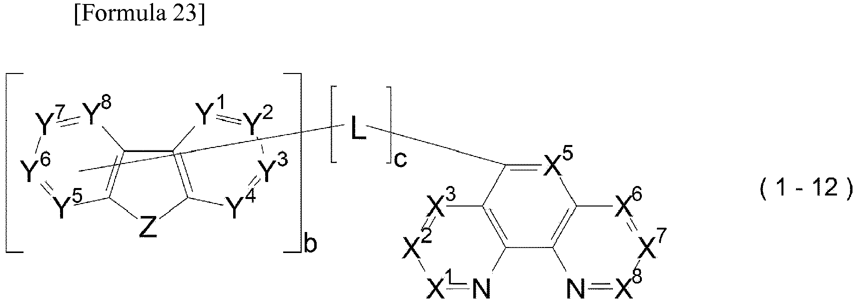

Unlike in the compound represented by the formula (1-12), X⁵ instead of X⁴ may be a carbon atom to be bonded to the group represented by the formula (2).

In the compound according to the exemplary embodiment, X¹ and X⁷ in the formula (1) are preferably carbon atoms to be bonded to the group represented by the formula (2). Specifically, the compound according to the exemplary embodiment is preferably a compound represented by a formula (1-13) below.

In the formula (1-13), X² to X⁶ and X⁸ each independently represent CR^{X} or a nitrogen atom in which R^{X} represents the same as R^{X} in the formula (1). In the formula (1-13), Y¹ to Y¹⁶, Z¹, Z², L₁, L₂, b₁, b₂, c₁ and c₂ respectively represent the same as Y¹ to Y¹⁶, Z¹, Z², L₁, L₂, b₁, b₂, c₁ and c₂ in the formula (1-8).

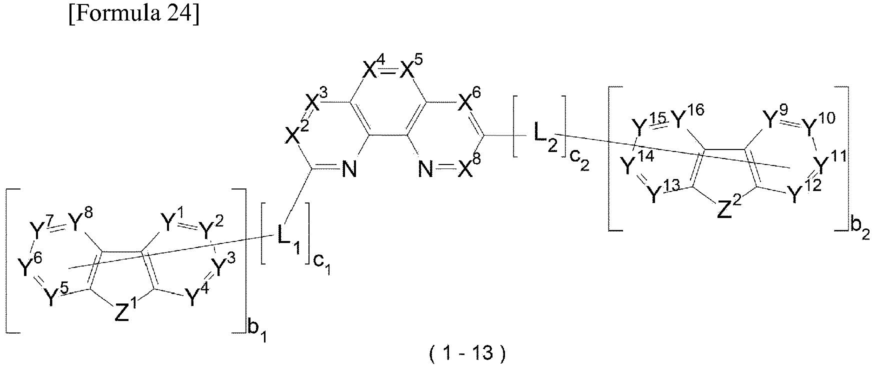

In the compound according to the exemplary embodiment, X² and X⁷ in the formula (1) are preferably carbon atoms to be bonded to the group represented by the formula (2). Specifically, the compound according to the exemplary embodiment is preferably a compound represented by a formula (1-14) below.

In the formula (1-14), X¹, X³ to X⁶ and X⁸ each independently represent CR^{X} or a nitrogen atom in which R^{X} represents the same as R^{X} in the formula (1). In the formula (1-14), Y¹ to Y¹⁶, Z¹, Z², L₁, L₂, b₁, b₂, c₁ and c₂ respectively represent the same as Y¹ to Y¹⁶, Z¹, Z², L₁, L₂, b₁, b₂, c₁ and c₂ in the formula (1-8).

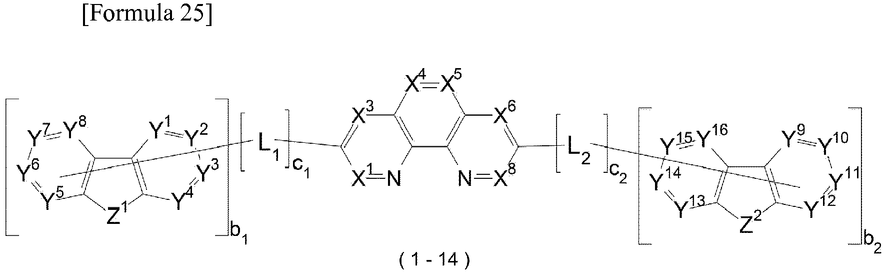

In the compound according to the exemplary embodiment, X¹, X², X⁷ and X⁸ in the formula (1) are preferably carbon atoms to be bonded to the group represented by the formula (2). Specifically, the compound according to the exemplary embodiment is preferably a compound represented by a formula (1-15) below.

In the formula (1-15), X³ to X⁶ each independently represent CR^{X} or a nitrogen atom in which R^{X} represents the same as R^{X} in the formula (1). In the formula (1-15), Y¹ to Y³² each independently represent a nitrogen atom, CR^{Y} or a carbon atom to be bonded to L, in which R^{Y} represents the same as R^{Y} in the formula (2). In the formula (1-15), Z¹ to Z⁴ each independently represent the same as Z in the formula (2). In the formula (1-15), L₁ to L₄ each independently represent the same as L in the formula (2). In the formula (1-15), b₁ to b₄ are each independently an integer of 1 to 5 and c₁ to c₂ are each independently an integer of 1 to 8.

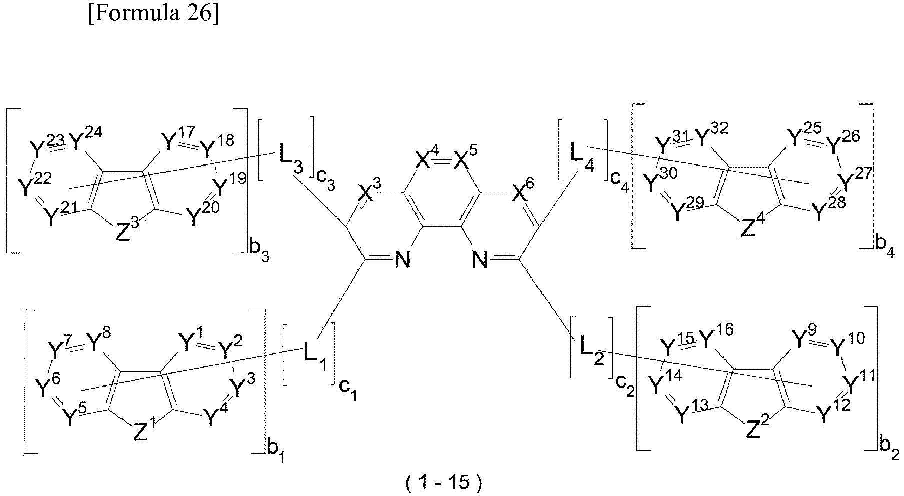

In the compound according to the exemplary embodiment, X¹, X³, X⁶ and X⁸ in the formula (1) are preferably carbon atoms to be bonded to the group represented by the formula (2). Specifically, the compound according to the exemplary embodiment is preferably a compound represented by a formula (1-16) below.

In the formula (1-16), X², X⁴, X⁵ and X⁷ each independently represent CR^{X} or a nitrogen atom in which R^{X} represents the same as R^{X} in the formula (1). In the formula (1-16), Y¹ to Y³² each independently represent a nitrogen atom, CR^{Y} or a carbon atom to be bonded to L, in which R^{Y} represents the same as R^{Y} in the formula (2). In the formula (1-16), Z¹ to Z⁴ each independently represent the same as Z in the formula (2). In the formula (1-16), L₁ to L₄ each independently represent the same as L in the formula (2). In the formula (1-16), b₁ to b₄ are each independently an integer of 1 to 5 and c₁ to c₂ are each independently an integer of 1 to 8.

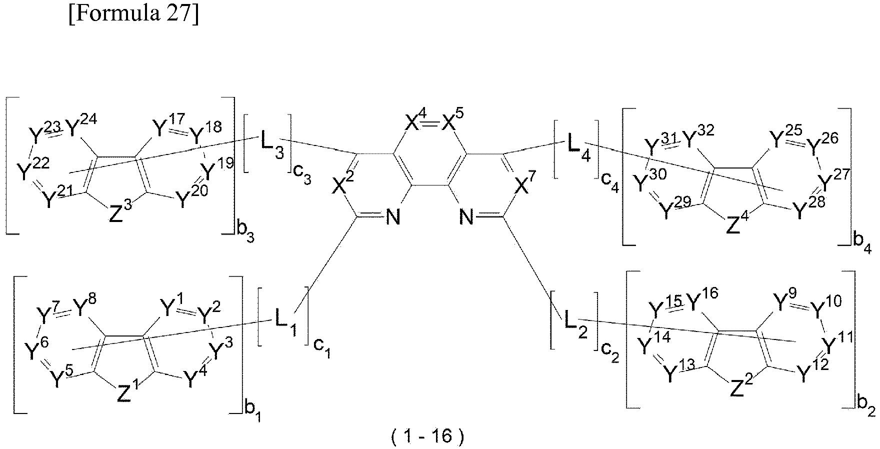

In the compound according to the exemplary embodiment, the formula (2) is preferably represented by a formula (2-2). At least one of X¹ to X⁸ is preferably a carbon atom to be bonded to the group represented by the formula (2-2).

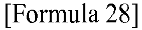

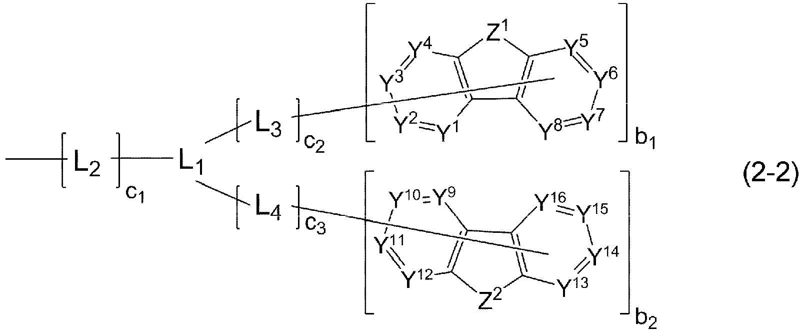

In the formula (2-2), b₁ and b₂ are each independently an integer of 1 to 5.

c₁, c₂ and c₃ are each independently an integer of 0 to 7 and c₁+c₂+c₃ is an integer of 7 or less.

Z represents an oxygen atom, sulfur atom or silicon atom. When b is from 2 to 5, a plurality of Z are mutually the same or different. When Z is a silicon atom, R⁹ and R¹⁰ are bonded to the silicon atom. R⁹ and R¹⁰ each independently represent the same as R^{X} in the formula (10). R⁹ and R¹⁰ may be bonded to the structure represented by the formula (10).

L₁ represents a linking group. The linking group is a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms.

L₂, L₃ and L₄ are each independently selected from a single bond and a linking group. The linking group is a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms.

The polyvalent heteroaryl group having 5 to 40 ring atoms in L₁ to L₄ of the formula (2-2) also includes a substituted or unsubstituted polyvalent group derived from a phenanthroline ring represented by the formula (10). When c₁ is an integer of 2 or more, a plurality of L₂ are mutually the same or different. When c₂ is an integer of 2 or more, a plurality of L₃ are mutually the same or different. When c₃ is an integer of 2 or more, a plurality of L₄ are mutually the same or different.

Y¹ to Y⁸ each independently represent a nitrogen atom, CR^{Y} or a carbon atom to be bonded to L₃.

Y⁹ to Y¹⁶ each independently represent a nitrogen atom, CR^{Z} or a carbon atom to be bonded to L₄.

R^{Y} and R^{Z} each independently represent the same as R^{X} in the formula (10). The heteroaryl group having 5 to 40 ring atoms in R^{Y} and R^{Z} includes a substituted or unsubstituted phenanthrolyl group derived from the phenanthroline ring represented by the formula (10). Adjacent ones of R^{Y} may be bonded to each other to form a cyclic structure or may not be bonded.

In the exemplary embodiment, X¹ or X⁸ is preferably a carbon atom to be bonded to the group represented by the formula (2-2).

In the exemplary embodiment, L₁ in the formula (2-2) is preferably a substituted or unsubstituted polyvalent aryl group having 6 to 40 ring carbon atoms, more preferably a substituted or unsubstituted polyvalent aryl group having 6 ring carbon atoms.

In the exemplary embodiment, L₂ in the formula (2-2) is preferably a substituted or unsubstituted polyvalent heteroaryl group having 5 to 40 ring atoms. In the the formula (2-2), L₂ directly bonded to L₁ is preferably a substituted or unsubstituted polyvalent heteroaryl group having 5 to 40 ring atoms.

In the exemplary embodiment, it is preferable that, in the formula (2-2), c₁ is 2 or more and a plurality of L₂ include a substituted or unsubstituted polyvalent heteroaryl group having 5 to 40 ring atoms and a substituted or unsubstituted polyvalent aryl group having 6 to 40 ring carbon atoms. In this arrangement, in the formula (2-2), L₂ directly bonded to L₁ is preferably a substituted or unsubstituted polyvalent heteroaryl group having 5 to 40 ring atoms.

In the exemplary embodiment, L₃ and L₄ in the formula (2-2) are preferably each independently a single bond or a substituted or unsubstituted polyvalent aryl group having 6 to 40 ring carbon atoms, more preferably a substituted or unsubstituted polyvalent aryl group having 6 ring carbon atoms.

In the exemplary embodiment, c₂ and c₃ are each preferably 1. In this arrangement, L₃ and L₄ are each preferably a substituted or unsubstituted polyvalent aryl group having 6 to 40 ring carbon atoms, more preferably a single bond or a substituted or unsubstituted polyvalent aryl group having 6 ring carbon atoms.

In the compounds represented by the formulae (1-7) to (1-16), the compounds represented by the formulae (1-7) and (1-8) are preferable.

Moreover, the compound represented by the formula (1-1) is preferable.

In the compound according to the exemplary embodiment, Z and Z¹ to Z⁴ in the formulae (2), (2-1), (1-1) to (1-3), (1-5), (1-7) to (1-16) are each preferably an oxygen atom or a sulfur atom, more preferably an oxygen atom.

In the formulae (1), (1-1) to (1-16), (2-1), (1-x), (1-xx-1) and (1-xx-2), the rest of X¹ to X¹⁶ except for the carbon atom to be bonded to the group represented by the formula (2) are preferably CR^{X}, in which R^{X} is preferably a hydrogen atom, alkyl group or aryl group, further preferably a hydrogen atom or a phenyl group.

Next, each of the substituents described in the formulae (1), (1-1) to (1-16), (2), (2-1), (1-x), (1-xx-1) and (1-xx-2) will be described below. Examples of the substituents described in the formulae (1), (1-1) to (1-16), (2), (2-1), (1-x), (1-xx-1) and (1-xx-2) include a hydrogen atom, halogen atom, cyano group, nitro group, substituted or unsubstituted hydroxyl group, substituted or unsubstituted carboxyl group, substituted or unsubstituted sulfonyl group, substituted or unsubstituted boryl group, substituted or unsubstituted phosphino group, substituted or unsubstituted mercapto group, substituted or unsubstituted acyl group, substituted or unsubstituted amino group, substituted or unsubstituted silyl group, substituted or unsubstituted alkyl group having 1 to 30 carbon atoms, substituted or unsubstituted alkenyl group having 2 to 30 carbon atoms, substituted or unsubstituted alkynyl group having 2 to 30 carbon atoms, substituted or unsubstituted aralkyl group having 6 to 30 carbon atoms, substituted or unsubstituted aryl group having 6 to 40 ring carbon atoms, and substituted or unsubstituted heteroaryl group having 5 to 40 ring atoms.

Examples of the halogen atom in the formulae (1), (1-1) to (1-16), (2), (2-1), (1-x), (1-xx-1) and (1-xx-2) include fluorine, chlorine, bromine, and iodine, among which a fluorine atom is preferable.

Examples of the substituted or unsubstituted hydroxyl group in the formulae (1), (1-1) to (1-16), (2), (2-1), (1-x), (1-xx-1) and (1-xx-2) include a hydroxyl group (-OH) and a group represented by -OR^{A} that is obtained by substituting H of a hydroxyl group (-OH) with R^{A}.

Herein, when R^{A} is an alkyl group, the group represented by -OR^{A} is an alkoxy group, preferably a substituted or unsubstituted alkoxy group having 1 to 30 carbon atoms. The alkyl group as R^{A} is preferably an alkyl group having 1 to 30 carbon atoms below. Examples of the alkoxy group include a methoxy group, ethoxy group, propoxy group, butoxy group, pentyloxy group and hexyloxy group. Among the alkoxy group, an alkoxy group having 1 to 10 carbon atoms is preferable and an alkoxy group having 1 to 8 carbon atoms is more preferable. An alkoxy group having 1 to 4 carbon atoms is particularly preferable.

Here, examples of the substituted or unsubstituted alkoxy group include a haloalkoxy group that is obtained by substituting an alkyl group (R^{A}) with one or more halogen atoms described above.

Moreover, when R^{A} is an aryl group, the group represented by -OR^{A} is an aryloxy group, preferably a substituted or unsubstituted aryloxy group having 6 to 40 ring carbon atoms. The aryl group as R^{A} is preferably an aryl group having 6 to 40 ring carbon atoms below. The aryloxy group is exemplified by a phenoxy group.

Here, examples of the substituted or unsubstituted aryloxy group include a haloaryloxy group that is obtained by substituting an aryl group (R^{A}) with one or more halogen atoms described above.

Moreover, when R^{A} is a heteroaryl group, the group represented by -OR^{A} is a heteroaryloxy group, preferably a substituted or unsubstituted heteroaryloxy group having 5 to 40 ring atoms. The heteroaryl group as R^{A} is preferably a heteroaryl group having 5 to 40 ring atoms below.

Examples of the substituted or unsubstituted carboxyl group in the formulae (1), (1-1) to (1-16), (2), (2-1), (1-x), (1-xx-1) and (1-xx-2) include a carboxyl group (-COOH) and a group represented by -COOR^{B} that is obtained by substituting H of a carboxyl group (-COOH) with R^{B}.

Herein, when R^{B} is an alkyl group, the group represented by-COOR^{B} is an alkoxycarbonyl group, preferably a substituted or unsubstituted alkoxycarbonyl group having 2 to 30 carbon atoms. The alkyl group as R^{B} is preferably an alkyl group having 1 to 30 carbon atoms below.

Moreover, when R^{B} is an aryl group, the group represented by-COOR^{B} is an aryloxycarbonyl group, preferably a substituted or unsubstituted aryloxycarbonyl group having 7 to 40 carbon atoms. The aryl group as R^{B} is preferably an aryl group having 6 to 40 ring carbon atoms below.

Moreover, when R^{B} is a heteroaryl group, the group represented by-COOR^{B} is a heteroarylcarbonyl group, preferably a substituted or unsubstituted heteroarylcarbonyl group having 5 to 40 ring atoms. The heteroaryl group as R^{B} is preferably a heteroaryl group having 5 to 40 ring atoms below.

Examples of the substituted or unsubstituted boryl group in the formulae (1), (1-1) to (1-16), (2), (2-1), (1-x), (1-xx-1) and (1-xx-2) include a boryl group (-BH₂) and a group represented by -BR^{E}R^{E} that is obtained by substituting H of a boryl group (-BH₂) with R^{E},R^{E}.

Herein, when R^{E} is an alkyl group, the group represented by -BR^{E}R^{E} is an alkylboryl group, preferably a substituted or unsubstituted alkylboryl. The alkyl group as R^{E} is preferably an alkyl group having 1 to 30 carbon atoms below.

When R^{E} is an aryl group, the group represented by -BR^{E}R^{E} is an arylboryl group, preferably a substituted or unsubstituted arylboryl. The aryl group as R^{E} is preferably an aryl group having 6 to 40 ring carbon atoms below.

When R^{E} is a heteroaryl group, the group represented by -BR^{E}R^{E} is a heteroarylboryl group, preferably a substituted or unsubstituted heteroarylboryl. The heteroaryl group as R^{E} is preferably a heteroaryl group having 5 to 40 ring atoms below.

In addition, a dihydroxyboryl group (-B(OH)₂) is also usable.

Examples of the substituted or unsubstituted phosphino group in the formulae (1), (1-1) to (1-16), (2), (2-1), (1-x), (1-xx-1) and (1-xx-2) include a phosphino group (-PH₂), a group represented by -PR^{F}R^{F} that is obtained by substituting H of a phosphino group (-PH₂) with R^{F},R^{F}, and a group represrented by -P(O)R^{F}R^{F}.

Herein, when R^{F} is an alkyl group, the group represented by -PR^{F}R^{F} is an alkylphosphino group, preferably a substituted or unsubstituted alkylphosphino. The alkyl group as R^{F} is preferably an alkyl group having 1 to 30 carbon atoms below.

When R^{F} is an aryl group, the group represented by -PR^{F}R^{F} is an arylphosphino group, preferably a substituted or unsubstituted arylphosphino. The aryl group as R^{F} is preferably an aryl group having 6 to 40 ring carbon atoms below.

When R^{F} is a heteroaryl group, the group represented by -PR^{F}R^{F} is a heteroarylphosphino group, preferably a substituted or unsubstituted heteroarylphosphino. The heteroaryl group as R^{F} is preferably a heteroaryl group having 5 to 40 ring atoms below.

Examples of the substituted or unsubstituted mercapto group in the formulae (1), (1-1) to (1-16), (2), (2-1), (1-x), (1-xx-1) and (1-xx-2) include a mercapto group (-SH) and a group represented by -SR^{C} that is obtained by substituting H of a mercapto group (-SH) with R^{C}.

Herein, when R^{C} is an alkyl group, the group represented by -SR^{C} is an alkylthio group, preferably a substituted or unsubstituted alkylthio group having 1 to 30 carbon atoms. The alkyl group as R^{C} is preferably an alkyl group having 1 to 30 carbon atoms below.

Moreover, when R^{C} is an aryl group, the group represented by -SR^{C} is an arylthio group, preferably a substituted or unsubstituted arylthio group having 6 to 40 ring carbon atoms. The aryl group as R^{C} is preferably an aryl group having 6 to 40 ring carbon atoms below.

Moreover, when R^{C} is a heteroaryl group, the group represented by -SR^{C} is a heteroarylthio group, preferably a substituted or unsubstituted heteroarylthio group having 5 to 40 ring atoms. The heteroaryl group as R^{C} is preferably a heteroaryl group having 5 to 40 ring atoms below.

The substituted or unsubstituted acyl group in the formulae (1), (1-1) to (1-16), (2), (2-1), (1-x), (1-xx-1) and (1-xx-2) is represented by -CO-R^{D}.

Herein, when R^{D} is an alkyl group, the acyl group is an alkylcarbonyl group, preferably a substituted or unsubstituted alkylcarbonyl group having 2 to 30 carbon atoms. The alkyl group as R^{D} is preferably an alkyl group having 1 to 30 carbon atoms below. Examples of the alkylcarbonyl group include an acetyl group, propionyl group, butyryl group, valeryl group, pivaloyl group, palmitoyl group, stearoyl group and oleoyl group.

Moreover, when R^{D} is an aryl group, the acyl group is an arylcarbonyl group (also referred to as an aroyl group), preferably a substituted or unsubstituted arylcarbonyl group having 6 to 40 ring carbon atoms. The aryl group as R^{D} is preferably an aryl group having 6 to 40 ring carbon atoms below. Examples of the arylcarbonyl group include a benzoyl group, toluoyl group, salicyloyl group, cinnamoyl group, naphthoyl group and phthaloyl group.

Moreover, when R^{D} is a heteroaryl group, the represented by -CO-R^{D} is a heteroarylcarbonyl group, preferably a substituted or unsubstituted heteroarylcarbonyl group having 5 to 40 ring atoms. The heteroaryl group as R^{D} is preferably a heteroaryl group having 5 to 40 ring atoms below. Examples of the heteroarylcarbonyl group include a furoyl group, pyrrolylcarbonyl group, pyridylcarbonyl group and thienylcarbonyl group.

It should be noted that a formyl group (-CO-H) in which R^{D} is substituted with a hydrogen atom is included in the examples of the acyl group.

Examples of the substituted or unsubstituted amino group in the formulae (1), (1-1) to (1-16), (2), (2-1), (1-x), (1-xx-1) and (1-xx-2) include an amino group (-NH₂) and an amino group in which H of an amino group (-NH₂) are substituted with substituents. Examples of the substituted amino group include an alkylamino group substituted with a substituted or unsubstituted alkyl having 1 to 30 carbon atoms, an aryl amino group substituted with a substituted or unsubstituted aryl group having 6 to 40 ring carbon atoms, a heteroarylamino group substituted with a substituted or unsubstituted heteroaryl group having 5 to 40 ring atoms, and an acylamino group substituted with a substituted or unsubstituted acyl group having 2 to 30 carbon atoms.

The alkyl group having 1 to 30 carbon atoms in the alkylamino group is preferably an alkyl group having 1 to 30 carbon atoms listed below. When the amino group is substituted with two alkyl groups, the two alkyl groups may be mutually the same or different.

The aryl group having 6 to 40 ring carbon atoms in the arylamino group is preferably an aryl group having 6 to 40 ring carbon atoms. The arylamino group is preferably an amino group substituted with a phenyl group. When the amino group is substituted with two aryl groups, the two aryl groups may be mutually the same or different.

The heteroaryl group having 5 to 40 ring atoms in the heteroarylamino group is preferably a heteroaryl group having 5 to 40 ring atoms below. When the amino group is substituted with two heteroaryl groups, the two heteroaryl groups may be mutually the same or different.

The acyl group having 2 to 30 carbon atoms in the acylamino group is preferably selected from the above acyl group.

The substituted amino group may be an amino group substituted with two selected from a hydrogen atom, alkyl group, aryl group, heteroaryl group and acyl group.

The substituted amino group may be an amino group substituted with an alkyl group and an aryl group. Examples of the substituted amino group include an alkylarylamino group, alkylheteroarylamino group, arylheteroarylamino group, alkylacylamino group and arylacylamino group.

Examples of the substituted or unsubstituted silyl group in the formulae (1), (1-1) to (1-16), (2), (2-1), (1-x), (1-xx-1) and (1-xx-2) include an unsubstituted silyl group, an alkylsilyl group substituted with a substituted or unsubstituted alkyl group having 1 to 30 carbon atoms, an arylsilyl group substituted with a substituted or unsubstituted aryl group having 6 to 40 ring carbon atoms, and a heteroarylsilyl group substituted with a substituted or unsubstituted heteroaryl group having 5 to 40 ring atoms.

The alkylsilyl group is exemplified by a trialkylsilyl group having the above alkyl group having 1 to 30 carbon atoms. Specific examples of the trialkylsilyl group include a trimethylsilyl group, triethylsilyl group, tri-n-butylsilyl group, tri-n-octylsilyl group, triisobutylsilyl group, dimethylethylsilyl group, dimethylisopropylsilyl group, dimethyl-n-propylsilyl group, dimethyl-n-butylsilyl group, dimethyl-t-butylsilyl group, diethylisopropylsilyl group, vinyldimethylsilyl group, propyldimethylsilyl group and triisopropylsilyl group. Three alkyl groups in the trialkylsilyl group may be mutually the same or different.

The arylsilyl group is exemplified by a triarylsilyl group including three of an aryl group having 6 to 40 ring carbon atoms below. The triarylsilyl group preferably has 18 to 30 carbon atoms. The three aryl groups may be mutually the same or different.

The heteroarylsilyl group is exemplified by a triheteroarylsilyl group having three of a heteroaryl group having 5 to 40 ring atoms below. The three heteroaryl groups may be mutually the same or different.

The substituted silyl group may be a silyl group substituted with at least two groups selected from an alkyl group, aryl group and heteroaryl group.

The substituted silyl group may be a silyl group substituted with an alkyl group and an aryl group. Examples of the substituted silyl group include an alkylarylsilyl group, dialkylarylsilyl group, diarylsilyl group, alkkyldiarylsilyl group and triarlysilyl group. A plurality of aryl groups or alkyl groups may be mutually the same or different.

The dialkylarylsilyl group is exemplified by a dialkylarylsilyl group including two of the alkyl group listed as the examples of the alkyl group having 1 to 30 carbon atoms and one of the aryl group listed as the examples of the aryl group having 6 to 40 ring carbon atoms. The dialkylarylsilyl group preferably has 8 to 30 carbon atoms. The two alkyl groups may be mutually the same or different.

The alkyldiarylsilyl group is exemplified by an alkyldiarylsilyl group including one of the alkyl group listed as the examples of the alkyl group having 1 to 30 carbon atoms and two of the aryl group listed as the examples of the aryl group having 6 to 40 ring carbon atoms. The alkyldiarylsilyl group preferably has 13 to 30 carbon atoms. The two aryl groups may be mutually the same or different.

Examples of the above arylsilyl group are a phenyldimethylsilyl group, a diphenylmethylsilyl group, a diphenyl-t-butylsilyl group and a triphenylsilyl group.

The substituted silyl group may be a silyl group substituted with an alkyl group and a heteroaryl group, a silyl group substituted with an aryl group and a heteroaryl group, and a silyl group substituted with an alkyl group, aryl group and heteroaryl group.

The alkyl group having 1 to 30 carbon atoms in the formulae (1), (1-1) to (1-16), (2), (2-1), (1-x), (1-xx-1) and (1-xx-2) may be linear, branched or cyclic. Examples of the substituted alkyl group having 1 to 30 carbon atoms include a haloalkyl group. The haloalkyl group is exemplified by a haloalkyl group obtained by substituting the alkyl group having 1 to 30 carbon atoms with one or more halogen atoms. Examples of the substituted or unsubstituted linear or branched alkyl group are a methyl group, ethyl group, propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, 2-methylpentyl group, 1-pentylhexyl group, 1-butylpentyl group, 1-heptyloctyl group, 3-methylpentyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 1,2-dinitroethyl group, 2,3-dinitro-t-butyl group, 1,2,3-trinitropropyl group, fluoromethyl group, difluoromethyl group, trifluoromethyl group, fluoroethyl group, 2,2,2-trifluoroethyl group and 1,1,1,3,3,3-hexafluoro-2-propyl group.

The substituted or unsubstituted cyclic alkyl group (cycloalkyl group) is preferably a substituted or unsubstituted cycloalkyl group having 3 to 30 carbon atoms. Examples of the cycloalkyl group include a cyclopropyl group, cyclobutyl group, cyclopentyl group, cyclohexyl group, cyclooctyl group, 4-methylcyclohexyl group, 3,5-tetramethylcyclohexyl group, 1-adamantyl group, 2-adamantyl group, 1-norbornyl group and 2-norbornyl group.

Among the above alkyl group, an alkyl group having 1 to 10 carbon atoms is preferable. An alkyl group having 1 to 8 carbon atoms is more preferable. An alkyl group having 1 to 6 carbon atoms is particularly preferable. Among the above alkyl group, a methyl group, isopropyl group, t-butyl group and cyclohexyl group are preferable.

The substituted or unsubstituted alkenyl group having 2 to 30 carbon atoms in the formulae (1), (1-1) to (1-16), (2), (2-1), (1-x), (1-xx-1) and (1-xx-2) may be linear, branched or cyclic. Examples of the alkenyl group are a vinyl group, propenyl group, butenyl group, oleyl group, eicosapentaenyl group, docosahexaenyl group, styryl group, 2,2-diphenylvinyl group, 1,2,2-triphenylvinyl group and 2-phenyl-2-propenyl group. Among the above alkenyl group, a vinyl group is preferable.

Examples of the substituted or unsubstituted alkynyl group having 2 to 30 carbon atoms in the formulae (1), (1-1) to (1-16), (2), (2-1), (1-x), (1-xx-1) and (1-xx-2) include an ethynyl group, propynyl group and and 2-phenylethynyl group. Among the above alkynyl group, an ethynyl group is preferable.

The aralkyl group having 7 to 40 carbon atoms in the formulae (1), (1-1) to (1-16), (2), (2-1), (1-x), (1-xx-1) and (1-xx-2) is represented by -R^{E}-R^{F}. R^{E} is exemplified by an alkylene group provided as a divalent group derived from the alkyl group having 1 to 30 carbon atoms described below. R^{F} is exemplified by the examples of an aryl group having 6 to 40 ring carbon atoms below. In the aralkyl group, an aryl group moiety has 6 to 40 carbon atoms, preferably 6 to 20 carbon atoms, more preferably 6 to 12 carbon atoms. In the aralkyl group, an alkyl group moiety has 1 to 30 carbon atoms, preferably 1 to 20 carbon atoms, more preferably 1 to 10 carbon atoms, further preferably 1 to 6 carbon atoms. Examples of the aralkyl group are a benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrorylmethyl group, 2-(1-pyroryl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

Examples of the aryl group having 6 to 40 ring carbon atoms in the formulae (1), (1-1) to (1-16), (2), (2-1), (1-x), (1-xx-1) and (1-xx-2) include a non-fused aryl group and a fused aryl group. Specific examples of the aryl group include a phenyl group, naphthyl group, anthryl group, phenanthryl group, biphenyl group, terphenyl group, quaterphenyl group, fluoranthenyl group, pyrenyl group, triphenylenyl group, phenanthrenyl group, fluorenyl group, 9,9-dimethylfluorenyl group, spirofluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group and benzo[b]fluoranthenyl group. Among the above aryl group, an aryl group having 6 to 30 ring carbon atoms is preferable. An aryl group having 6 to 20 ring carbon atoms is more preferable. An aryl group having 6 to 12 ring carbon atoms is particularly preferable.

Moreover, since a high triplet energy level is desired in use for an electron transporting material, an aryl group having triplet energy T1 exceeding 2.1 eV is preferable. Examples of the aryl group include a phenyl group, naphthyl group, phenanthryl group, biphenyl group, terphenyl group, quaterphenyl group, fluoranthenyl group, triphenylenyl group, phenanthrenyl group, fluorenyl group, 9,9-dimethylfluorenyl group, spirofluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group and benzo[b]fluoranthenyl group.

Examples of the heteroaryl group having 5 to 40 ring atoms in the formulae (1), (1-1) to (1-16), (2), (2-1), (1-x), (1-xx-1) and (1-xx-2) include a non-fused heteroaryl group and a fused heteroaryl group. Specific examples of the heteroaryl group include a pyroryl group, pyrazinyl group, pyridinyl group, indolyl group, isoindolyl group, furyl group, benzofuranyl group, isobenzofuranyl group, dibenzofuranyl group, dibenzothiophenyl group, quinolyl group, isoquinolyl group, quinoxalinyl group, carbazolyl group, phenanthrydinyl group, acridinyl group, phenanthrolinyl group, thienyl group, and group derived from a pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyrane ring, dibenzofuran ring, benzo[c]dibenzofuran ring and silafluorene ring. Among the above heteroaryl group, a heteroaryl group having 5 to 40 ring atoms is preferable. A heteroaryl group having 5 to 20 ring atoms is more preferable. A heteroaryl group having 5 to 12 ring atoms is particularly preferable.

Examples of the polyvalent linear, branched or cyclic aliphatic hydrocarbon group when L and L₁ to L₁₂ are the linking groups in the formulae (1), (1-1) to (1-16), (2), (2-1), (1-x), (1-xx-1) and (1-xx-2) include: a polyvalent group derived from the polyvalent linear, branched or cyclic alkyl group having 1 to 30 carbon atoms; a polyvalent group derived from the polyvalent linear, branched or cyclic alkenyl group having 1 to 30 carbon atoms; and a polyvalent group derived from the polyvalent linear, branched or cyclic alkynyl group having 1 to 30 carbon atoms, among which a divalent or trivalent group is preferable and a divalent group is more preferable. The divalent group may be substituted with the above-described substituents. Specific examples of the divalent group include a methylene group, ethylene group, acethylenylene and vinylidene group.

Examples of the polyvalent aryl group having 6 to 40 ring carbon atoms when L and L₁ to L₁₂ are the linking groups in the formulae (1), (1-1) to (1-16), (2), (2-1), (1-x), (1-xx-1) and (1-xx-2) include a polyvalent group derived from the aryl group having 6 to 40 ring carbon atoms, among which a divalent or trivalent group is preferable and a divalent group is more preferable. Specifically, a divalent group derived from a phenyl group, biphenyl group, naphthyl group and 9,9-dimethylfluorenyl group is preferable. The divalent group may be subsitutued with the above-described substituents.

Examples of the polyvalent heteroaryl group having 5 to 40 ring atoms when L and L₁ to L₁₂ are the linking groups in the formulae (1), (1-1) to (1-16), (2), (2-1), (1-x), (1-xx-1) and (1-xx-2) include a polyvalent group derived from the heteroaryl group having 5 to 40 ring atoms, among which a divalent or trivalent group is preferable and a divalent group is more preferable. Specifically, a divalent group derived from a pyridyl group, pyrimidyl group, dibenzofuranyl group, silafluorenyl group and carbazolyl group is preferable. The divalent group may be subsitutued with the above-described substituents.

In the formulae (1), (1-1) to (1-16), (2), (2-1), (1-x), (1-xx-1) and (1-xx-2), the rest of Y¹ to Y¹⁶ except for the carbon atom to be bonded to L and L₁ to L₁₂ are preferably CR^{Y}, in which R^{Y} is preferably a hydrogen atom or alkyl group, particularly preferably a hydrogen atom.

In the invention, "carbon atoms forming a ring (ring carbon atoms)" mean carbon atoms forming a saturated ring, an unsaturated ring, or an aromatic ring. "Atoms forming a ring (ring atoms)" mean carbon atoms and hetero atoms forming a hetero ring including a saturated ring, unsaturated ring, or aromatic ring.

In the invention, a hydrogen atom includes isotope having different numbers of neutrons, specifically, protium, deuterium and tritium.

Examples of the substituent meant by "substituted or unsubstituted" are the above-described aryl group, heteroaryl group, alkyl group (linear or branched alkyl group, cycloalkyl group and haloalkyl group), alkoxy group, aryloxy group, aralkyl group, haloalkoxy group, alkylsilyl group, dialkylarylsilyl group, alkyldiarylsilyl group, triarylsilyl group, halogen atom, cyano group, hydroxyl group, nitro group and carboxy group. In addition, the alkenyl group and alkynyl group are also usable.

Among the above substituents, an aryl group, heteroaryl group, alkyl group, halogen atom, alkylsilyl group, arylsilyl group and cyano group are preferable. More preferable substituents are one listed as the preferable substituents described for each substituent.

"Unsubstituted" in "substituted or unsubstituted" means that a group is not substituted by the above-described substituents but bonded with a hydrogen atom.

Herein, "a to b carbon atoms" in the description of "substituted or unsubstituted XX group having a to b carbon atoms" represent carbon atoms of an unsubstituted XX group and does not include carbon atoms of a substituted XX group.

The same description as the above applies to "substituted or unsubstituted" in the following compound or a partial structure thereof.

Specific examples of the compound represented by the formula (1) are shown below, but the invention is not limited to thereto.

Examples of the compound represented by the formula (1-7) are as follows.

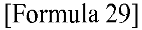

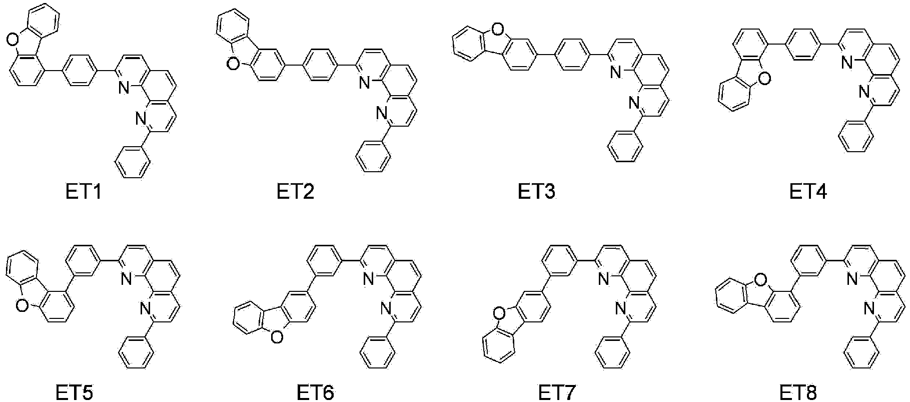

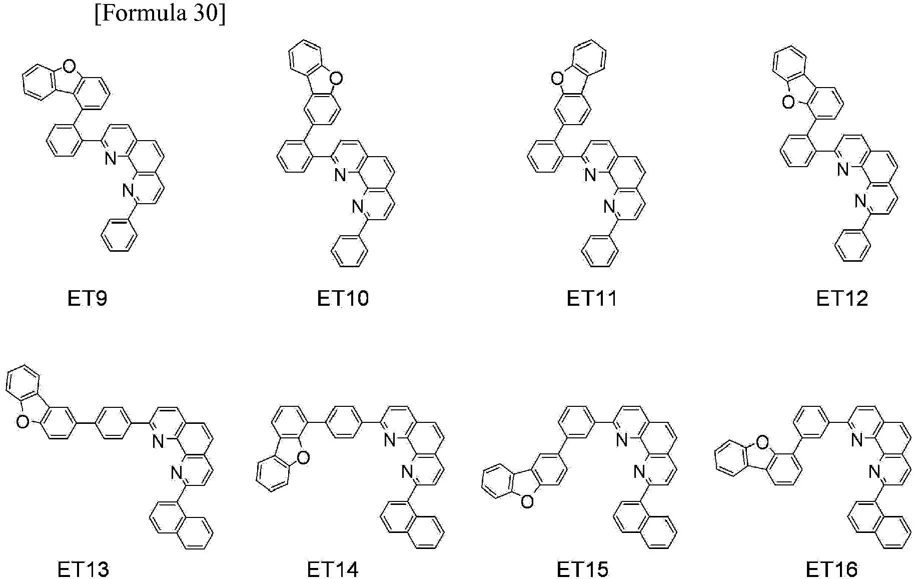

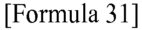

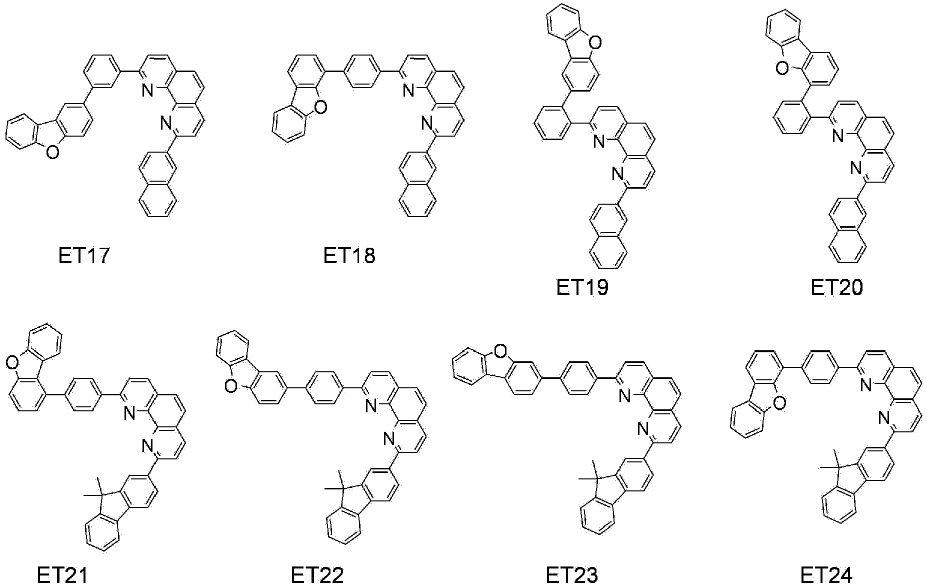

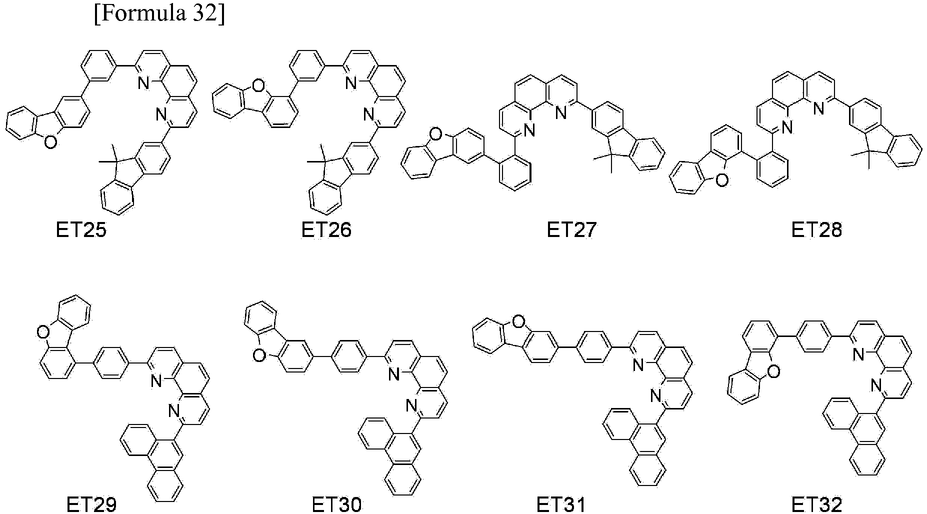

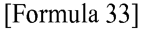

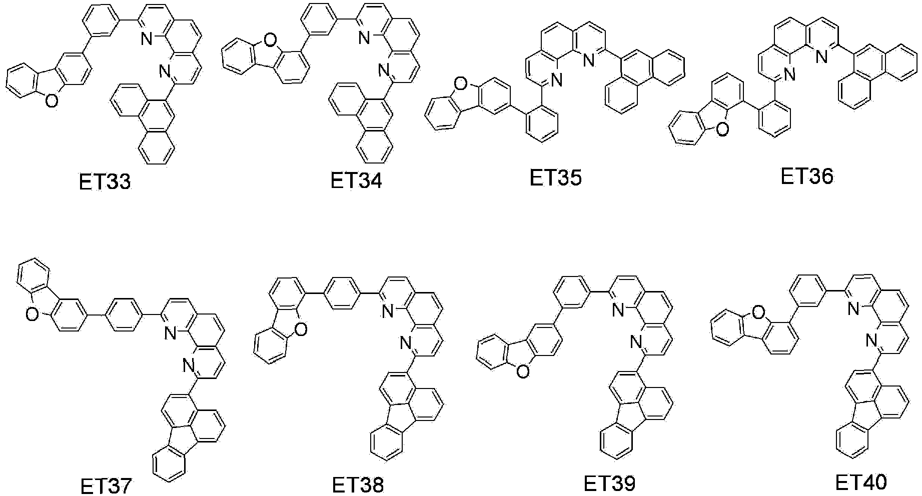

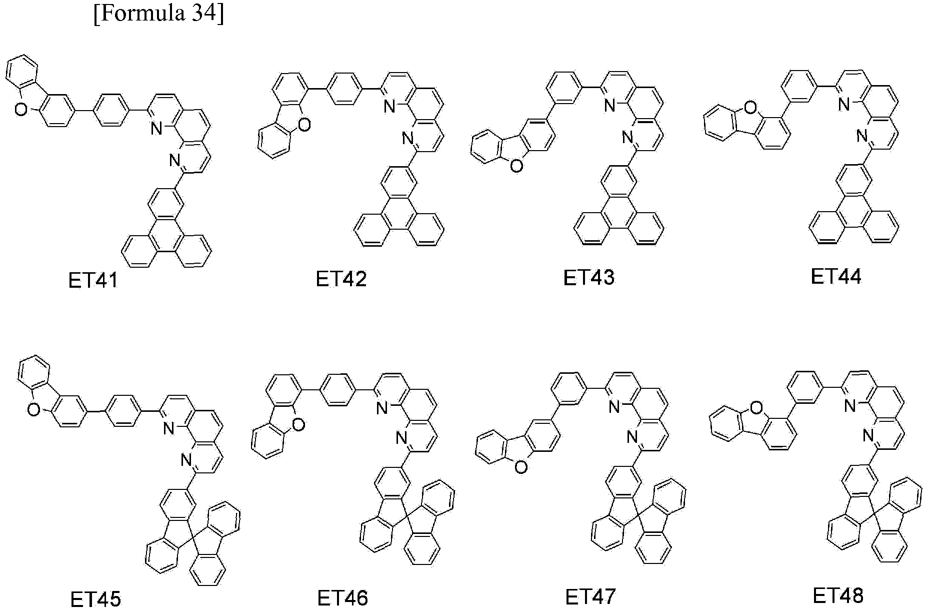

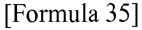

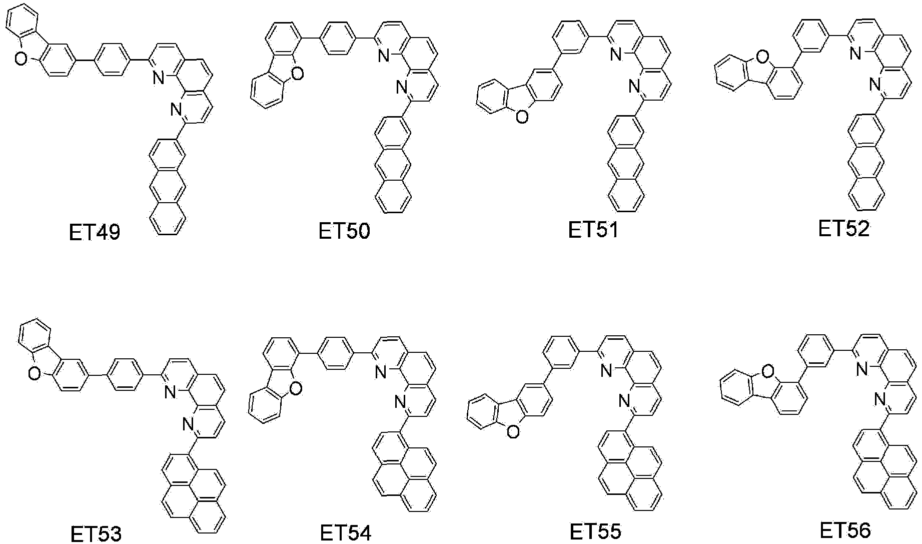

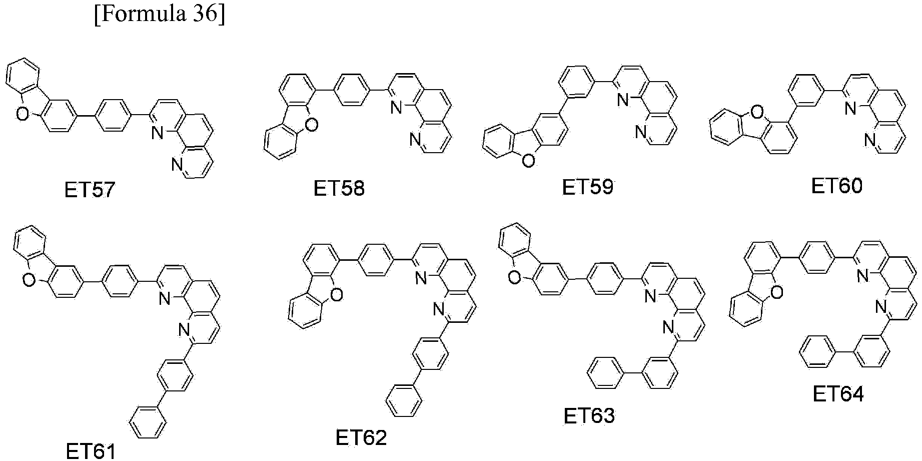

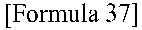

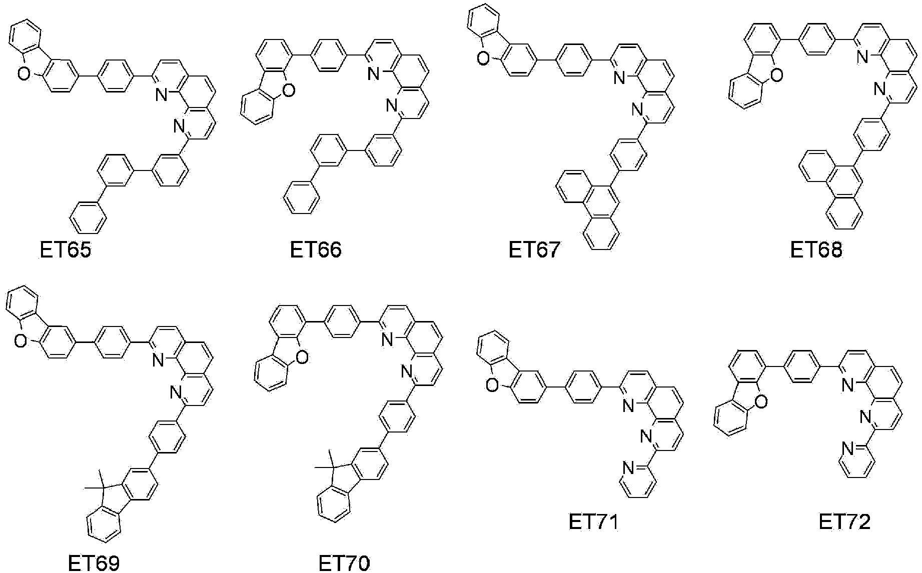

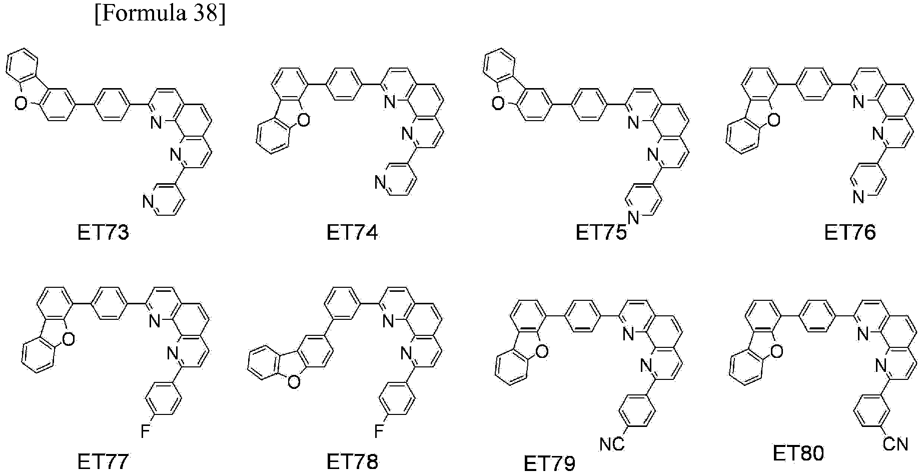

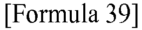

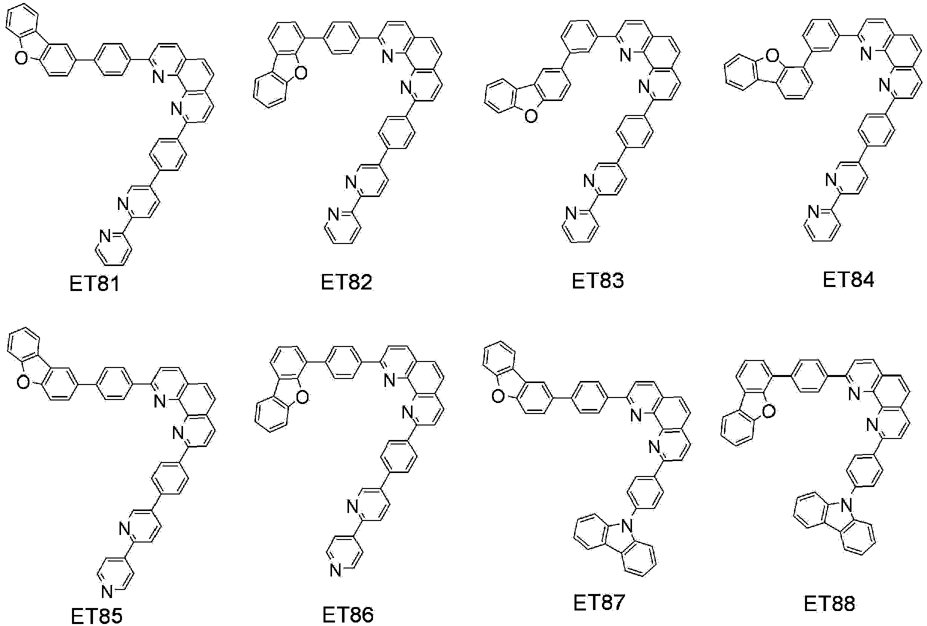

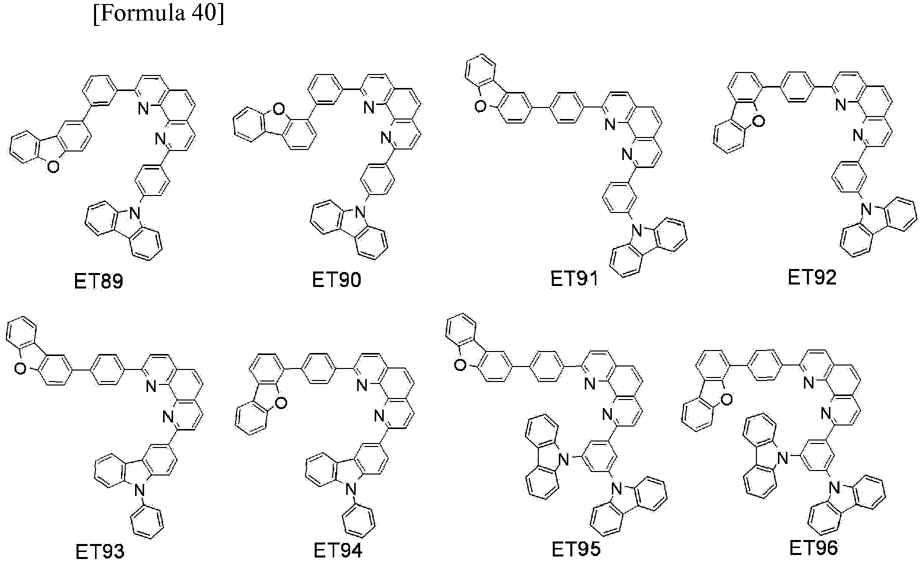

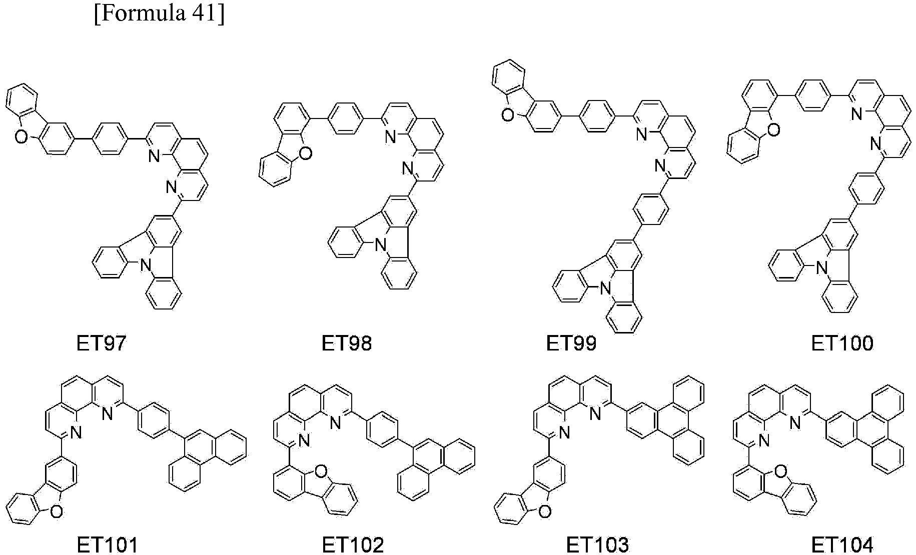

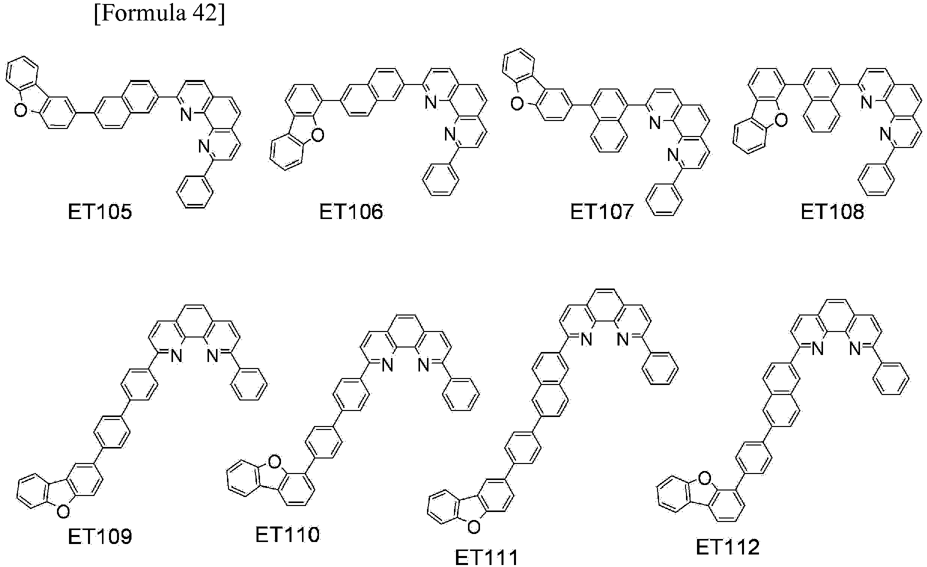

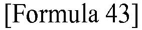

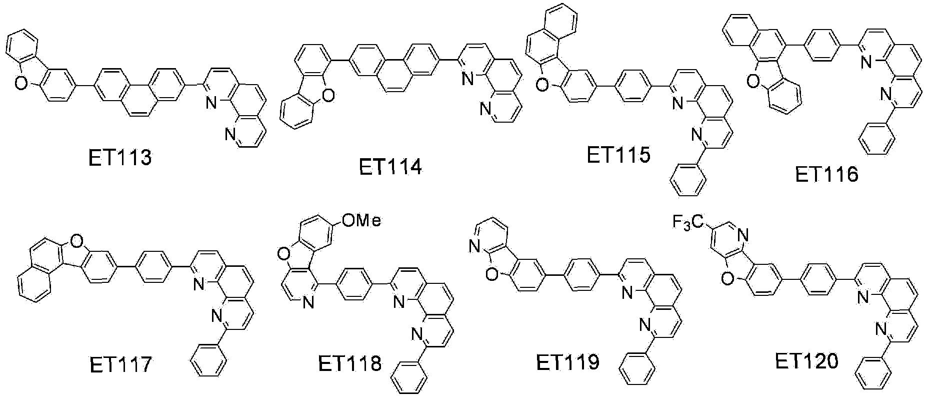

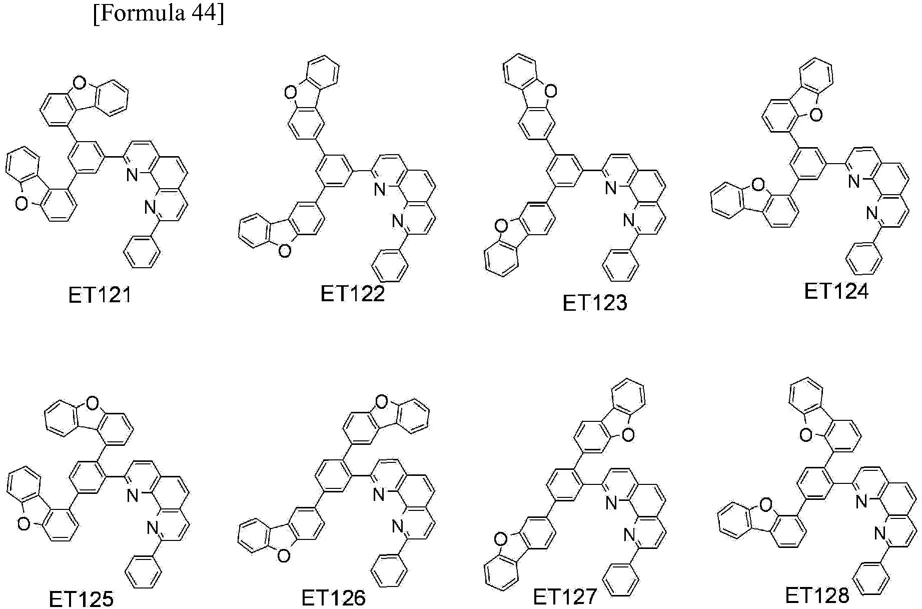

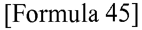

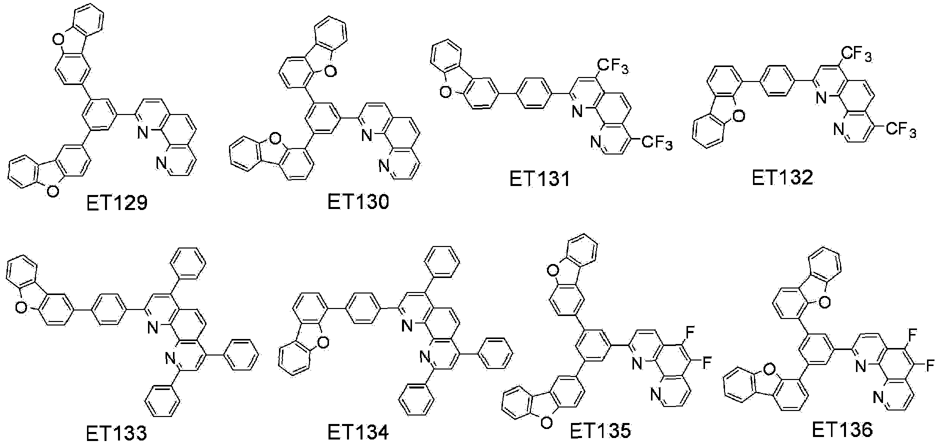

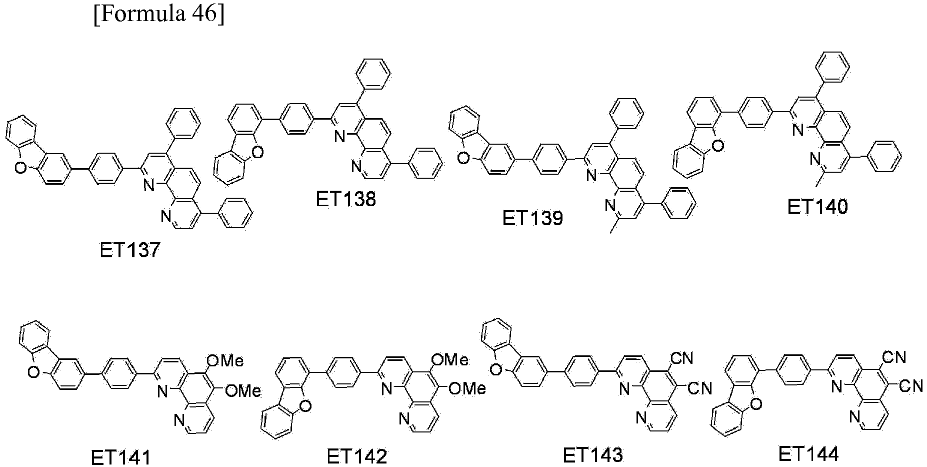

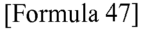

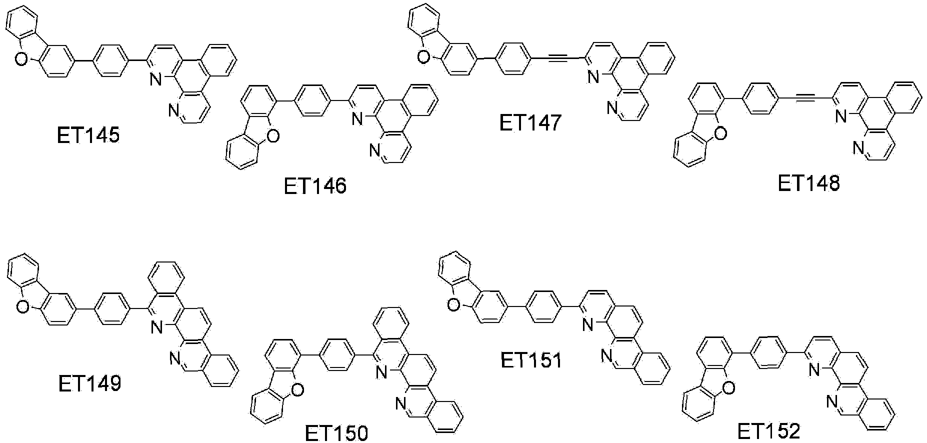

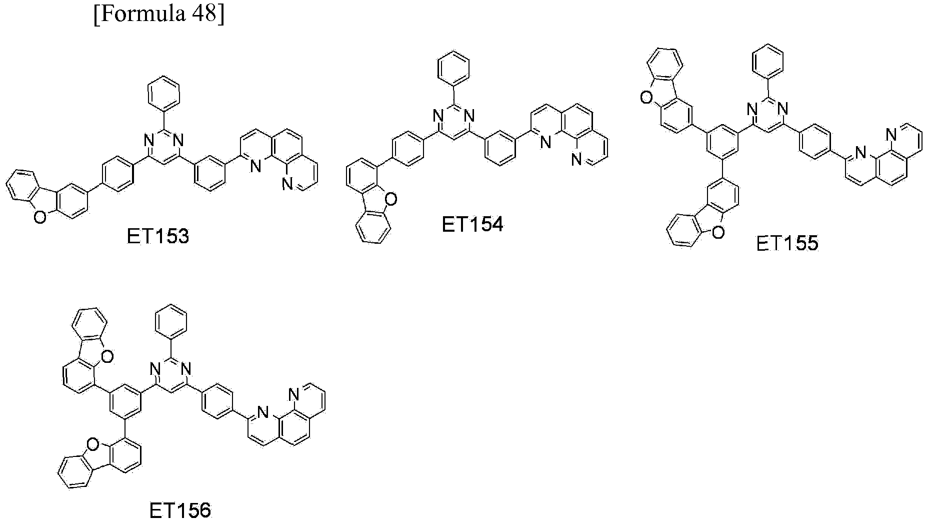

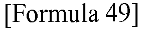

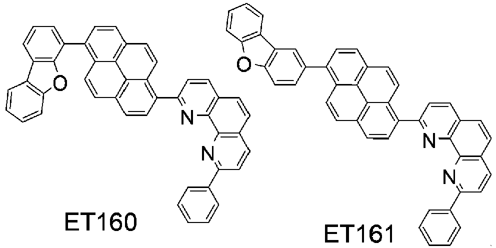

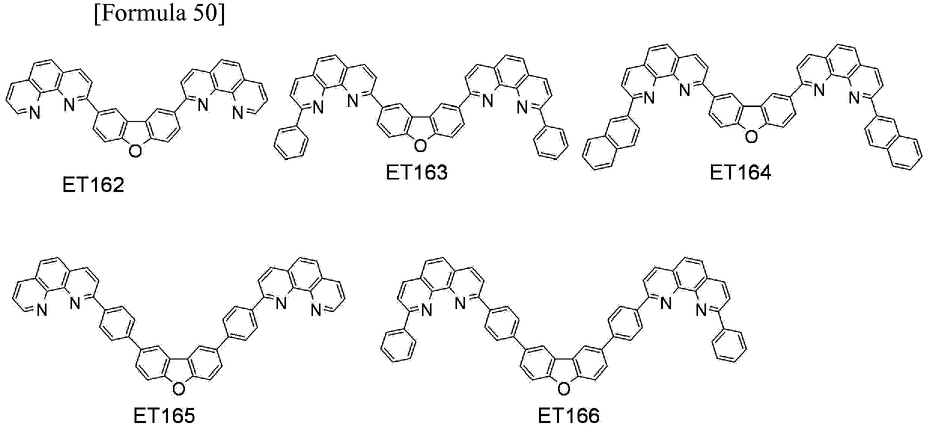

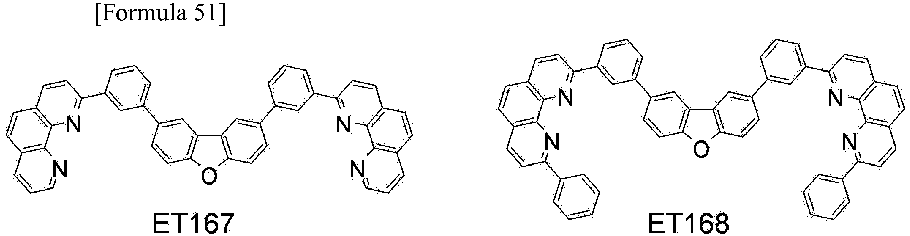

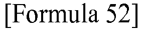

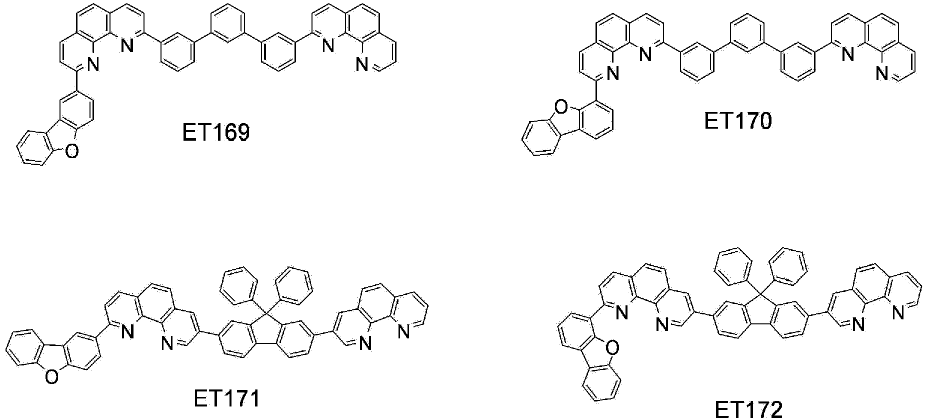

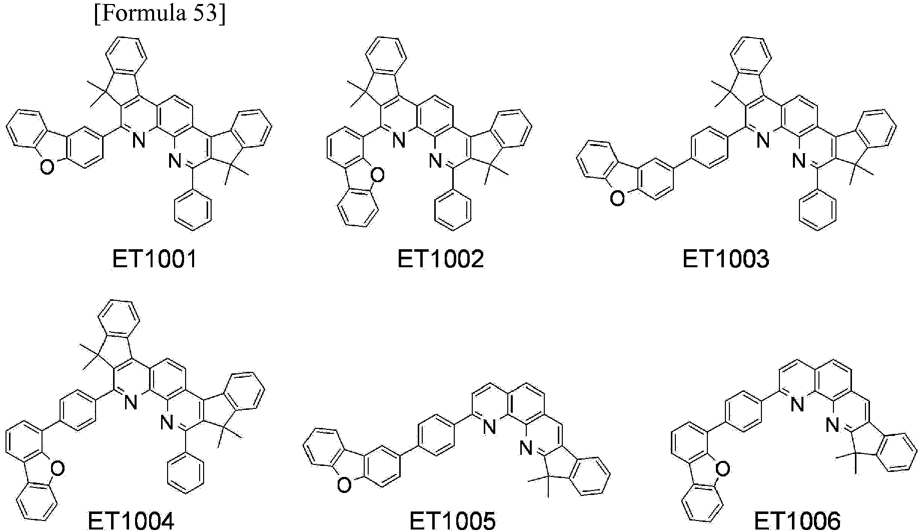

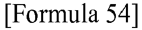

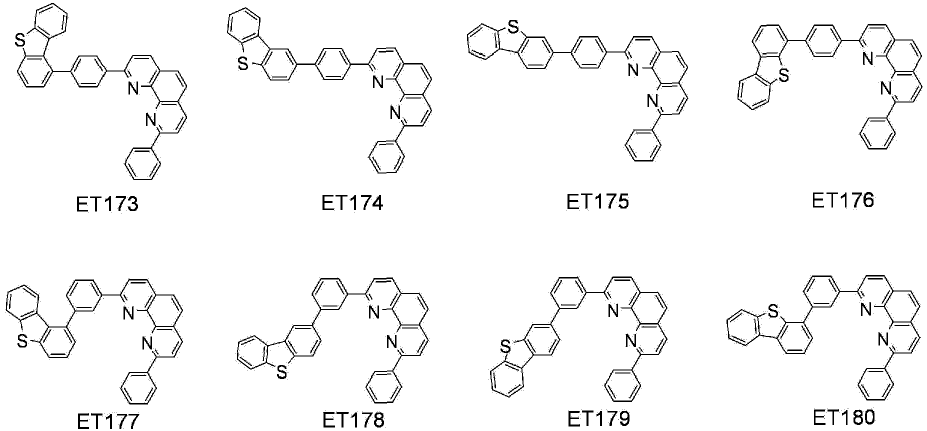

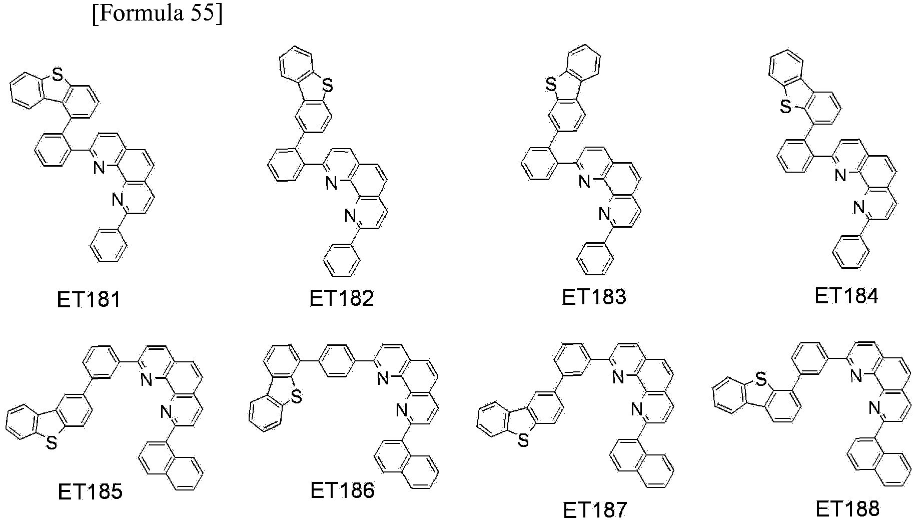

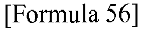

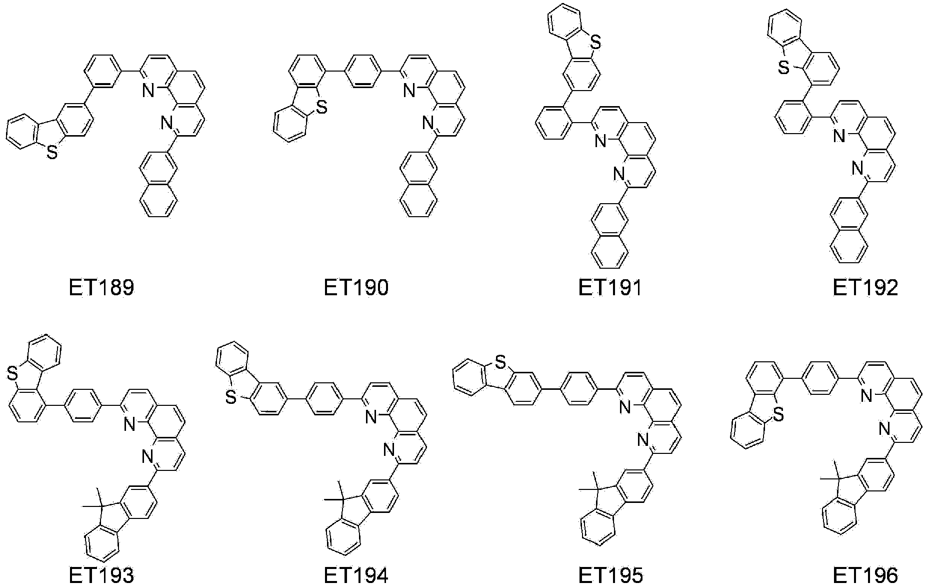

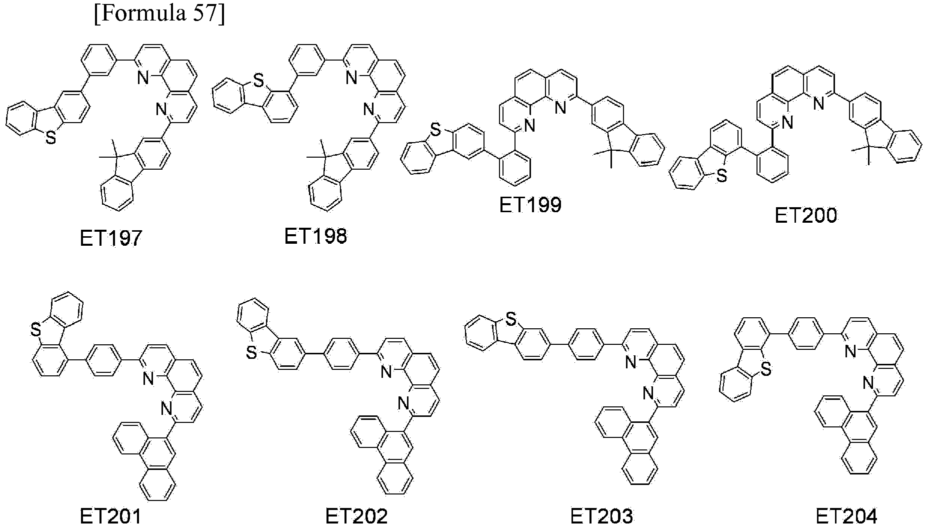

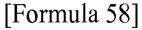

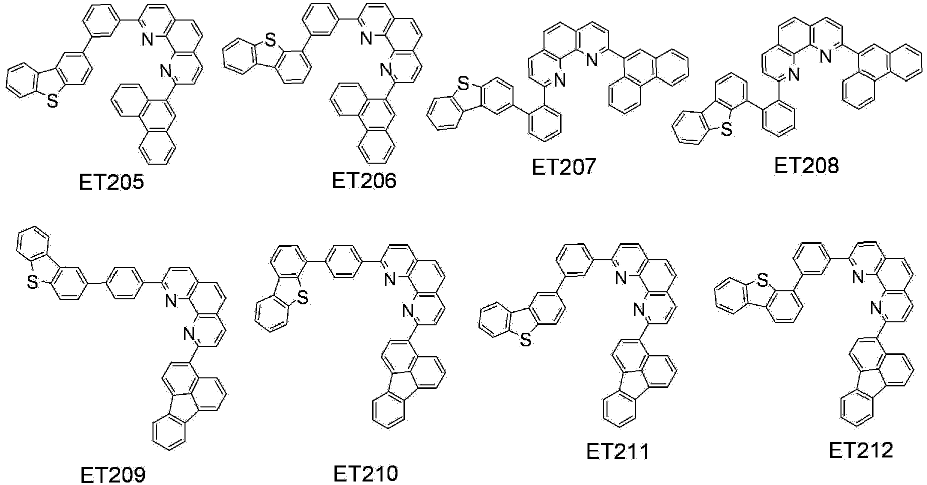

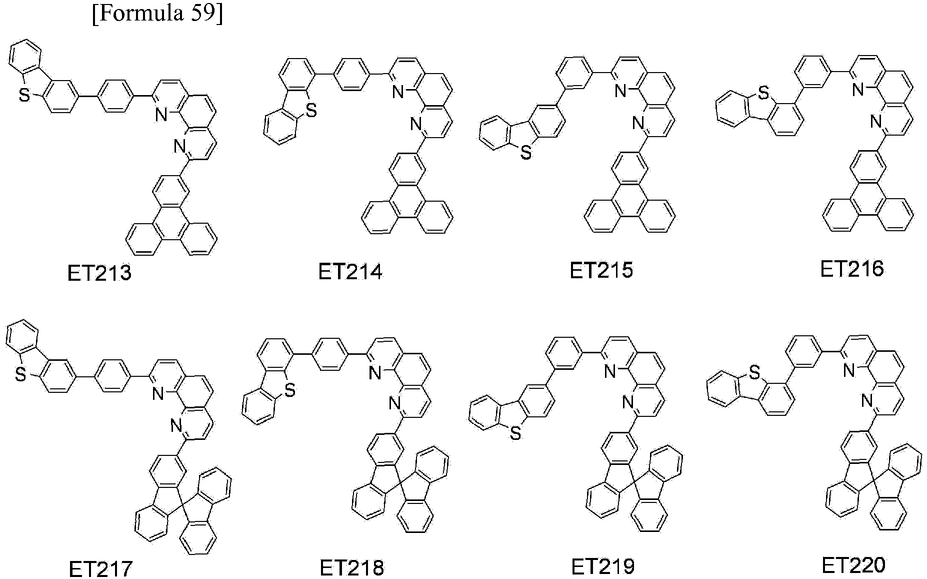

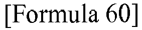

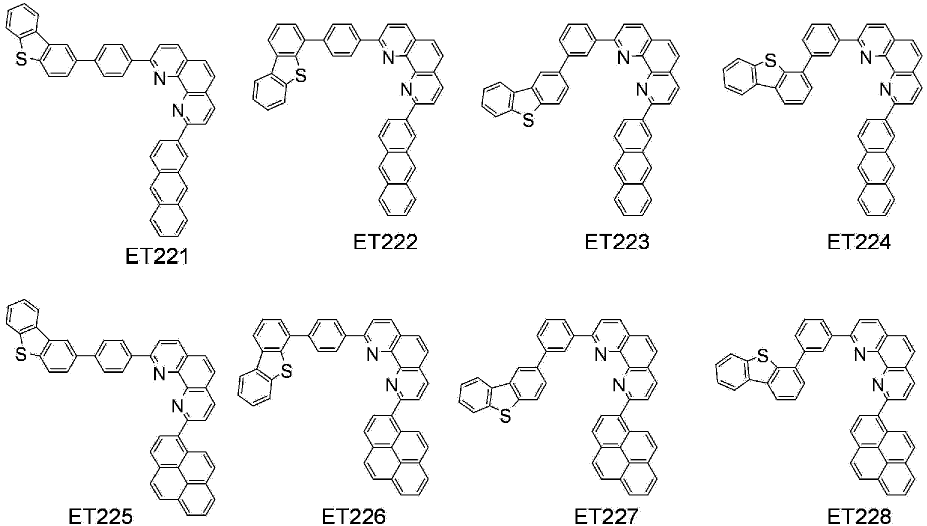

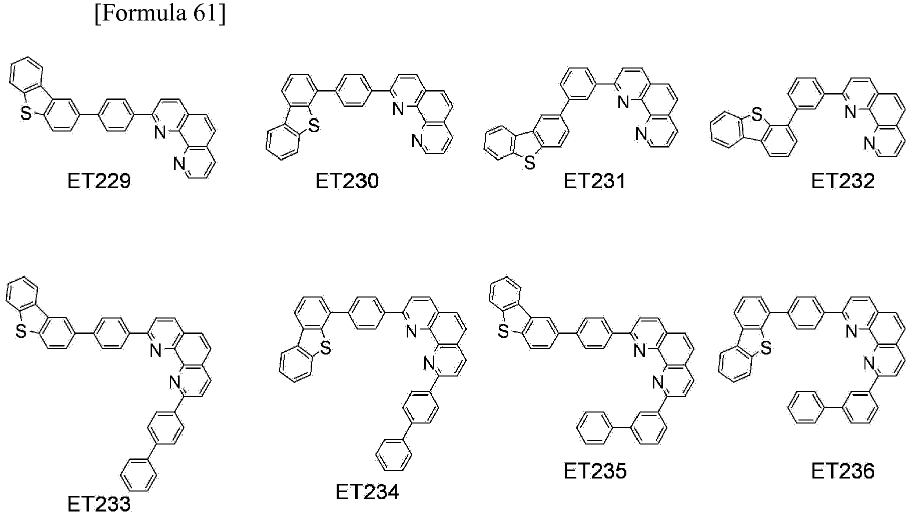

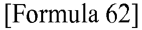

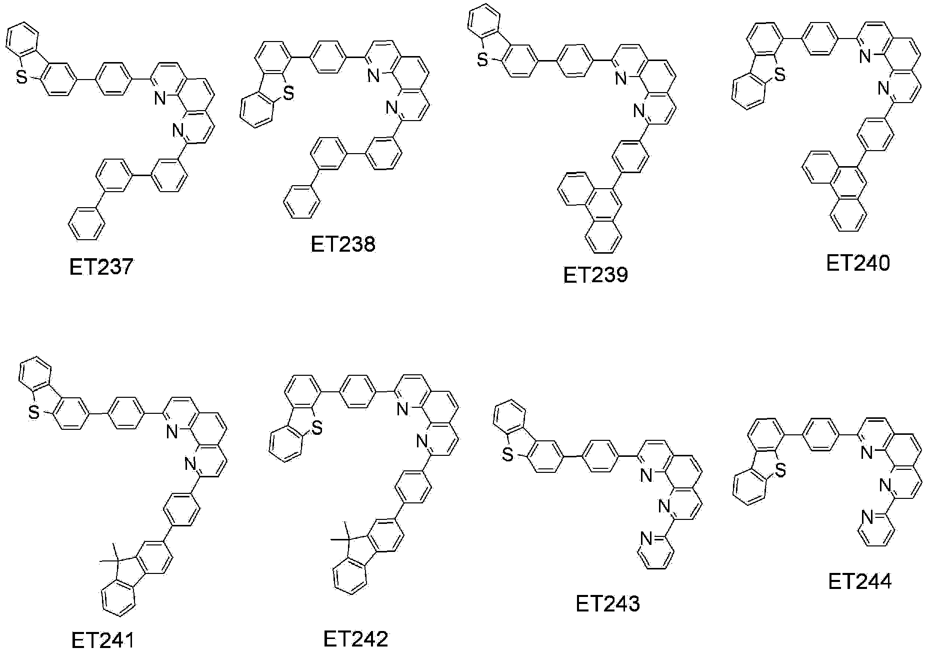

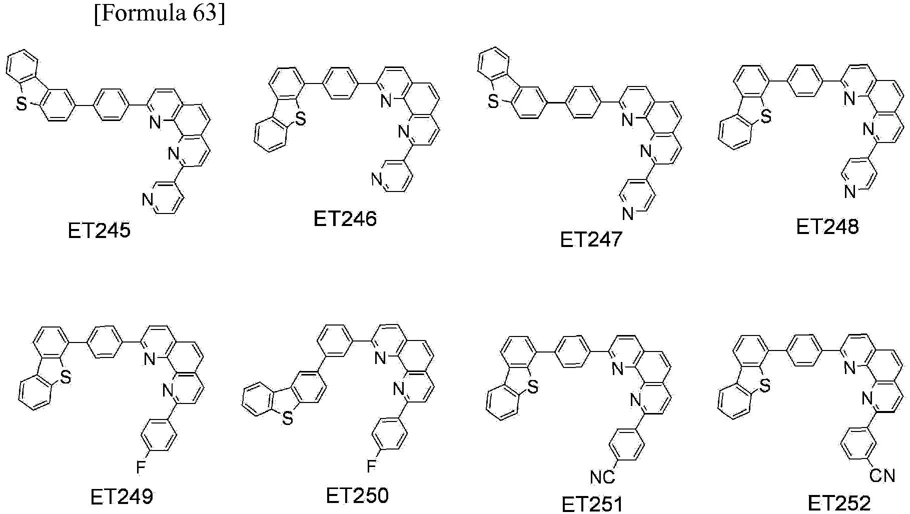

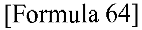

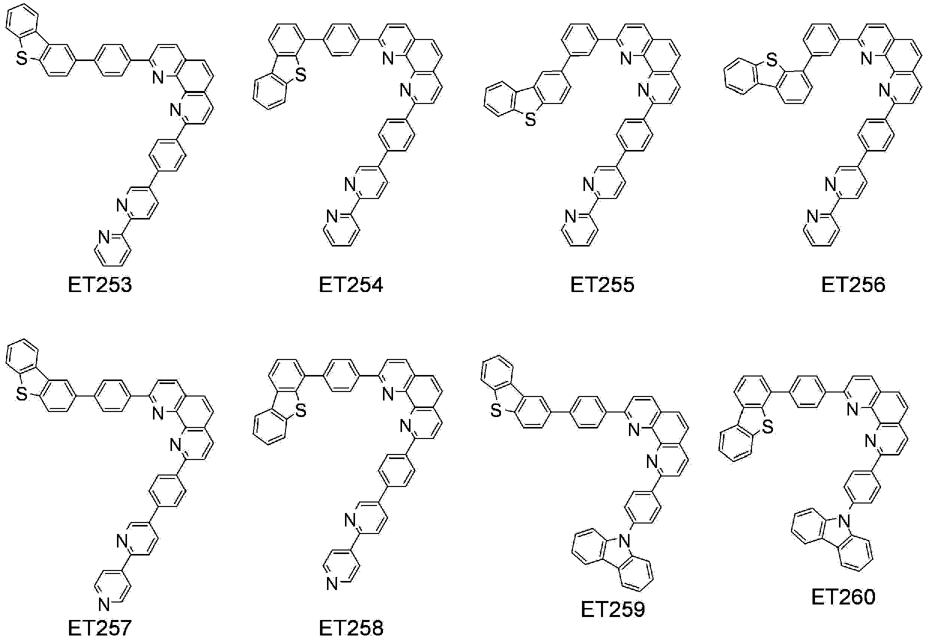

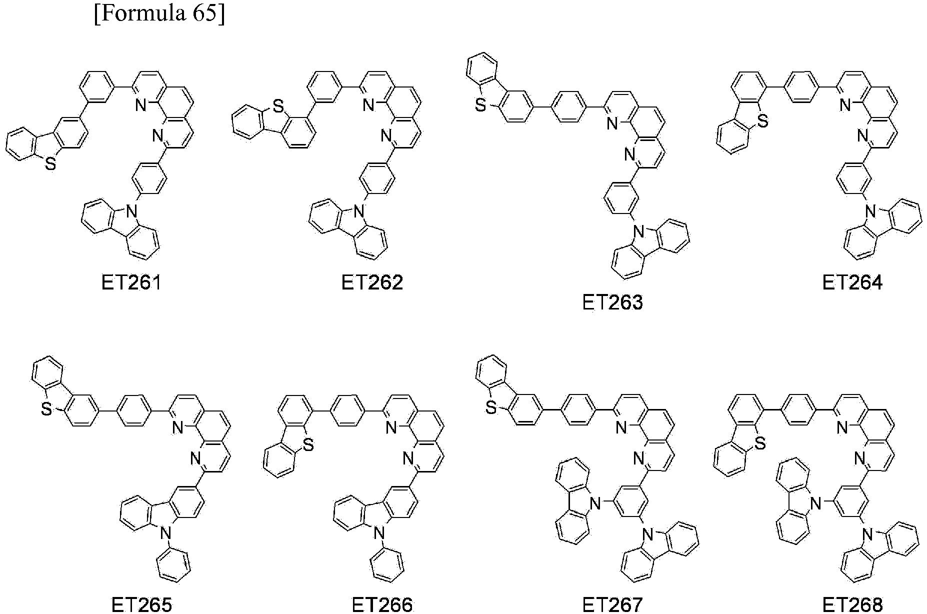

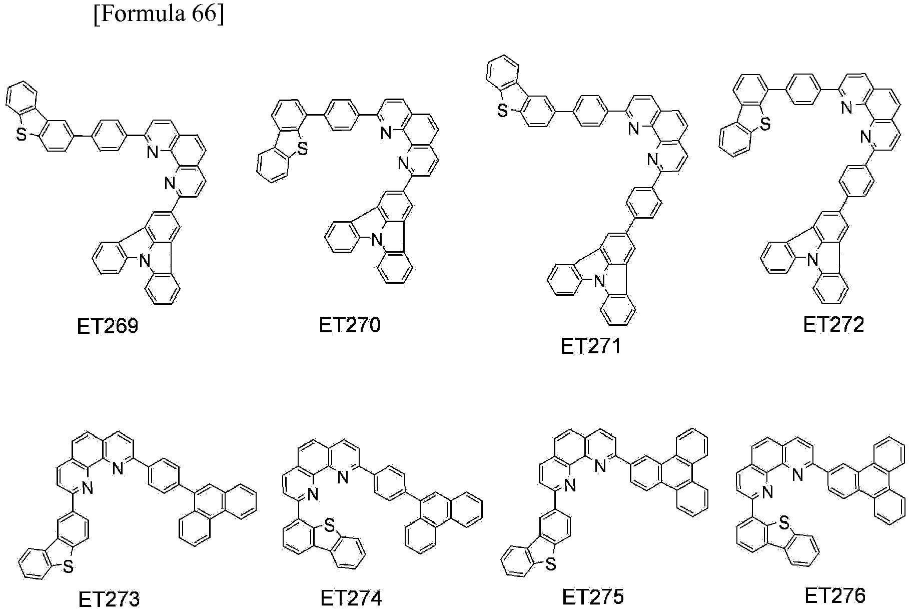

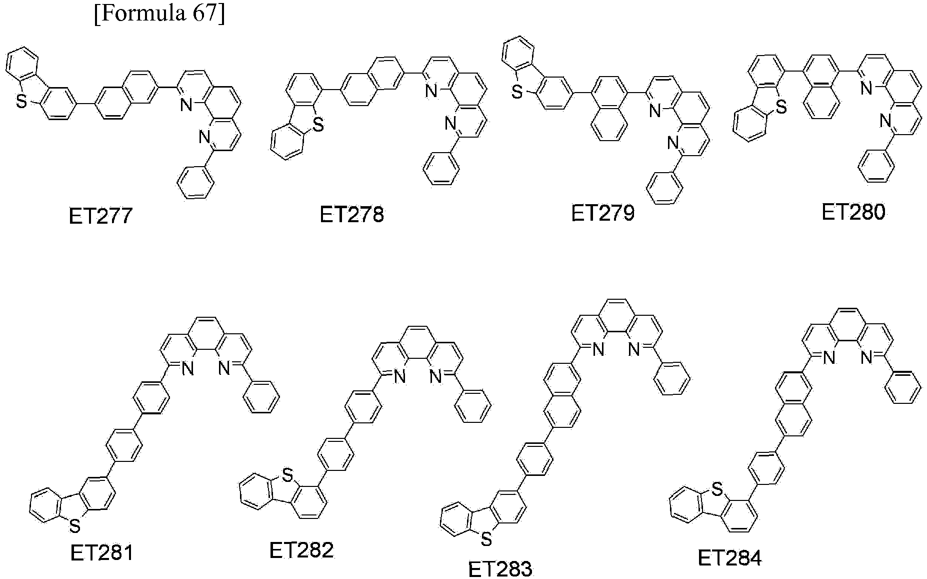

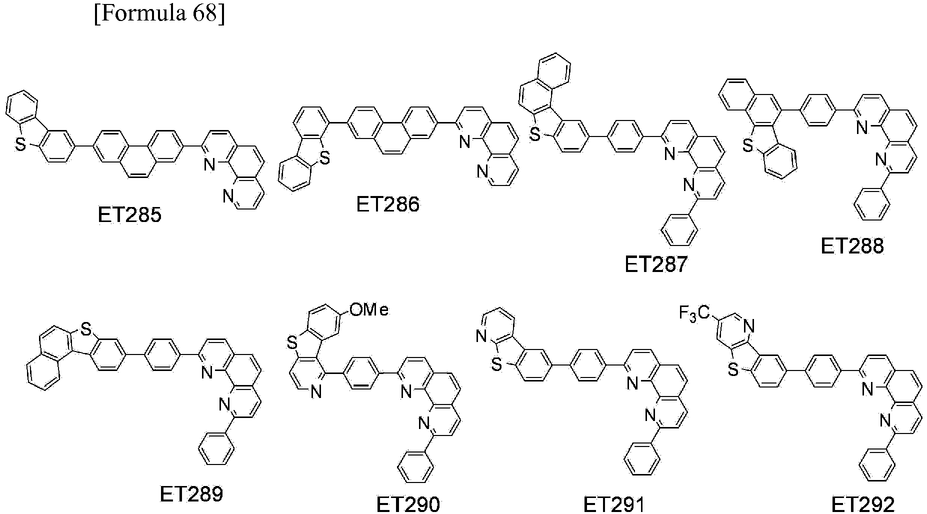

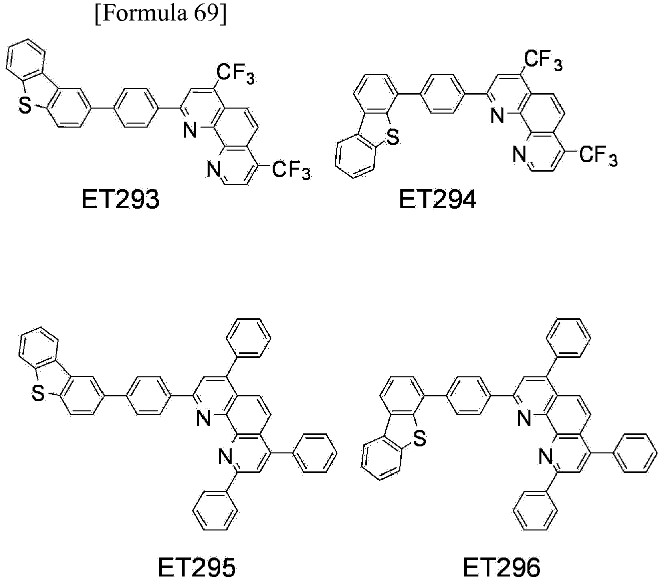

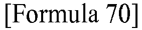

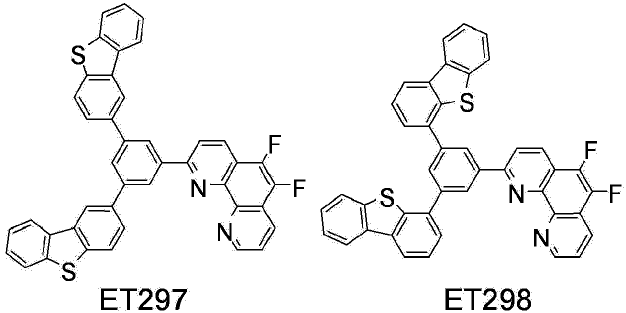

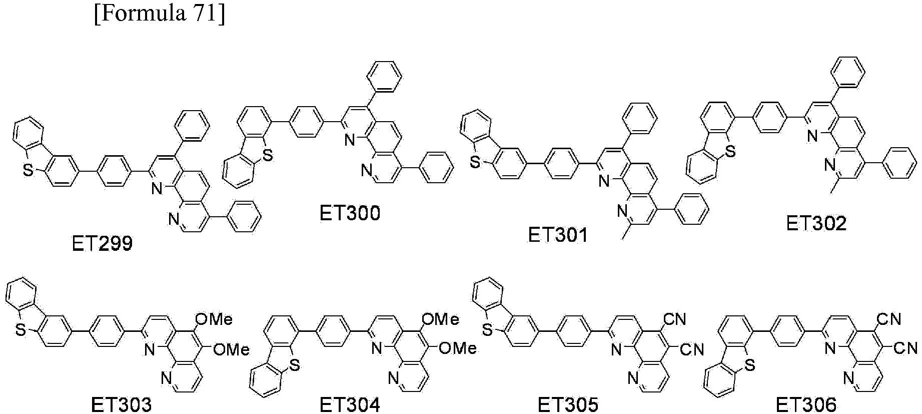

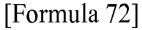

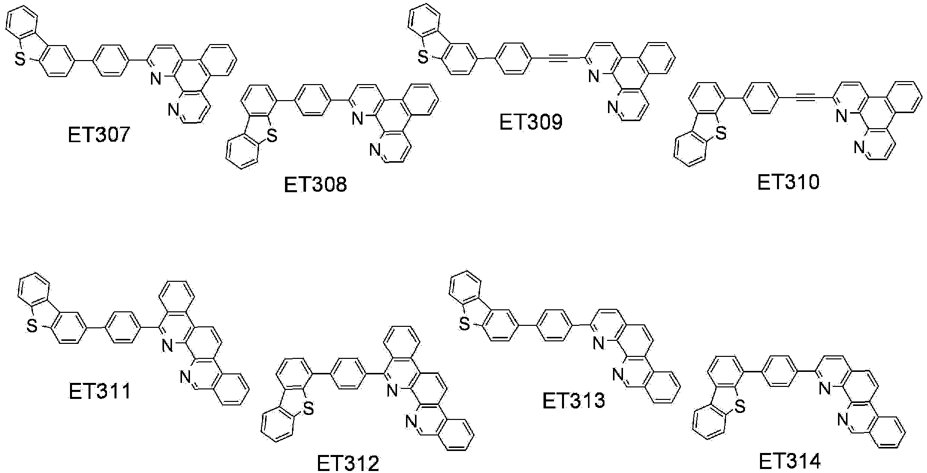

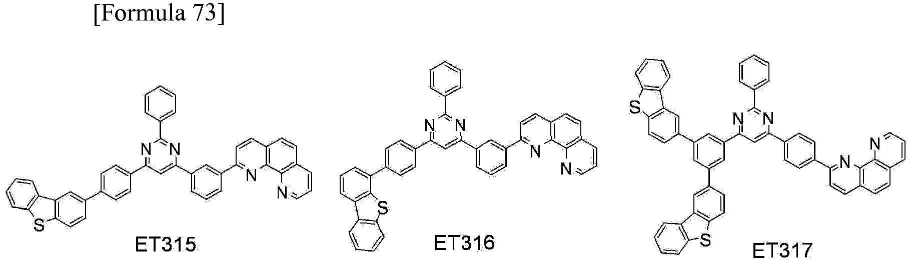

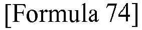

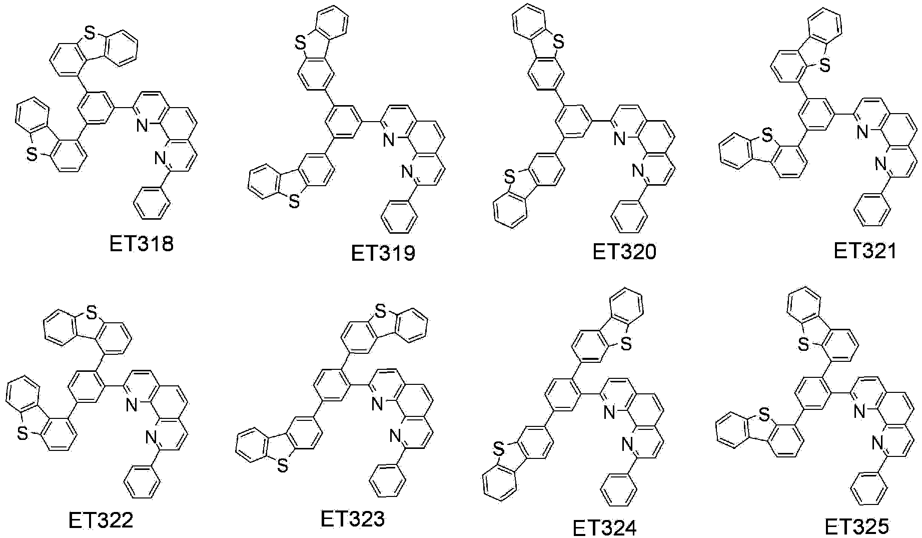

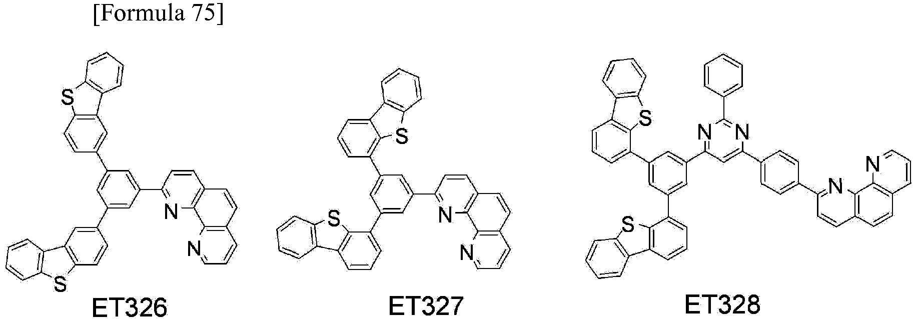

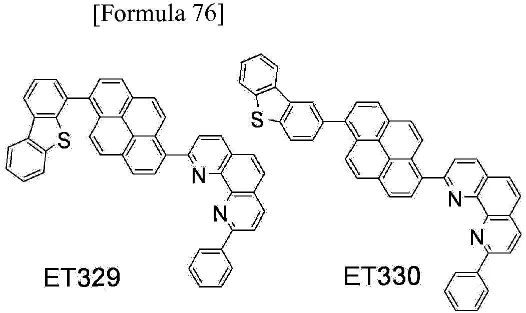

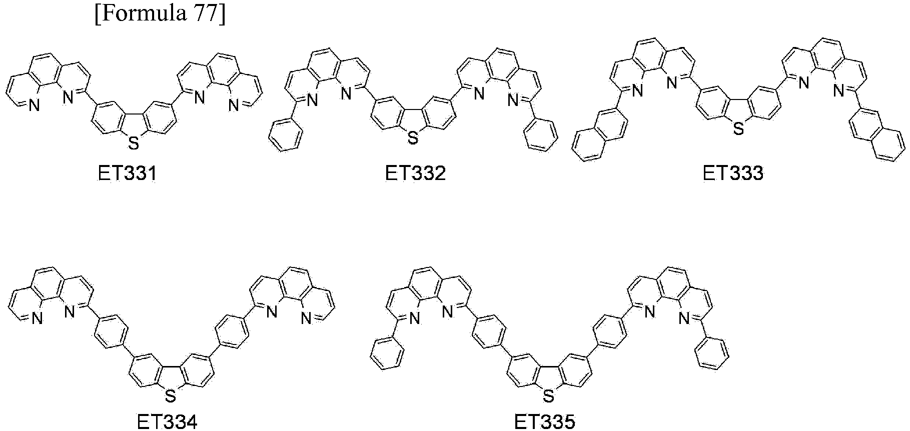

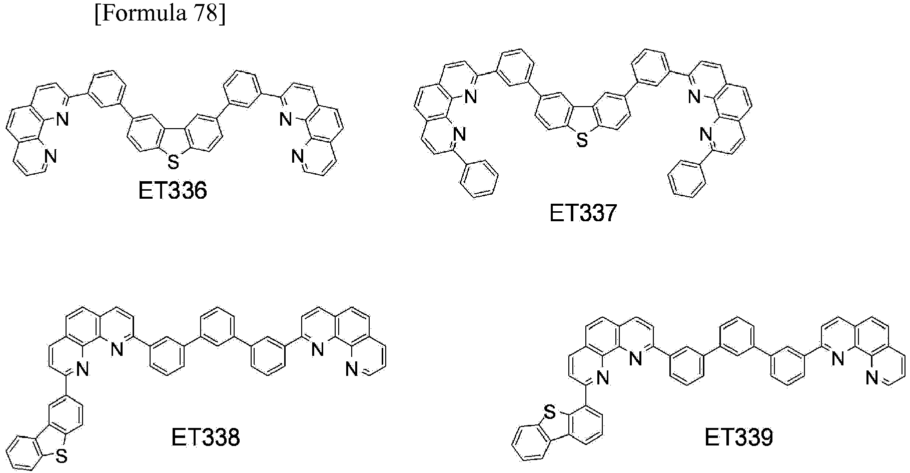

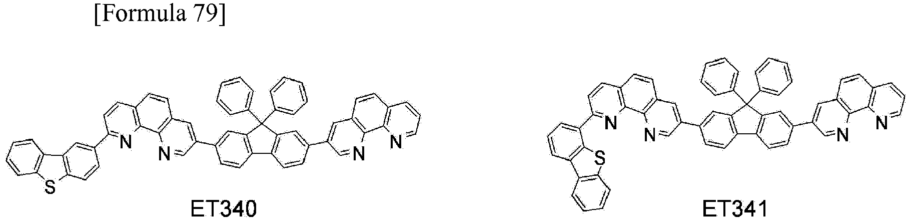

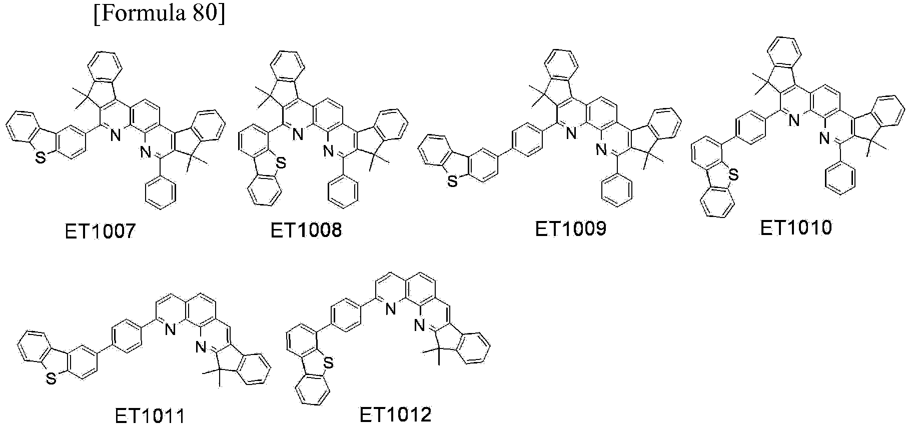

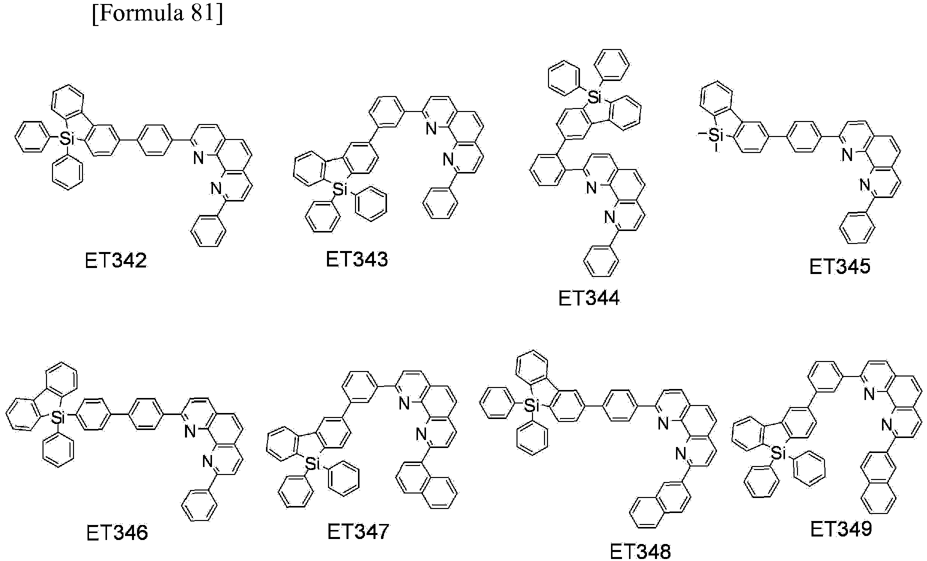

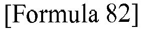

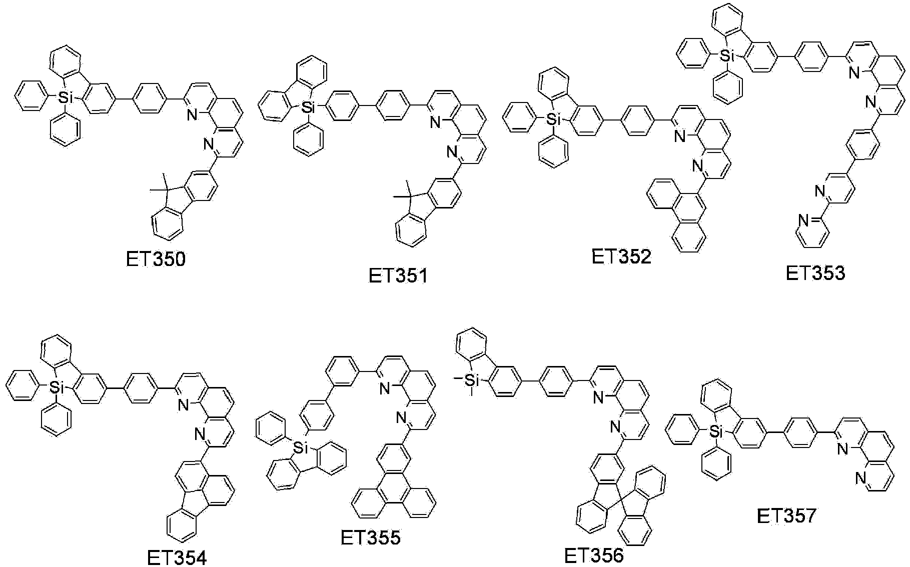

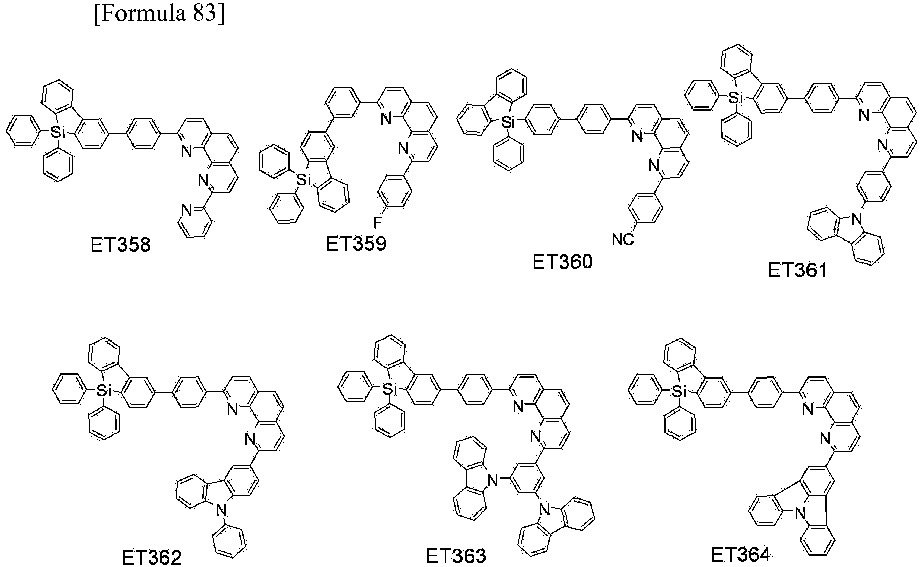

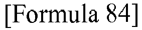

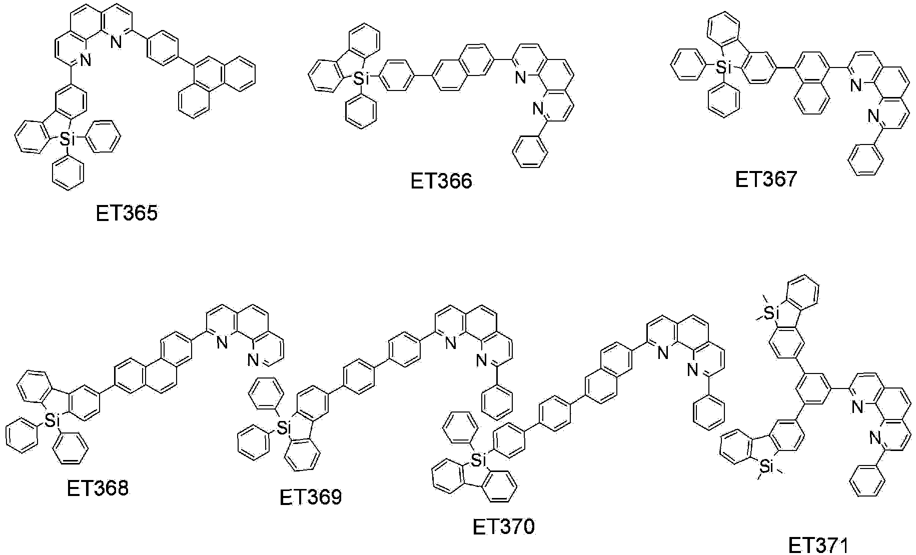

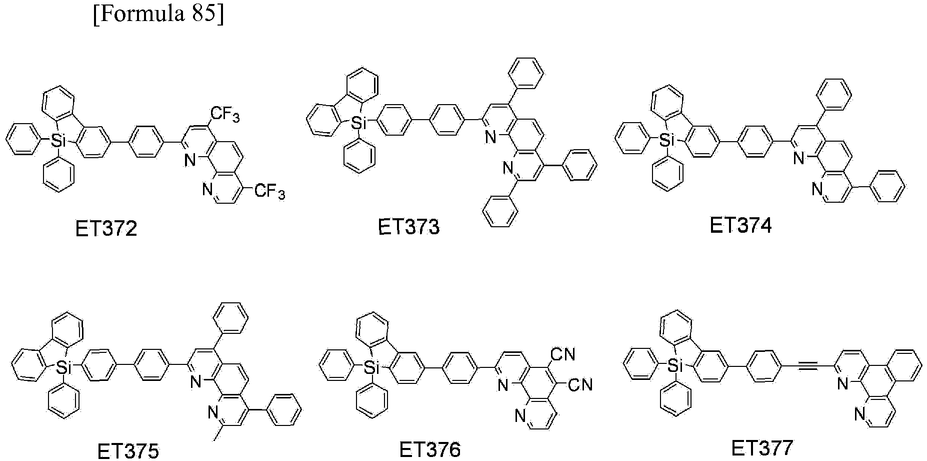

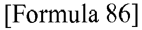

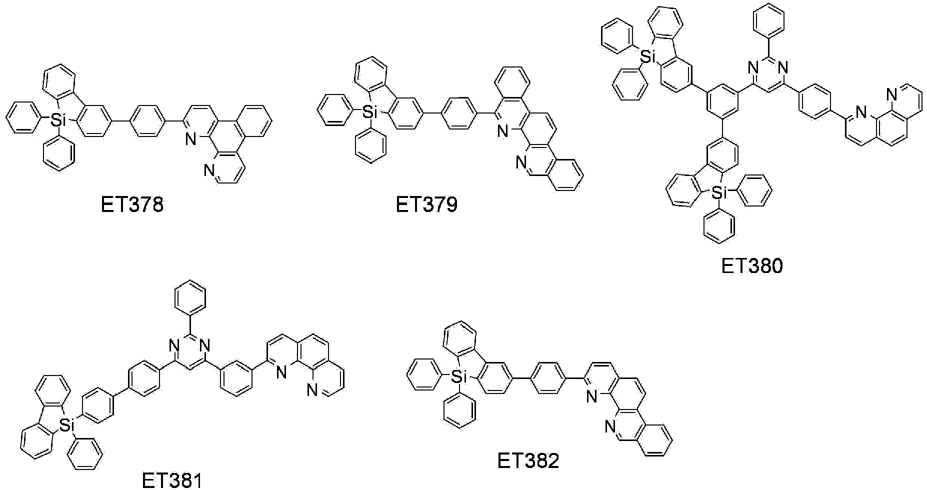

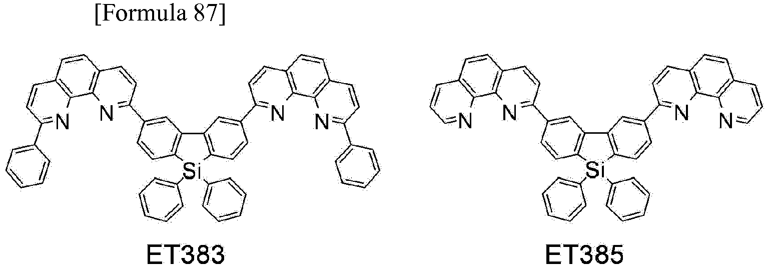

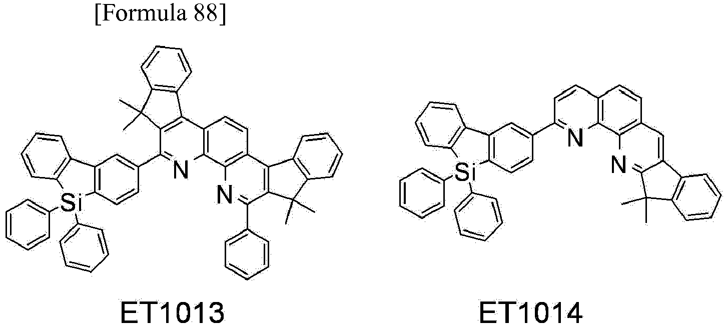

Among the above compounds listed as the examples of the compound represented by the formula (1-7), preferable examples of the compound according to the exemplary embodiment are the compounds ET1 to ET156, ET160 to ET385 and ET1001 to ET1014 represented by the formula (1-7) in which X² to X⁸ are CR^{X}. Further preferable examples of the compound according to the exemplary embodiment are the compounds ET1 to ET156, ET160 to ET341 and ET1001 to ET1012 represented by the formula (1-7) in which X² to X⁸ are CR^{X} and Z is an oxygen atom or a sulfur atom. More preferable examples of the compound according to the exemplary embodiment are the compounds ET1 to ET156, ET160 to ET172 and ET1001 to ET106 represented by the formula (1-7) in which X² to X⁸ are CR^{X} and Z is an oxygen atom.

Examples of the compound represented by the formula (1-8) are as follows.

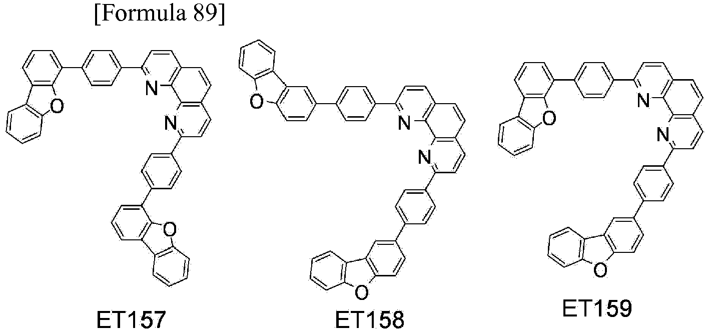

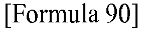

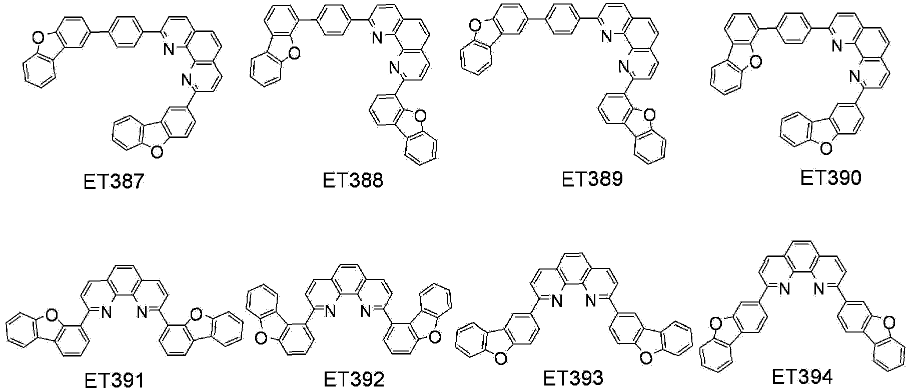

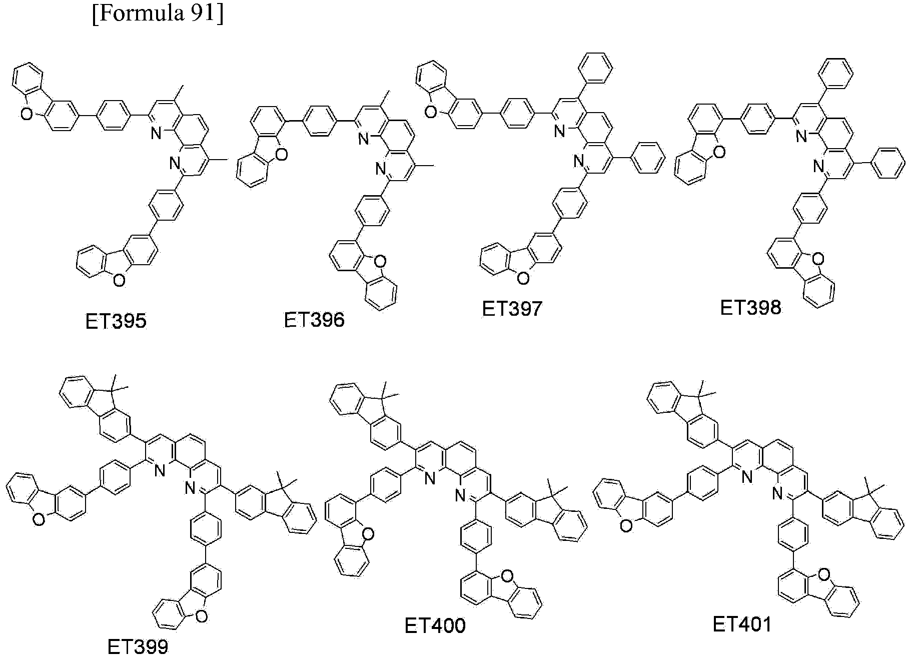

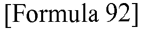

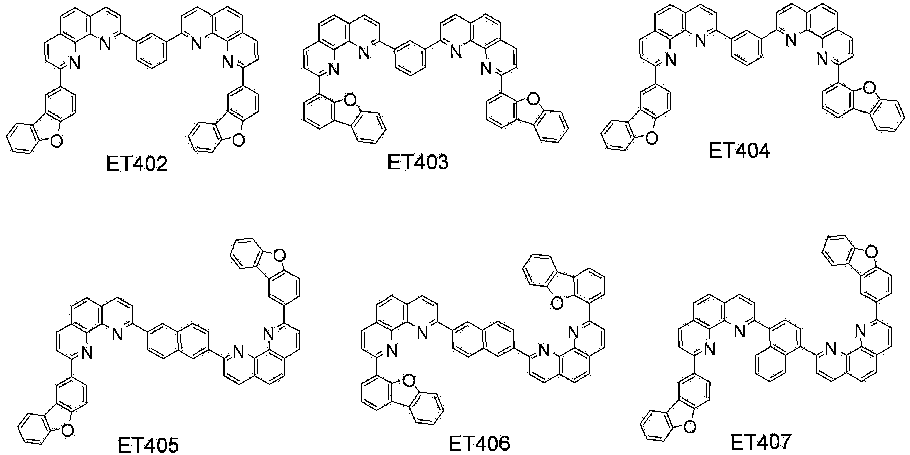

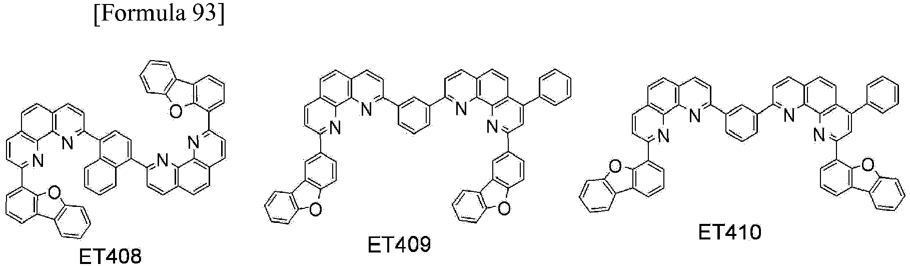

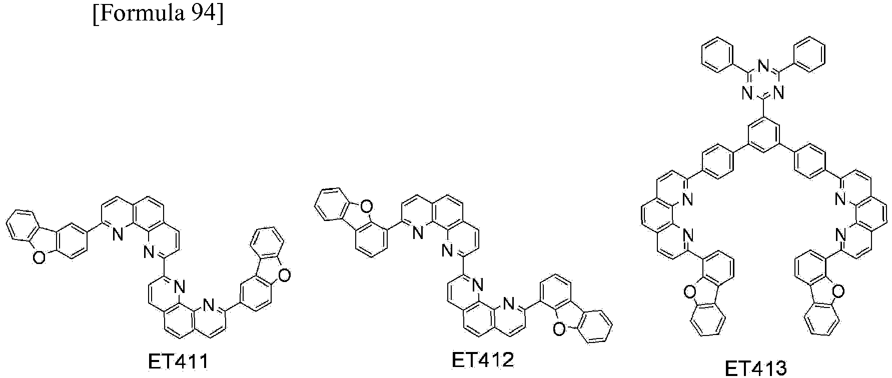

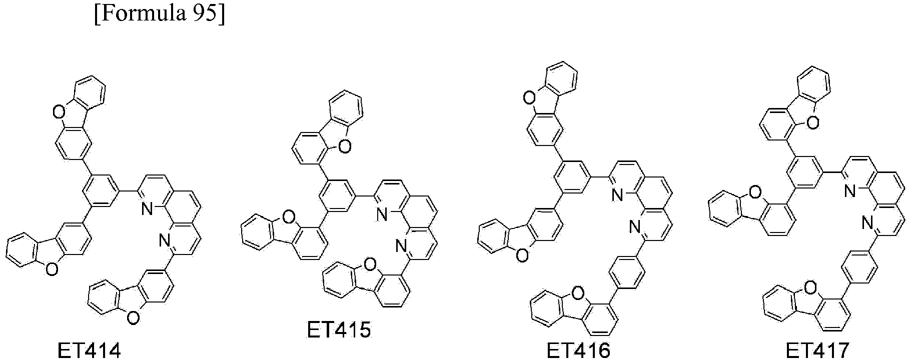

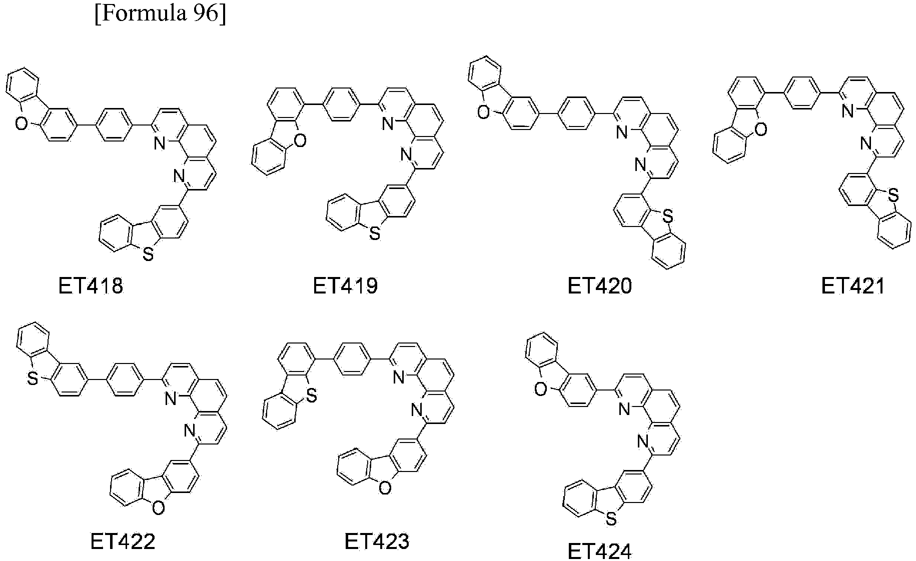

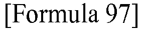

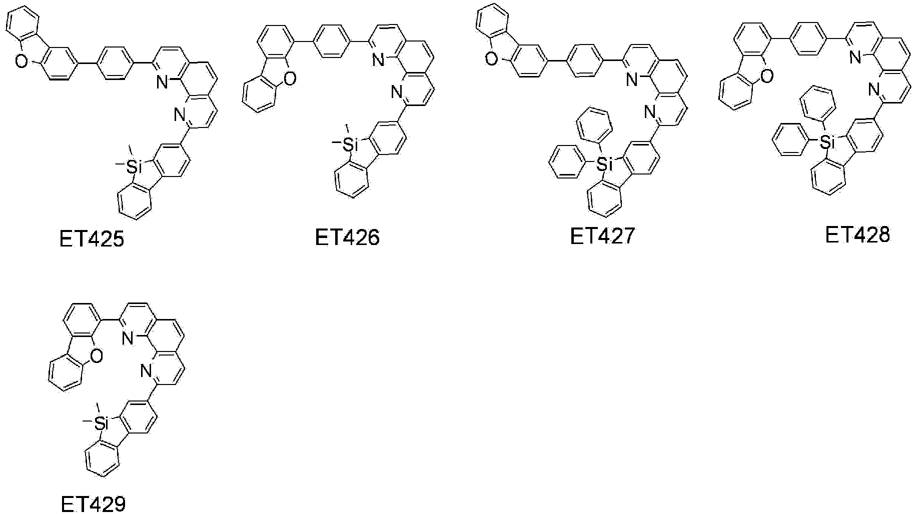

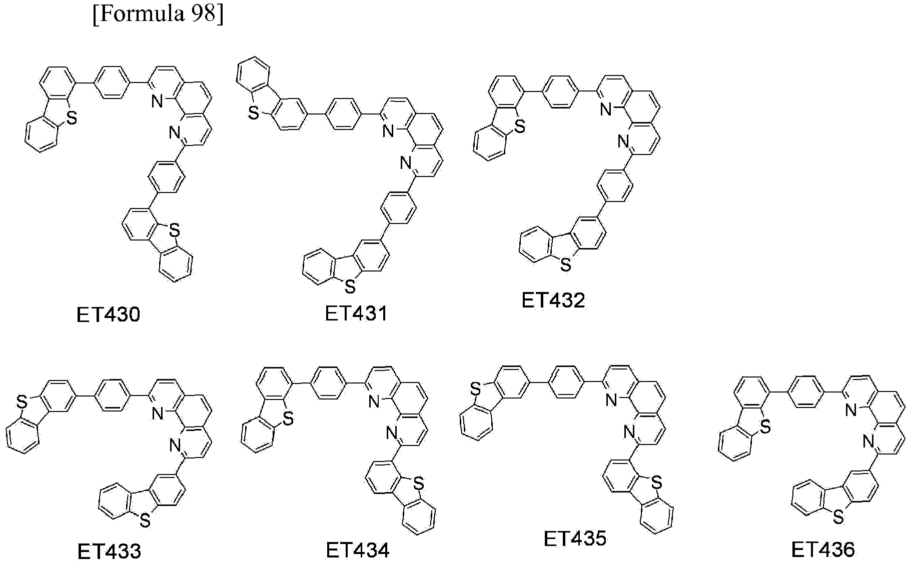

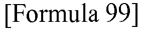

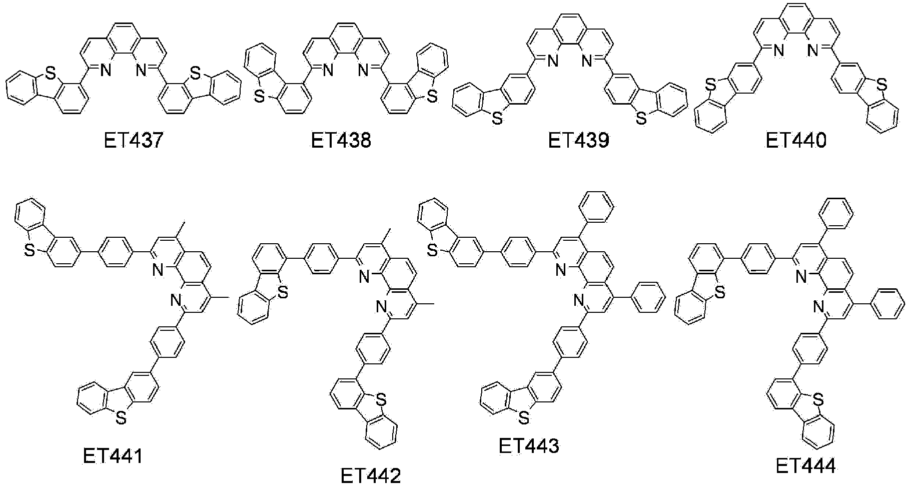

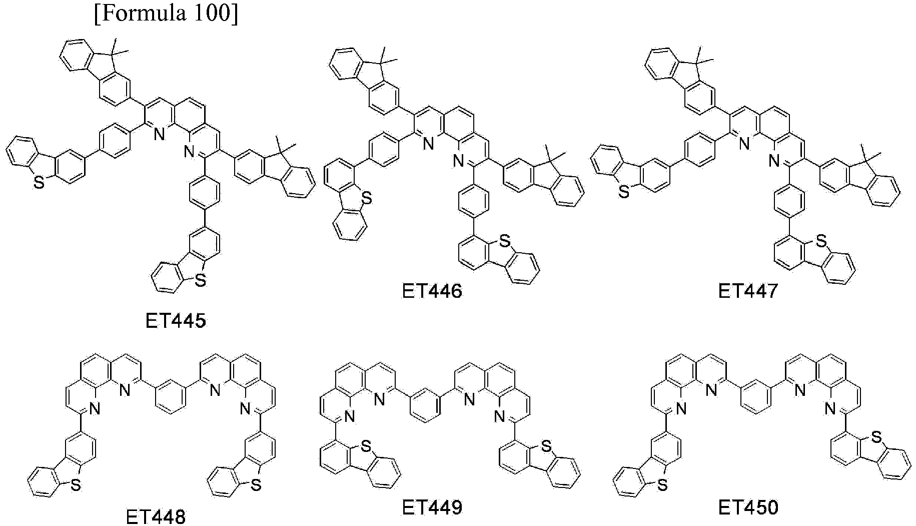

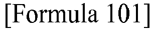

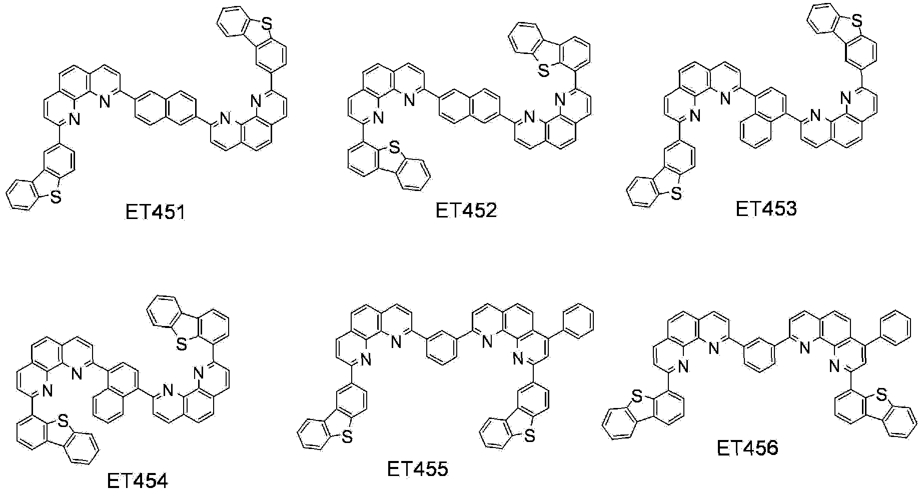

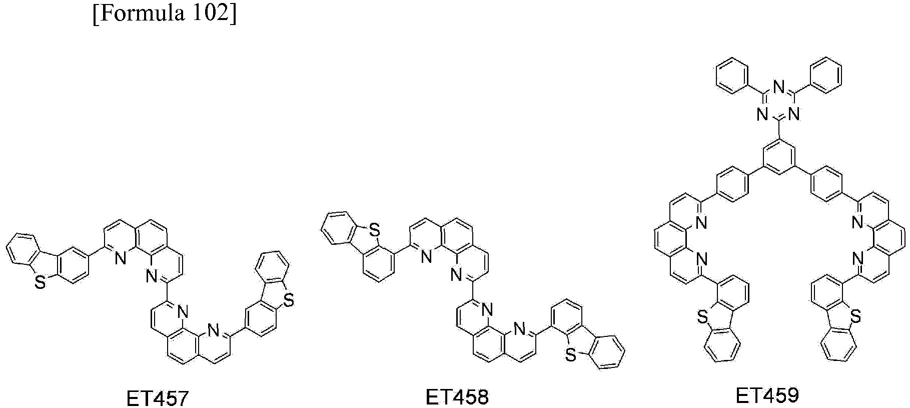

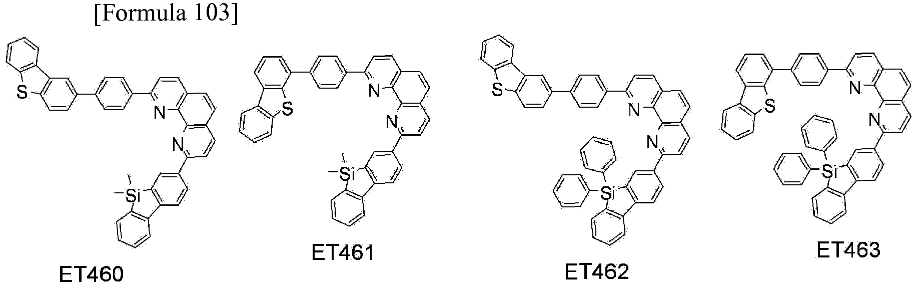

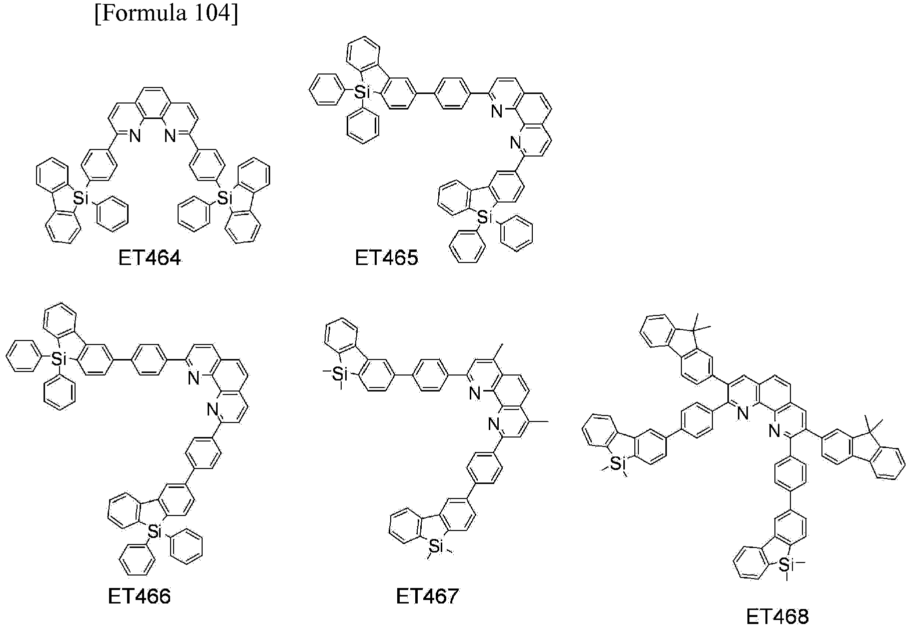

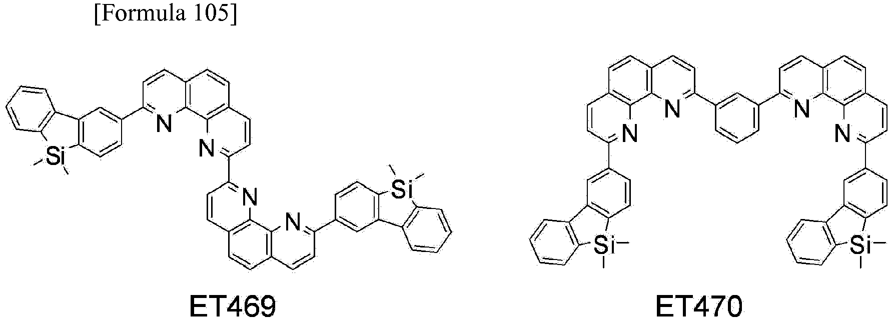

Among the above compounds listed as the examples of the compound represented by the formula (1-8), preferable examples of the compound according to the exemplary embodiment are the compounds ET157 to ET159 and ET387 to ET470 represented by the formula (1-8) in which X² to X⁷ are CR^{X}. Further preferable examples of the compound according to the exemplary embodiment are the compounds ET157 to ET159, ET387 to ET424 and ET430 to ET459 represented by the formula (1-8) in which X² to X⁷ are CR^{X} and Z is an oxygen atom or a sulfur atom. More preferable examples of the compound according to the exemplary embodiment are the compounds ET157 to ET159 and ET387 to ET417 represented by the formula (1-7) in which X² to X⁷ are CR^{X} and Z is an oxygen atom.

Examples of the compound represented by the formula (1-9) are as follows.

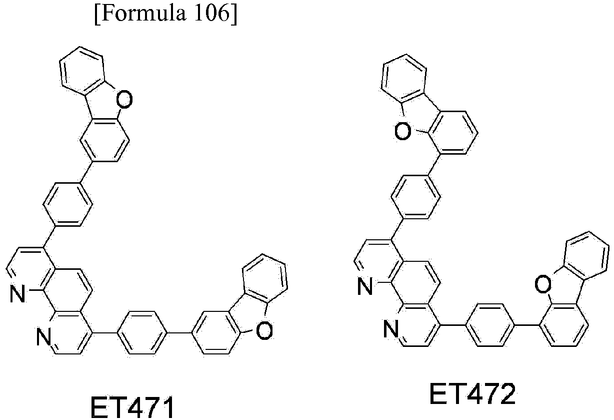

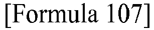

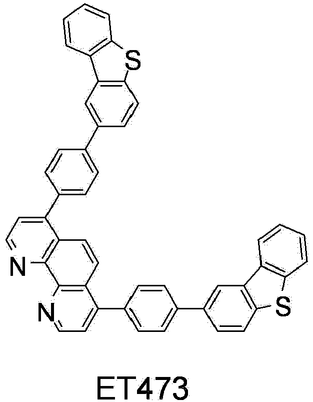

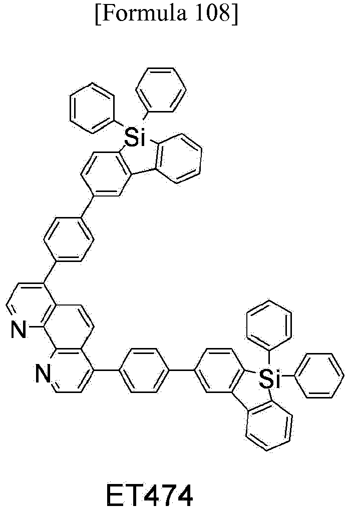

Among the above compounds listed as the examples of the compound represented by the formula (1-9), preferable examples of the compound according to the exemplary embodiment are the compounds ET471 to ET474 represented by the formula (1-9) in which X¹, X², X⁴, X⁵, X⁷ and X⁸ are CR^{X}. Further preferable examples of the compound according to the exemplary embodiment are the compounds ET471 to ET473 represented by the formula (1-9) in which X¹, X², X⁴, X⁵, X⁷ and X⁸ are CR^{X} and Z is an oxygen atom or a sulfur atom. More preferable examples of the compound according to the exemplary embodiment are the compounds ET471 to ET472 represented by the formula (1-9) in which X¹, X², X⁴, X⁵, X⁷ and X⁸ are CR^{X} and Z is an oxygen atom.

Examples of the compound represented by the formula (1-10) are as follows.

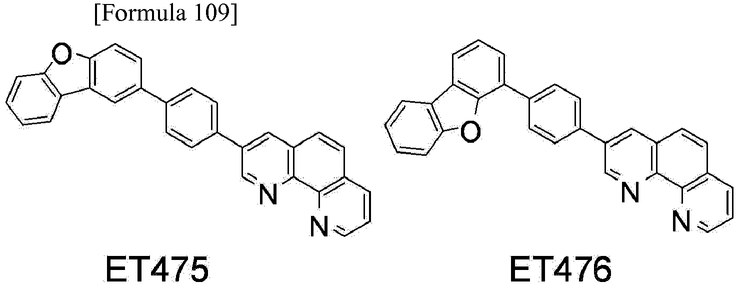

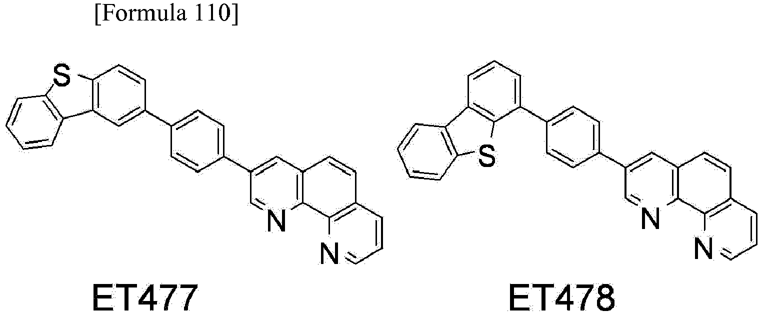

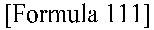

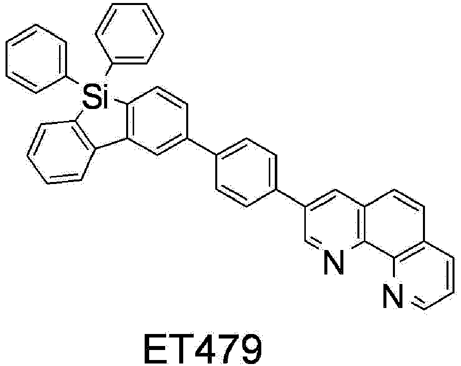

Among the above compounds listed as the examples of the compound represented by the formula (1-10), preferable examples of the compound according to the exemplary embodiment are the compounds ET475 to ET479 represented by the formula (1-10) in which X¹ and X³ to X⁸ are CR^{X}. Further preferable examples of the compound according to the exemplary embodiment are the compounds ET475 to ET478 represented by the formula (1-10) in which X¹ X³ to X⁸ are CR^{X} and Z is an oxygen atom or a sulfur atom. More preferable examples of the compound according to the exemplary embodiment are the compounds ET475 to ET476 represented by the formula (1-10) in which X¹, X³ to X⁸ are CR^{X} and Z is an oxygen atom.

Examples of the compound represented by the formula (1-11) are as follows.

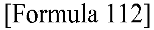

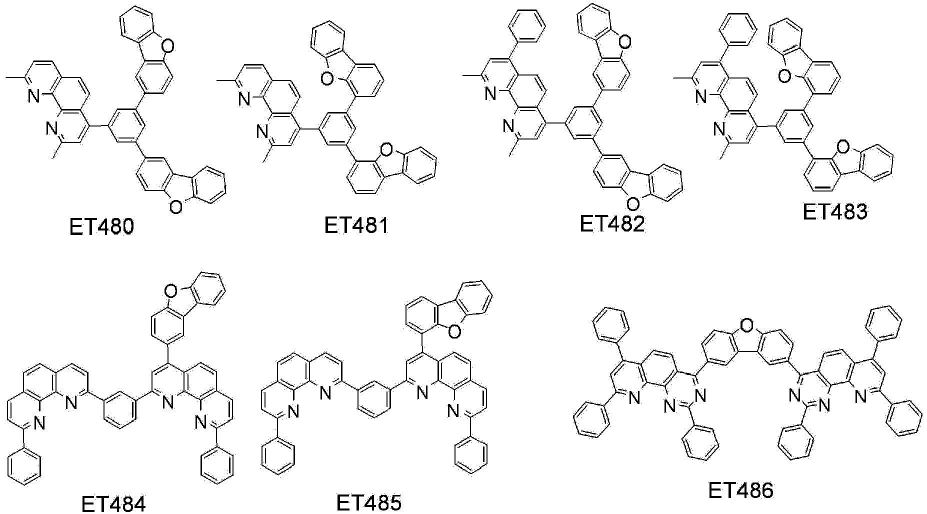

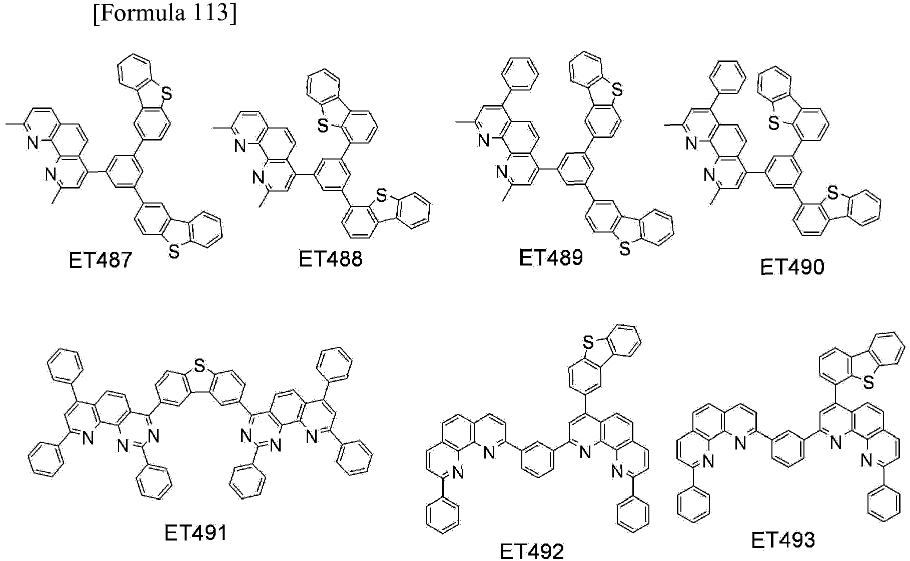

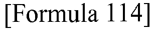

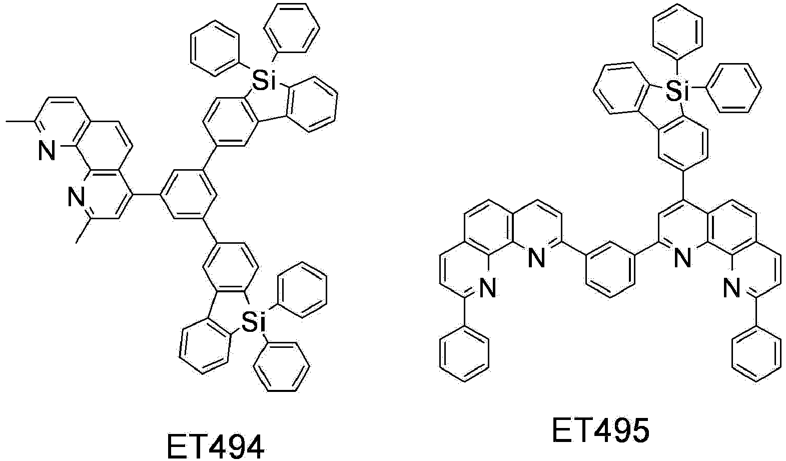

Among the above compounds listed as the examples of the compound represented by the formula (1-11), preferable examples of the compound according to the exemplary embodiment are the compounds ET480 to ET485, ET487 to ET490 and ET492 to ET495 represented by the formula (1-11) in which X¹, X² and X⁴ to X⁸ are CR^{X}. Further preferable examples of the compound according to the exemplary embodiment are the compounds ET480 to ET485, ET487 to ET490 and ET492 to ET493 represented by the formula (1-11) in which X¹, X² and X⁴ to X⁸ are CR^{X} and Z is an oxygen atom or a sulfur atom. More preferable examples of the compound according to the exemplary embodiment are the compounds ET480 to ET485 represented by the formula (1-11) in which X¹, X² and X⁴ to X⁸ are CR^{X} and Z is an oxygen atom.

Examples of the compound represented by the formula (1-12) are as follows.

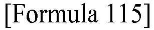

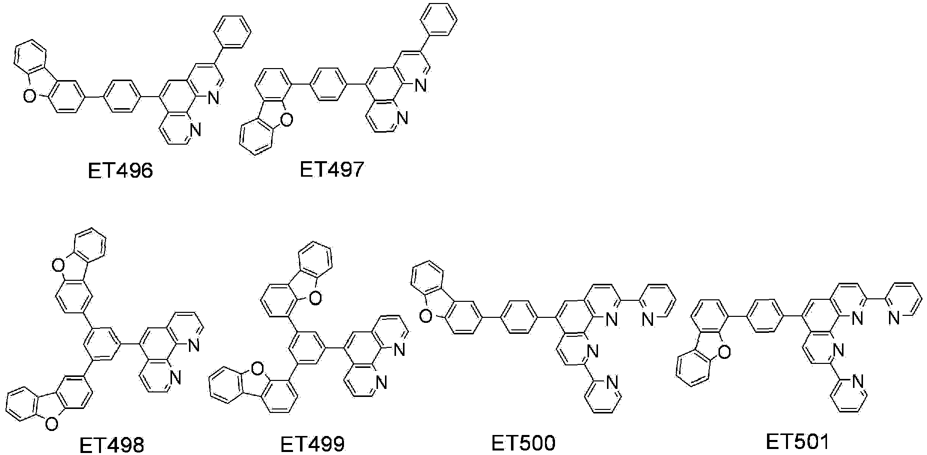

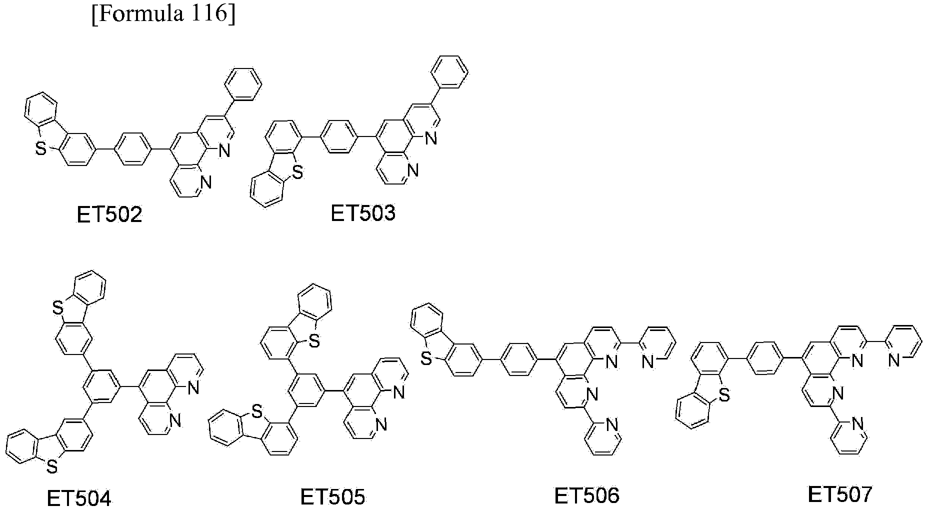

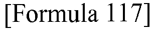

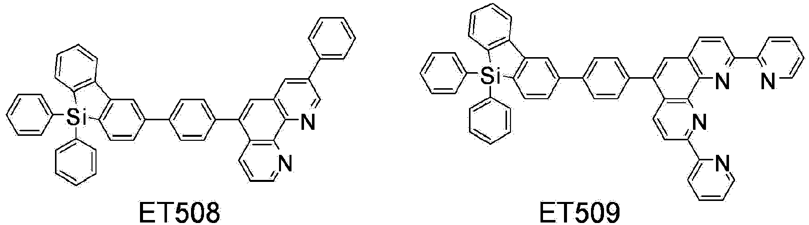

Among the above compounds listed as the examples of the compound represented by the formula (1-12), preferable examples of the compound according to the exemplary embodiment are the compounds ET496 to ET509 represented by the formula (1-12) in which X¹ to X³ and X⁵ to X⁸ are CR^{X}. Further preferable examples of the compound according to the exemplary embodiment are the compounds ET496 to ET507 represented by the formula (1-12) in which X¹ to X³ and X⁵ to X⁸ are CR^{X} and Z is an oxygen atom or a sulfur atom. More preferable examples of the compound according to the exemplary embodiment are the compounds ET496 to ET501 represented by the formula (1-12) in which X¹ to X³ and X⁵ to X⁸ are CR^{X} and Z is an oxygen atom.

Examples of the compound represented by the formula (1-13) are as follows.

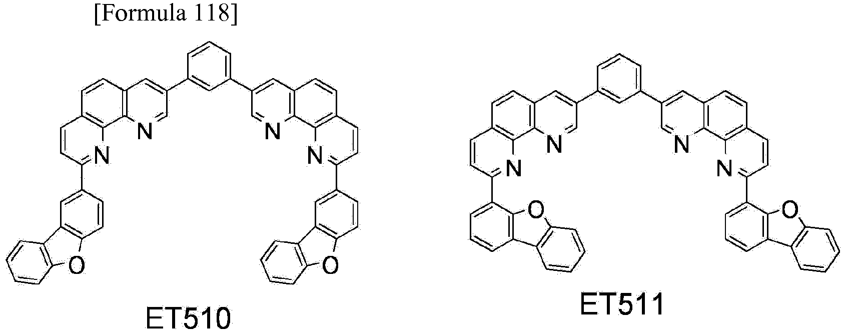

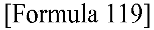

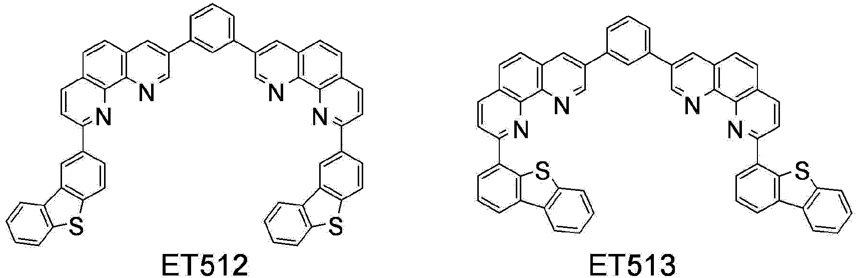

Among the above compounds listed as the examples of the compound represented by the formula (1-13), preferable examples of the compound according to the exemplary embodiment are the compounds ET510 to ET513 represented by the formula (1-13) in which X² to X⁶ and X⁸ are CR^{X}. Further preferable examples of the compound according to the exemplary embodiment are the compounds ET510 to ET513 represented by the formula (1-13) in which X² to X⁶ and X⁸ are CR^{X} and Z is an oxygen atom or a sulfur atom. More preferable examples of the compound according to the exemplary embodiment are the compounds ET510 to ET511 represented by the formula (1-13) in which X² to X⁶ and X⁸ are CR^{X} and Z is an oxygen atom.

Examples of the compound represented by the formula (1-14) are as follows.

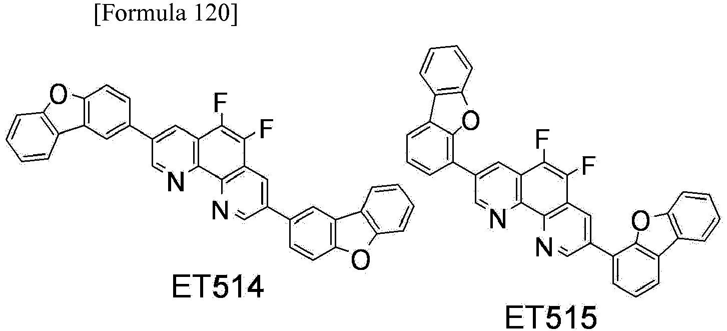

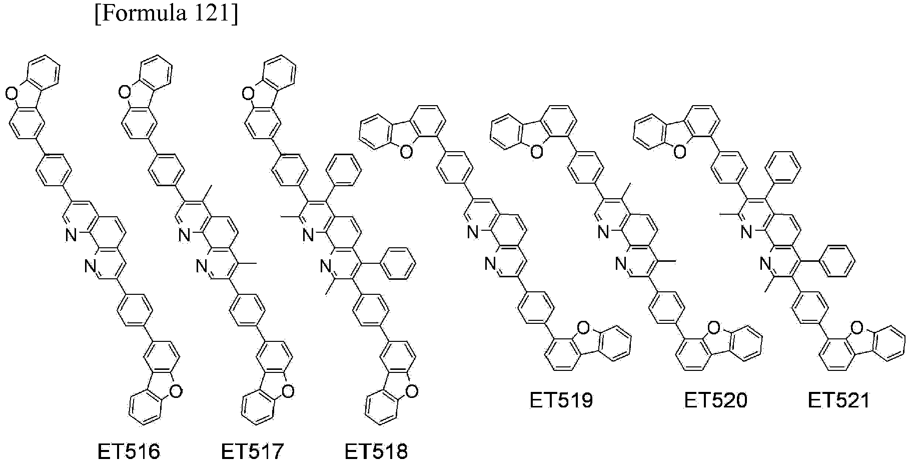

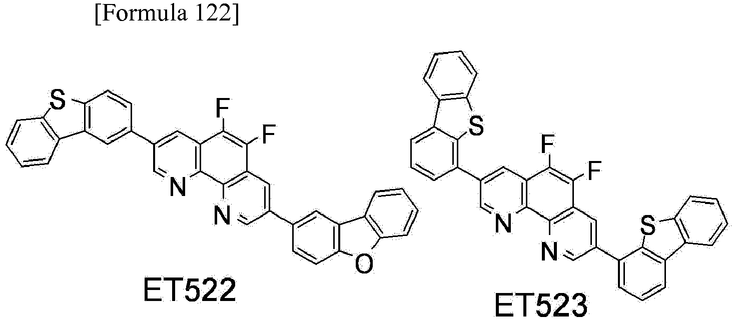

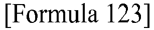

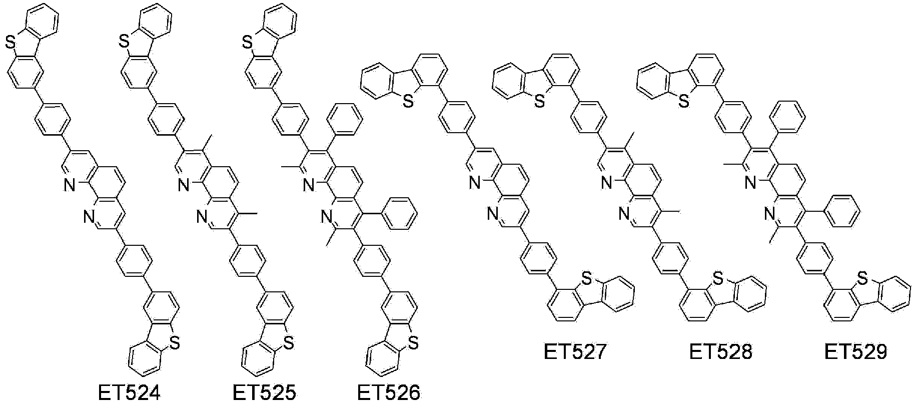

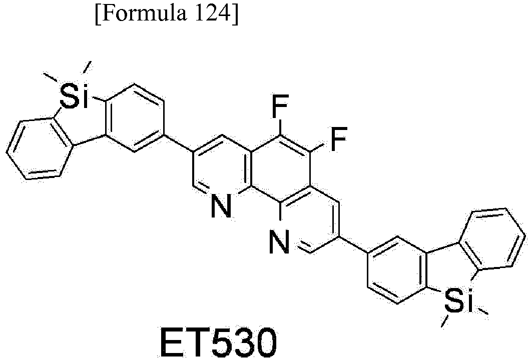

Among the above compounds listed as the examples of the compound represented by the formula (1-14), preferable examples of the compound according to the exemplary embodiment are the compounds ET514 to ET530 represented by the formula (1-14) in which X¹, X³ to X⁶ and X⁸ are CR^{X}. Further preferable examples of the compound according to the exemplary embodiment are the compounds ET514 to ET529 represented by the formula (1-14) in which X¹, X³ to X⁶ and X⁸ are CR^{X} and Z is an oxygen atom or a sulfur atom. More preferable examples of the compound according to the exemplary embodiment are the compounds ET514 to ET521 represented by the formula (1-14) in which X¹, X³ to X⁶ and X⁸ are CR^{X} and Z is an oxygen atom.

Examples of the compound represented by the formula (1-15) are as follows.

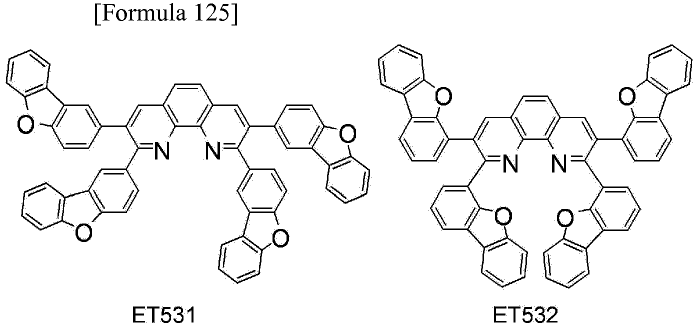

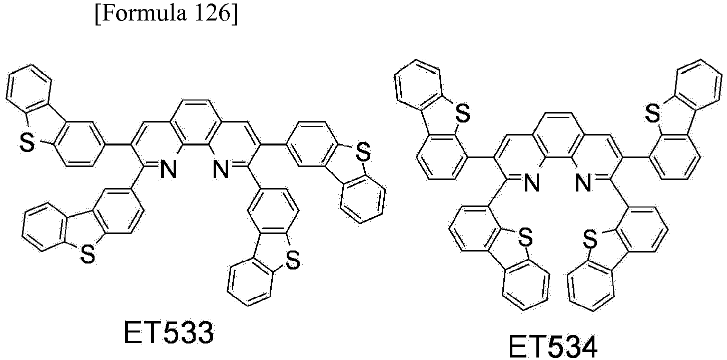

Among the above compounds listed as the examples of the compound represented by the formula (1-15), preferable examples of the compound according to the exemplary embodiment are the compounds ET531 to ET534 represented by the formula (1-15) in which X³ to X⁶ are CR^{X}. Further preferable examples of the compound according to the exemplary embodiment are the compounds ET531 to ET534 represented by the formula (1-15) in which X³ to X⁶ are CR^{X} and Z is an oxygen atom or a sulfur atom. More preferable examples of the compound according to the exemplary embodiment are the compounds ET531 to ET532 represented by the formula (1-15) in which X³ to X⁶ are CR^{X} and Z is an oxygen atom.

Examples of the compound represented by the formula (1-16) are as follows.

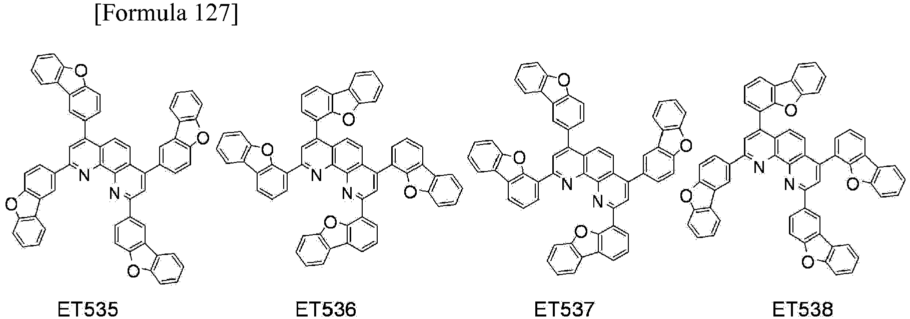

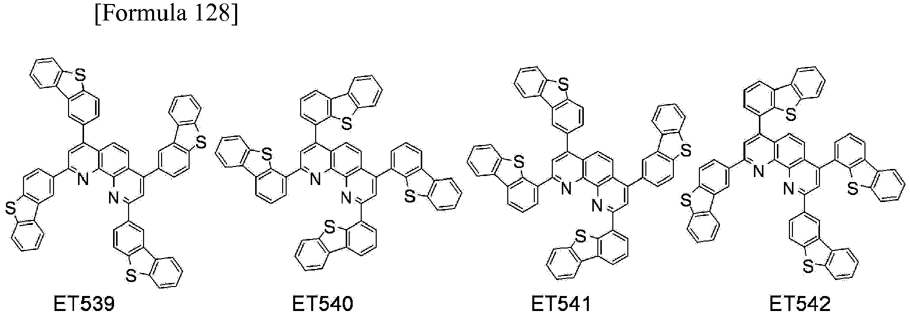

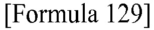

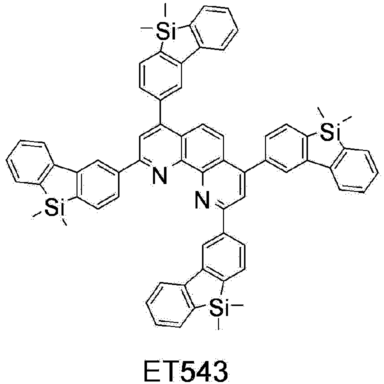

Among the above compounds listed as the examples of the compound represented by the formula (1-16), preferable examples of the compound according to the exemplary embodiment are the compounds ET535 to ET543 represented by the formula (1-16) in which X², X⁴, X⁵ and X⁷ are CR^{X}. Further preferable examples of the compound according to the exemplary embodiment are the compounds ET535 to ET542 represented by the formula (1-16) in which X², X⁴, X⁵ and X⁷ are CR^{X} and Z is an oxygen atom or a sulfur atom. More preferable examples of the compound according to the exemplary embodiment are the compounds ET535 to ET538 represented by the formula (1-16) in which X², X⁴, X⁵ and X⁷ are CR^{X} and Z is an oxygen atom.

Further, specific examples of the compound represented by the formula (1) are shown below, but the invention is not limited to thereto.

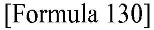

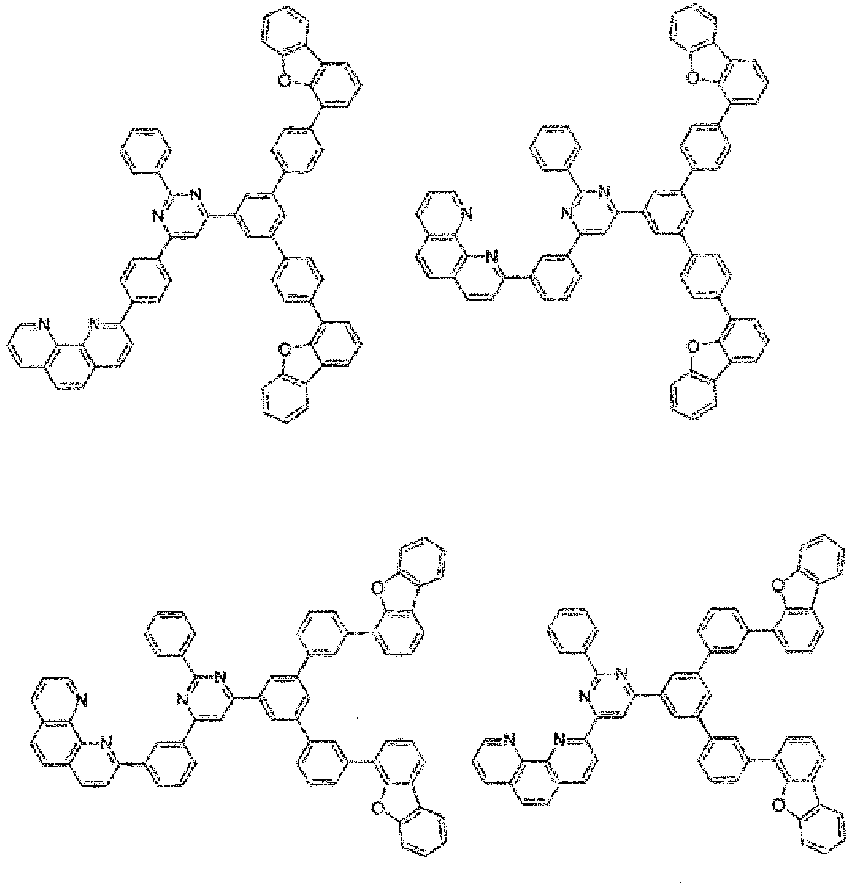

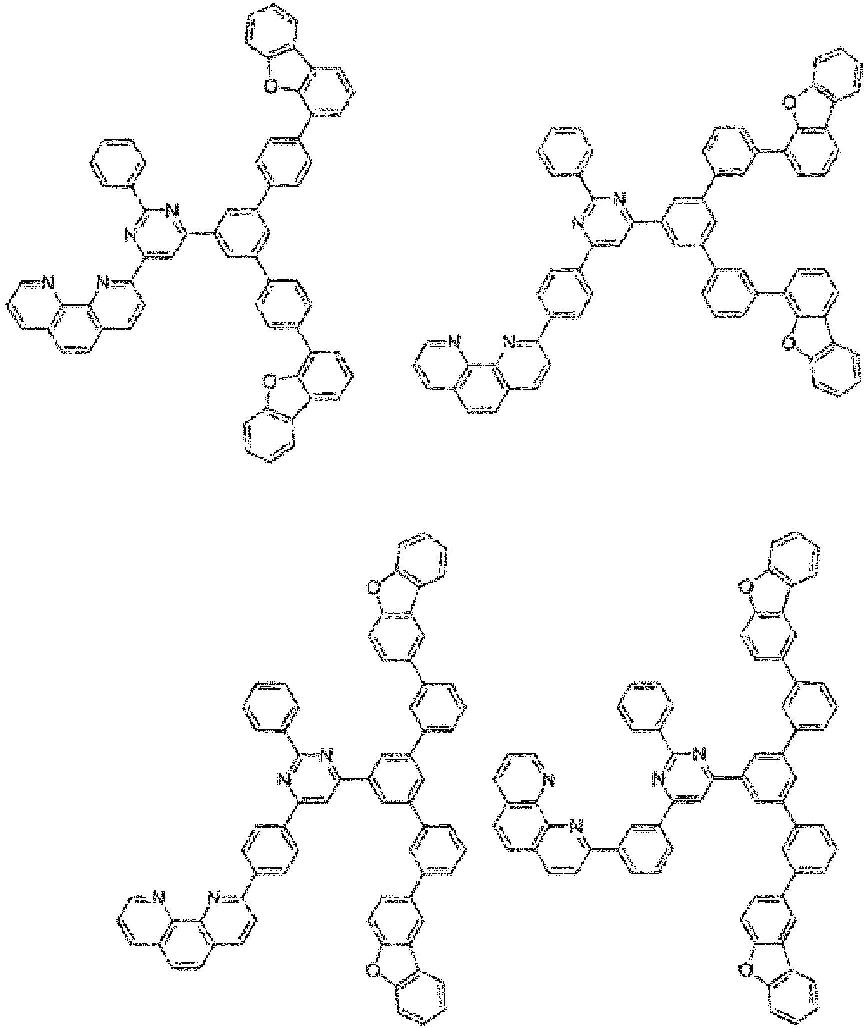

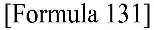

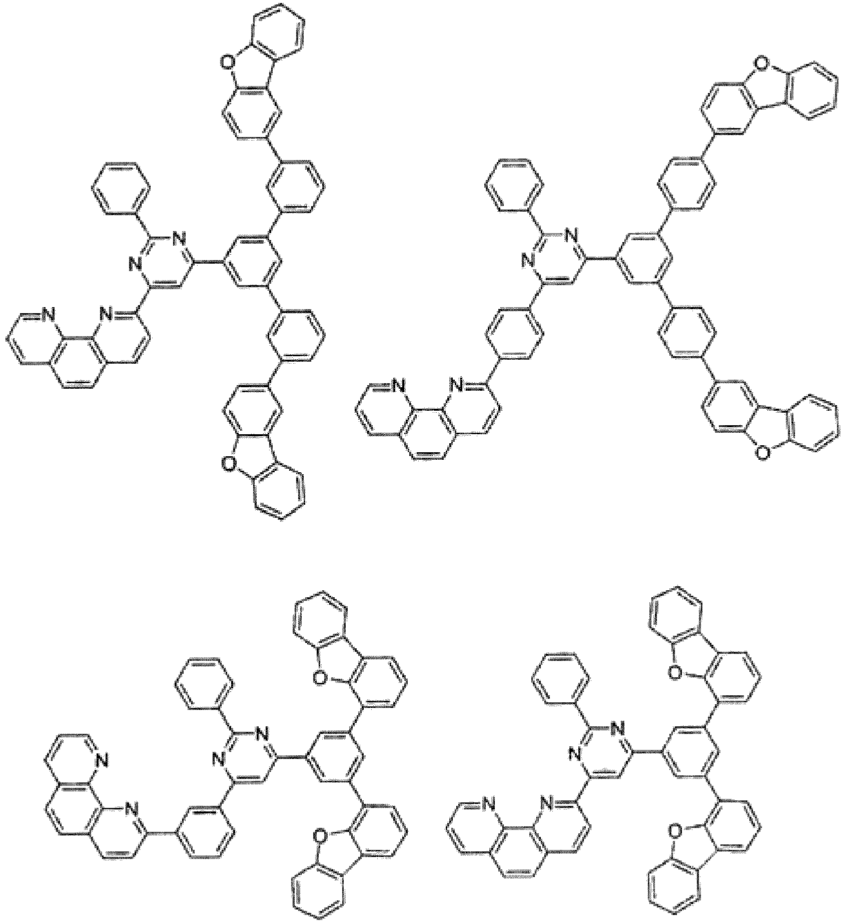

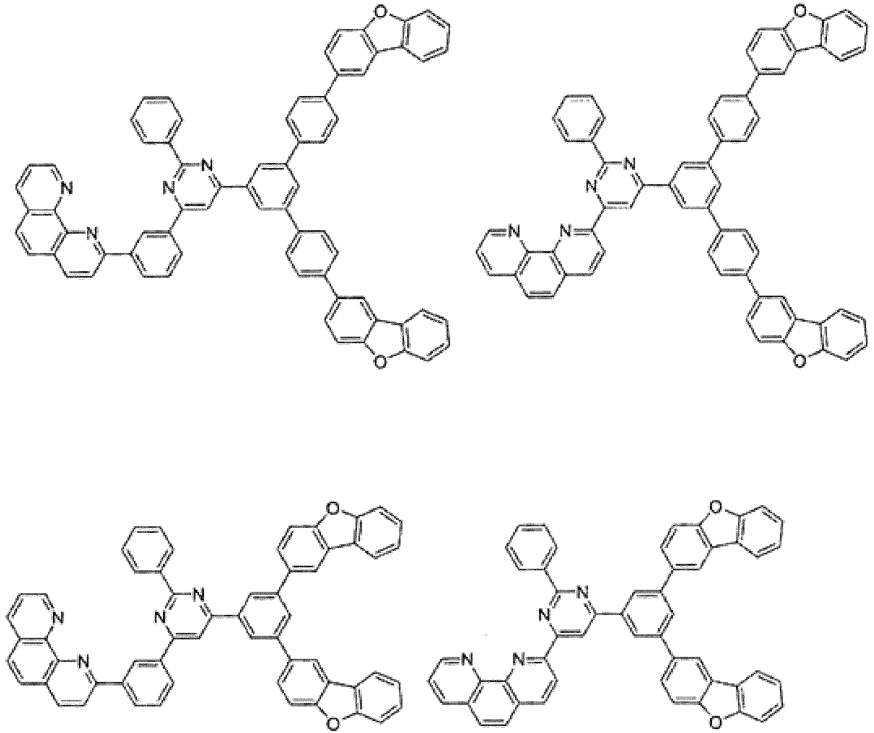

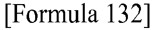

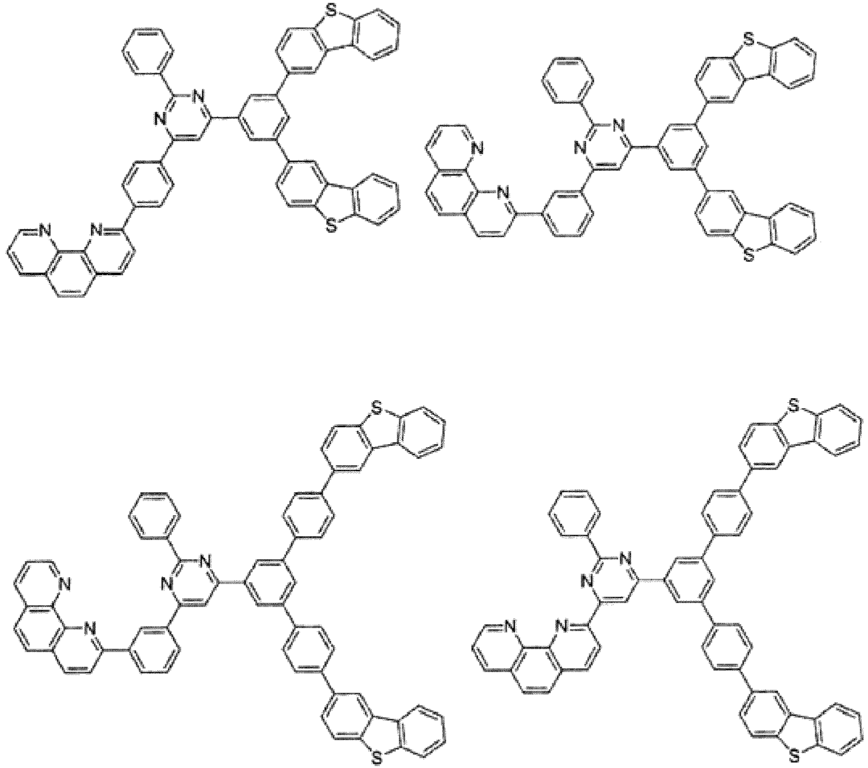

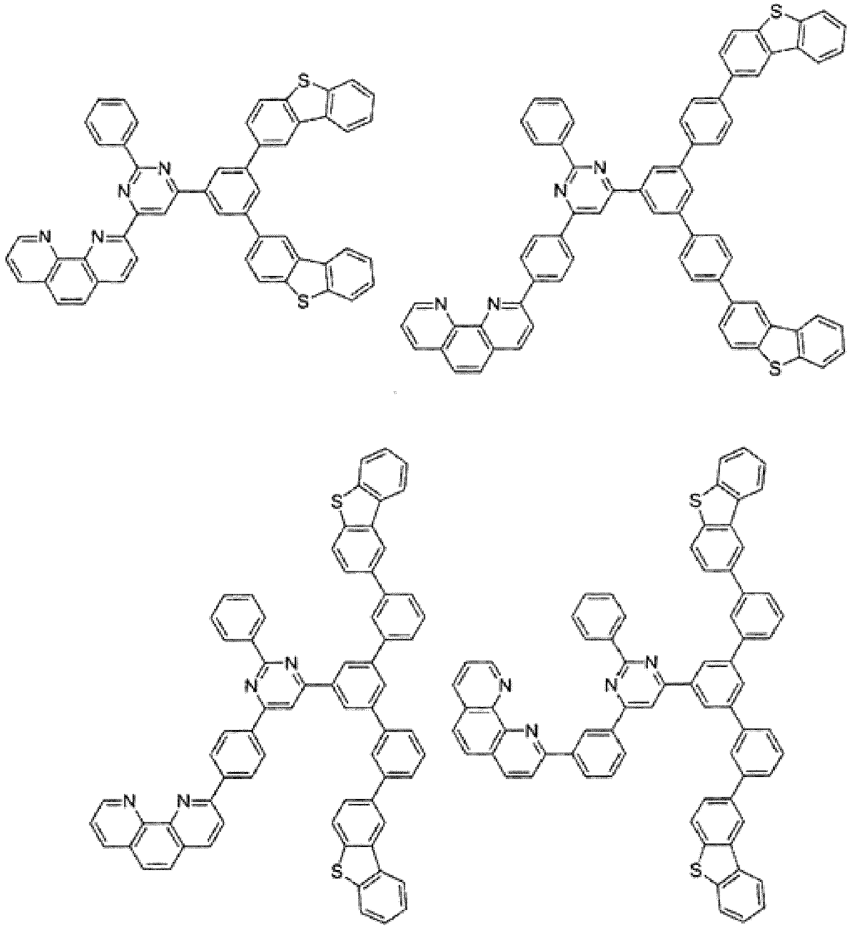

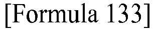

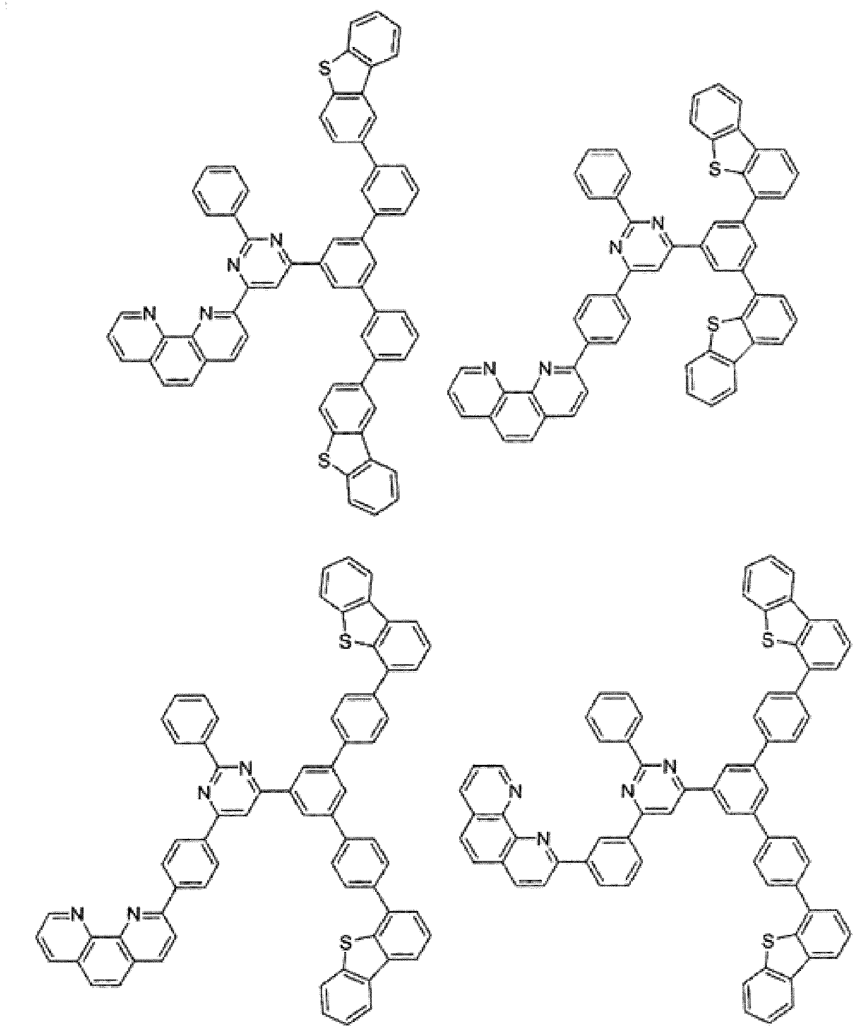

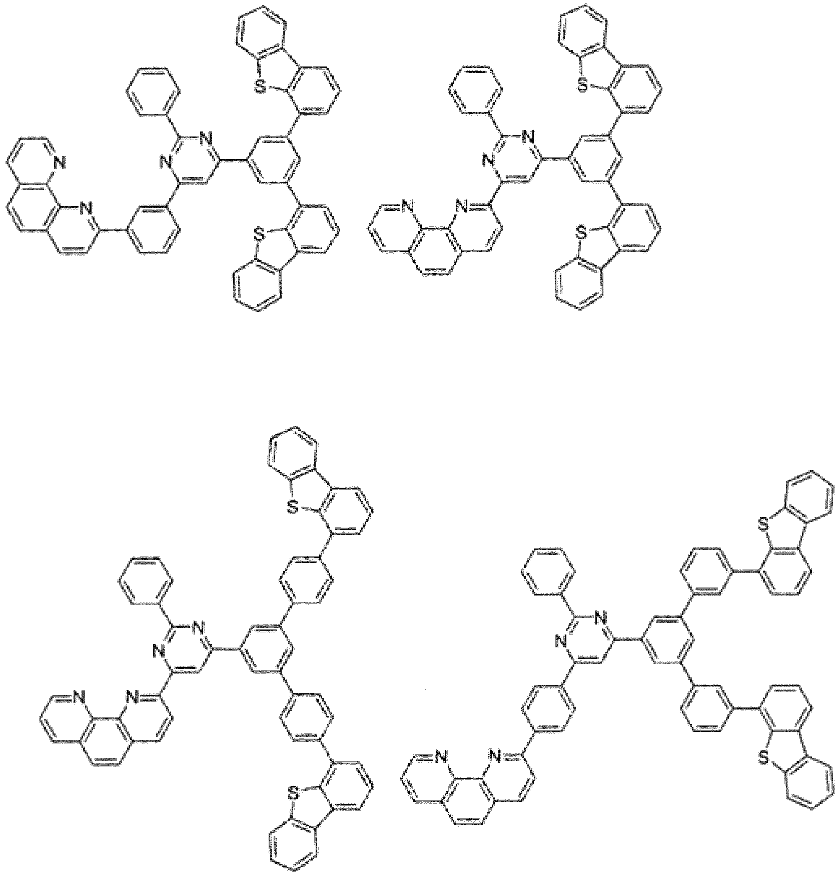

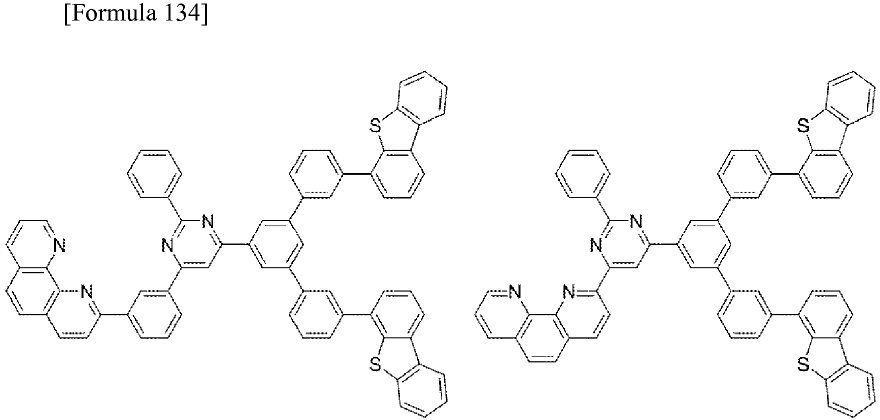

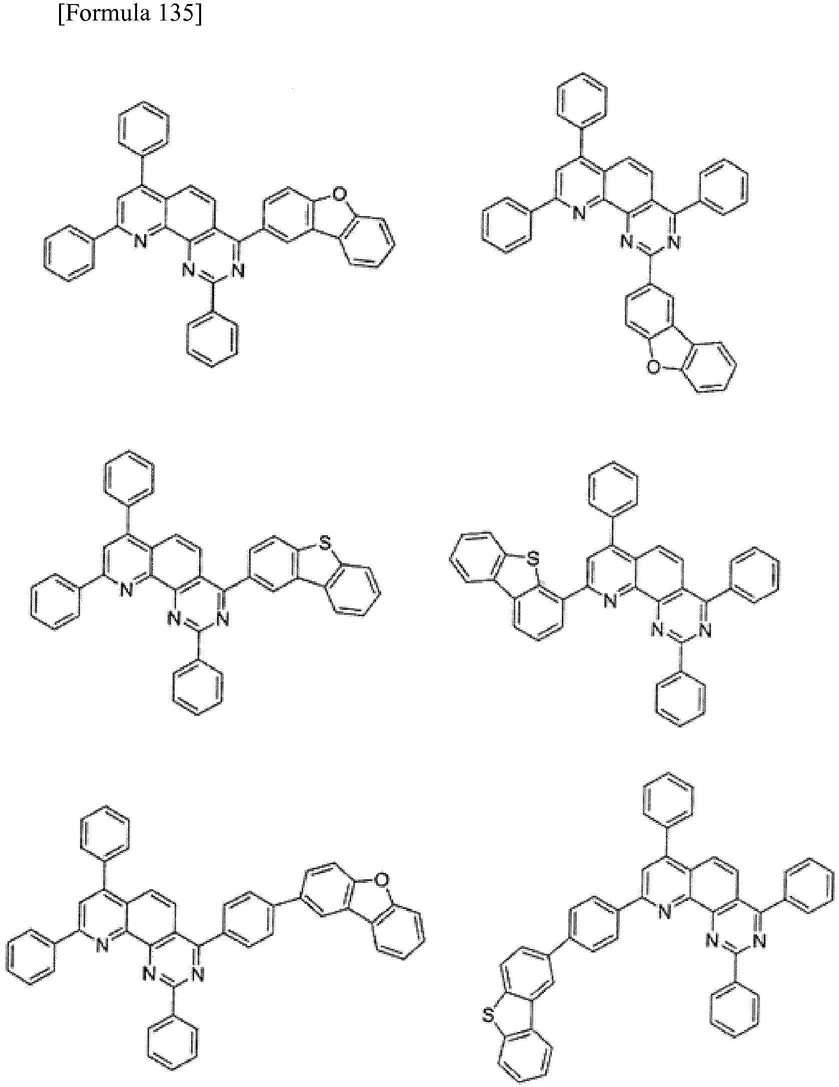

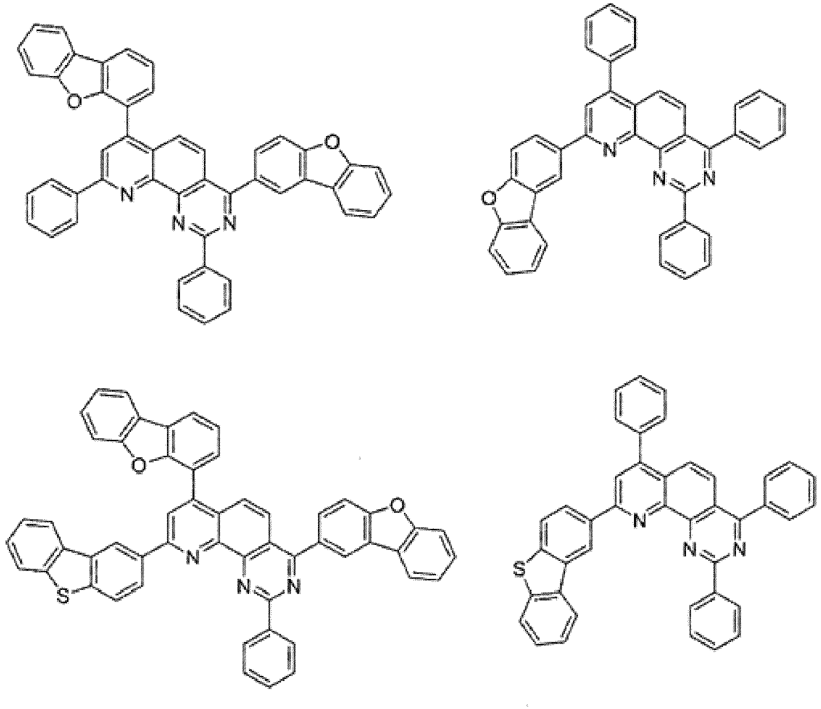

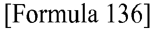

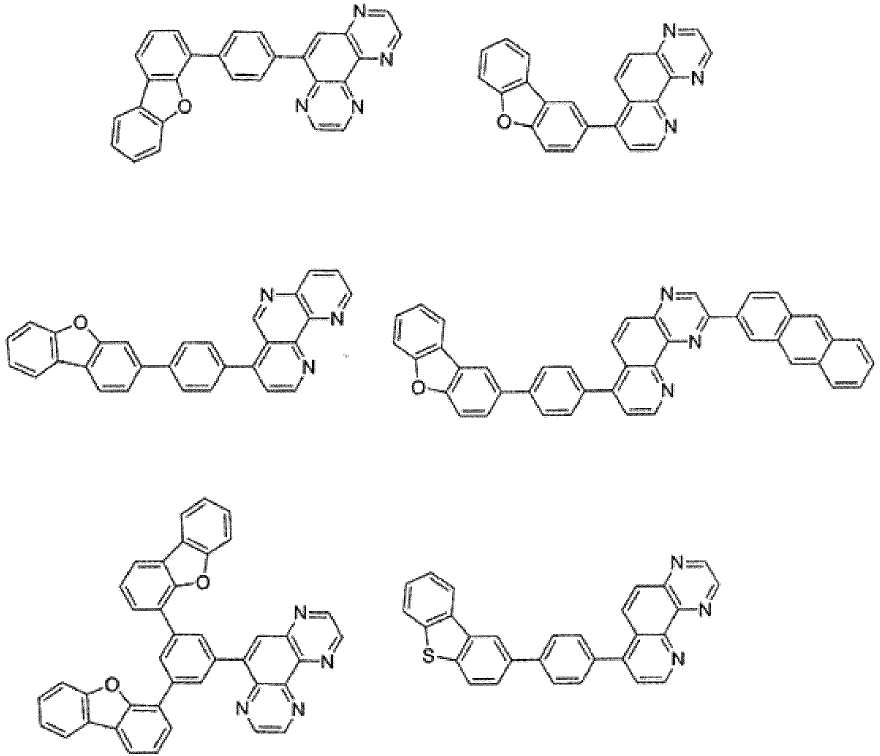

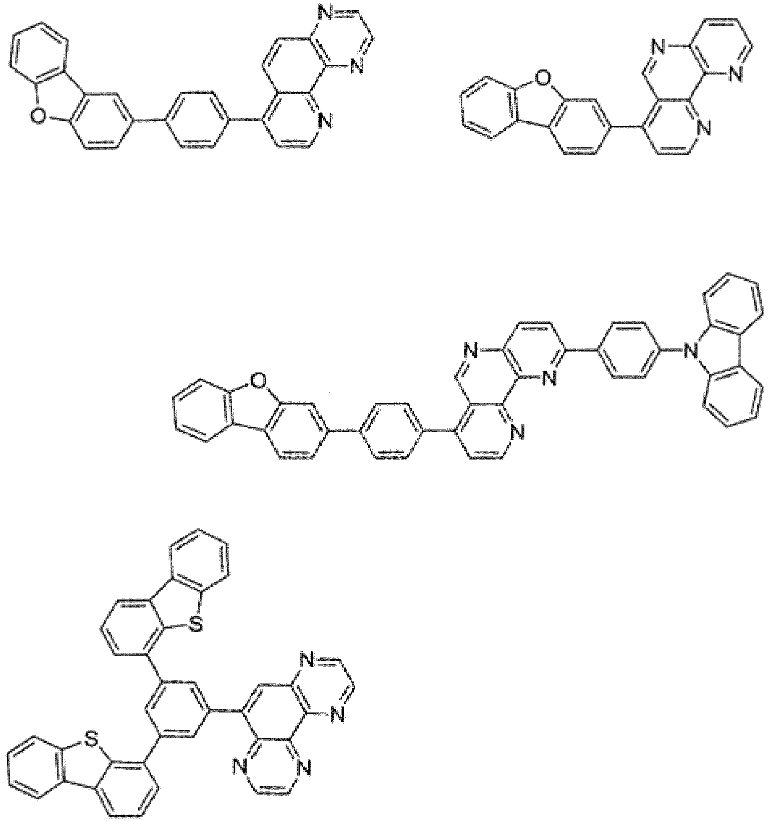

The compound according to the exemplary embodiment is preferably contained in an organic compound layer between an anode and a cathode of an organic EL device. Moreover, in the organic EL device including an emitting layer and an electron transporting layer between the anode and the cathode, the compound according to the exemplary embodiment is more preferably contained in the electron transporting layer.

### Material for Organic Electroluminescence Device

A material for an organic EL device (also referred to as an organic-EL-device material) preferably contains the compound according to the exemplary embodiment. The organic-EL-device material may be composed solely of the compound represented by the formula (1), or alternatively, may contain another compound in addition to the compound represented by the formula (1). The organic-EL-device material according to the exemplary embodiment is usable for forming the organic compound layer of the organic EL device.

The organic-EL-device material according to the exemplary embodiment is preferably contained in the organic compound layer between the anode and the cathode of the organic EL device. The organic-EL-device material according to the exemplary embodiment is more preferably contained in the electron transporting layer of the organic EL device, the organic EL device including the emitting layer and the electron transporting layer between the anode and the cathode.

### Organic EL Device

Typical device arrangements of an organic EL device include the following arrangements (a) to (e) and the like:
(a) anode/ emitting layer/ cathode;
(b) anode / hole injecting·transporting layer / emitting layer / cathode;
(c) anode / emitting layer / electron injecting·transporting layer / cathode;
(d) anode / hole injecting·transporting layer / emitting layer / electron injecting·transporting layer / cathode; and
(e) anode / hole injecting·transporting layer / emitting layer / blocking layer / electron injecting·transporting layer / cathode.

While the arrangements (d) and (e) are preferably used among the above arrangements, the arrangement of the invention is not limited to the above arrangements.

It should be noted that the aforementioned "emitting layer" is an organic layer having an emission function and, when a doping system is employed, containing a host material and a dopant material. At this time, the host material has a function to mainly promote recombination of electrons and holes and trap excitons within the emitting layer while the dopant material has a function to promote an efficient emission from the excitons obtained by the recombination. In a phosphorescent device, the host material has a function of trapping the excitons, which are generated mainly in the dopant, within the emitting layer.

The "hole injecting/transporting layer (or hole injecting·transporting layer) means "at least one of a hole injecting layer and a hole transporting layer while the "electron injecting/transporting layer (or electron injecting·transporting layer) means "at least one of an electron injecting layer and an electron transporting layer. Herein, when the hole injecting layer and the hole transporting layer are provided, the hole injecting layer is preferably closer to the anode. When the electron injecting layer and the electron transporting layer are provided, the electron injecting layer is preferably closer to the cathode.

In the exemplary embodiment, the electron transporting layer means an organic layer having the highest electron mobility among organic layer(s) providing an electron transporting zone existing between the emitting layer and the cathode. When the electron transporting zone is provided by a single layer, the single layer is the electron transporting layer. Moreover, in the phosphorescent organic EL device, a blocking layer having an electron mobility that is not always high may be provided as shown in the arrangement (e) between the emitting layer and the electron transporting layer in order to prevent diffusion of exciton energy generated in the emitting layer. Thus, the organic layer adjacent to the emitting layer does not always correspond to the electron transporting layer.

In an exemplary embodiment of the invention, the organic EL device includes: the cathode; the anode; and the organic compound layer between the cathode and the anode. The organic compound layer at least includes the emitting layer and the electron transporting layer. Further, the organic compound layer may include a layer(s) employed in a known organic EL device such as a hole injecting layer, a hole transporting layer, an electron injecting layer, a hole blocking layer and an electron blocking layer. The organic compound layer may include an inorganic compound.

The organic-EL-device material according to the exemplary embodiment is contained in the organic compound layer. When the organic compound layer is provided by a plurality of layers, the organic-EL-device material according to the exemplary embodiment is contained singularly or as a component of a mixture in at least one of the plurality of layers. The electron transporting layer preferably contains the organic-EL-device material according to the exemplary embodiment.

The organic EL device according to the exemplary embodiment of the invention includes a light-transmissive substrate, the anode, the cathode, and the organic compound layer disposed between the anode and the cathode.

The organic compound layer includes the hole injecting layer, the hole transporting layer, the emitting layer, the hole blocking layer, the electron transporting layer and the electron injection layer in this order from the anode.

### Electron Transporting Layer

The electron transporting layer of the organic EL device according to the exemplary embodiment contains a compound represented by a formula (10) below.

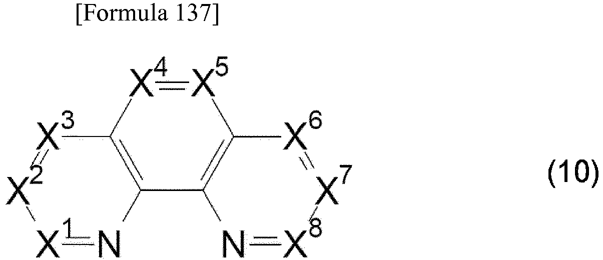

In the formula (10), X¹ to X⁸ each independently represent a carbon atom to be bonded to a group represented by a formula (20) below, CR^{X} or a nitrogen atom. At least one of X¹ to X⁸ is a carbon atom to be bonded to the group represented by the formula (20).

R^{X} is each independently selected from the group consisting of a hydrogen atom, halogen atom, hydroxyl group, cyano group, nitro group, carboxyl group, sulfonyl group, boryl group, phosphino group, mercapto group, acyl group, substituted or unsubstituted amino group, substituted or unsubstituted silyl group, substituted or unsubstituted alkyl group having 1 to 30 carbon atoms, substituted or unsubstituted alkenyl group having 2 to 30 carbon atoms, substituted or unsubstituted alkynyl group having 2 to 30 carbon atoms, substituted or unsubstituted aralkyl group having 6 to 30 carbon atoms, substituted or unsubstituted aryl group having 6 to 40 ring carbon atoms, and substituted or unsubstituted heteroaryl group having 5 to 40 ring atoms.

In the formula (10), R^{X} of adjacent ones of CR^{X} in X¹ to X⁸ may be bonded to each other to form a cyclic structure or may not be bonded.

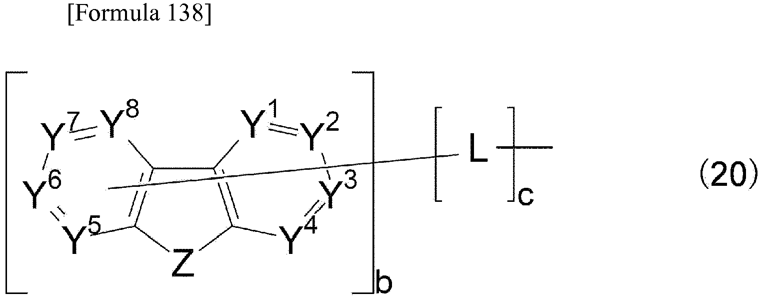

In the formula (20), b is an integer of 1 to 5. In the formula (20), c is an integer of 1 to 8.

In the formula (20), Z represents an oxygen atom, sulfur atom or silicon atom. When b is from 2 to 5, a plurality of Z are mutually the same or different. When Z is a silicon atom, R⁹ and R¹⁰ are bonded to the silicon atom. R⁹ and R¹⁰ each independently represent the same as R^{X} in the formula (10). R⁹ and R¹⁰ may be bonded to the structure represented by the formula (10).

In the formula (20), L is selected from a single bond and a linking group. The linking group represents a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms. The polyvalent heteroaryl group having 5 to 40 ring atoms in L of the formula (20) includes a substituted or unsubstituted polyvalent group derived from a phenanthroline ring represented by the formula (10). When c is from 2 to 8, a plurality of L are mutually the same or different.

In the formula (20), Y¹ to Y⁸ each independently represent a nitrogen atom, CR^{Y} or a carbon atom to be bonded to L.

In the formula (20), R^{Y} represents the same as R^{X} in the formula (10). The heteroaryl group having 5 to 40 ring atoms in R^{Y} includes a substituted or unsubstituted phenanthrolyl group derived from the phenanthroline ring represented by the formula (10). In the formula (20), adjacent ones of R^{Y} may be bonded to each other to form a cyclic structure or may not be bonded.

X¹ to X⁸ in the formula (10) respectively represent the same as X¹ to X⁸ in the formula (1).

Y¹ to Y⁸, Z, L, b and c in the formula (20) respectively represent the same as Y¹ to Y⁸, Z, L, b and c in the formula (2).

The substituents in the formulae (10) and (20) represent the same as the substituents described in the formulae (1), (1-1) to (1-16), (2), (2-1), (1-x) (1-xx-1) and (1-xx-2).

The formula (20) in which a substituted or unsubstituted polyvalent group derived from the phenanthroline ring represented by the formula (10) is included in L is exemplarily represented by a formula (20-1) below. In the formula (20-1), A1 represents the structure represented by the formula (10).

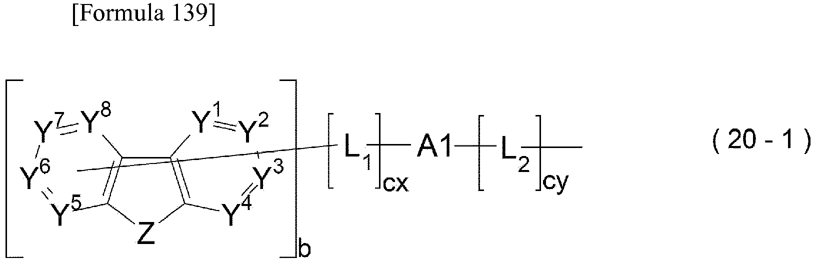

In the formula (20-1), cx is an integer of 0 to 7, cy is an integer of 0 to 7 and 0≤cx+cy≤7. In the formulae (20-1), X¹ to X⁸ of A1, Y¹ to Y⁸, Z, L and b respectively represent the same as X¹ to X⁸, Y¹ to Y⁸, Z, L and b in the formulae (10) and (20).

The formula (20) in which R^{Y} of CR^{Y} is a substituted or unsubstituted phenanthrolyl group derived from the phenanthroline ring represented by the formula (10) is exemplarily represented by a formula (10-1) below. In the formula (10-1), A2 and A3 represent the structure represented by the formula (10).

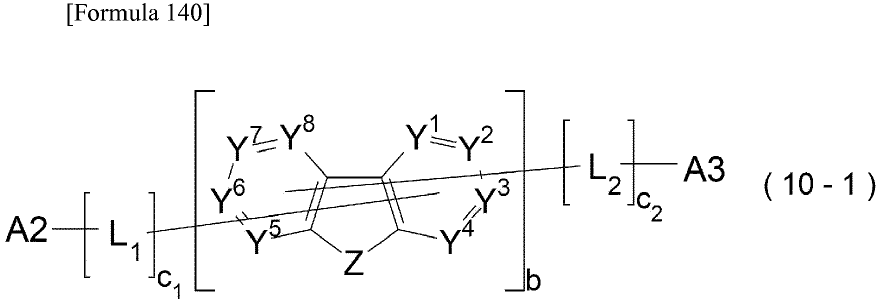

In the formula (10-1), c₁ is an integer of 1 to 8 and c₂ is an integer of 1 to 8. In the formula (10-1), L₁ and L₂ each independently represent the same as L in the formula (20). In the formula (10-1), X¹ to X⁸ in A2 and A3, Y¹ to Y⁸, Z and b respectively represent the same as X¹ to X⁸, Y¹ to Y⁸, Z and b in the formulae (10) and (20).

The formula (20) in which a substituted or unsubstituted polyvalent group derived from the phenanthroline ring represented by the formula (10) is included in L and in which R^{Y} of CR^{Y} is the substituted or unsubstituted phenanthrolyl group derived from the phenanthroline ring represented by the formula (10) is exemplarily represented by a formula (10-2) below. In the formula (10-2), A1, A2 and A3 represent the structure represented by the formula (10).

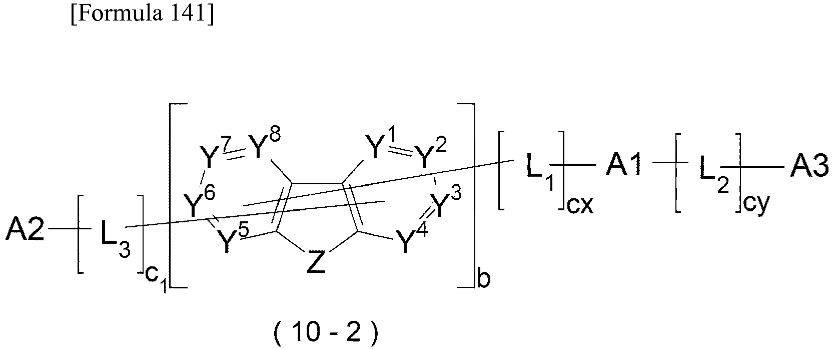

In the formula (10-2), X¹ to X⁸ in A1, A2 and A3, Y¹ to Y⁸, Z, L₁ to L₃, b, cx, cy and c₁ respectively represent the same as X¹ to X⁸, Y¹ to Y⁸, Z L₁ to L₃, b, cx, cy and c₁ in the formulae (10), (20), (10-1) and (20-1).

In the organic EL device according to the exemplary embodiment, X¹ or X⁸ in the formula (10) is preferably a carbon atom to be bonded to the group represented by the formula (20).

In the organic EL device according to the exemplary embodiment, X¹ and X⁸ in the formula (10) are preferably carbon atoms to be bonded to the group represented by the formula (20).

In the organic EL device according to the exemplary embodiment, X³ and X⁶ in the formula (10) are preferably carbon atoms to be bonded to the group represented by the formula (20).

In the organic EL device according to the exemplary embodiment, X² or X⁷ in the formula (10) is preferably a carbon atom to be bonded to the group represented by the formula (20).

In the organic EL device according to the exemplary embodiment, X³ or X⁶ in the formula (10) is preferably a carbon atom to be bonded to the group represented by the formula (20).

In the organic EL device according to the exemplary embodiment, X⁴ or X⁵ in the formula (10) is preferably a carbon atom to be bonded to the group represented by the formula (20).

In the organic EL device according to the exemplary embodiment, X¹ and X⁷ in the formula (10) are preferably carbon atoms to be bonded to the group represented by the formula (20).

In the organic EL device according to the exemplary embodiment, X² and X⁷ in the formula (10) are preferably carbon atoms to be bonded to the group represented by the formula (20).

In the organic EL device according to the exemplary embodiment, X¹, X², X⁷ and X⁸ in the formula (10) are preferably carbon atoms to be bonded to the group represented by the formula (20).

In the organic EL device according to the exemplary embodiment, X¹, X³, X⁶ and X⁸ in the formula (10) are preferably carbon atoms to be bonded to the group represented by the formula (20).

In the organic EL device according to the exemplary embodiment, X¹ or X⁸ in the formula (10) is preferably a carbon atom to be bonded to the group represented by the formula (20). X¹ and X⁸ in the formula (10) are more preferably carbon atoms to be bonded to the group represented by the formula (20).

In the organic EL device according to the exemplary embodiment, in the formulae (10) and (10-1) to (10-2), the rest of X¹ to X⁸ except for the carbon atom to be bonded to the group represented by the formula (20) are preferably CR^{X}, in which R^{X} is more preferably a hydrogen atom, alkyl group or aryl group, further preferably a hydrogen atom or a phenyl group.

Moreover, in the organic EL device according to the exemplary embodiment, Z in the formula (20) is preferably an oxygen atom or a sulfur atom, more preferably an oxygen atom.

It is also preferable that the electron transporting layer of the organic EL device according to the exemplary embodiment contains at least one of an electron-donating dopant and an organic metal complex. A content of the electron-donating dopant or the organic metal complex in the electron transporting layer is preferably in a range of 1 mass% to 50 mass%.

The electron-donating dopant material is preferably at least one material selected from the group consisting of an alkali metal, alkali earth metal, rare earth metal, oxide of alkali metal, halide of alkali metal, oxide of alkali earth metal, halide of alkali earth metal, oxide of rare earth metal, and halide of rare earth metal.

The organic metal complex is preferably at least one selected from the group consisting of an oganic metal complex including an alkali metal, oganic metal complex including an alkali earth metal, and oganic metal complex including a rare earth metal.

The electron-donating dopant and organic metal complex are described in detail below.

With the organic EL device in the exemplary embodiment, a drive voltage can be decreased since the electron transporting layer contains the compound represented by the formula (10). Moreover, since the electron transporting layer contains the compound represented by the formula (10) and at least one of the electron-donating dopant and the organic metal complex, the electron-donating dopant and/or the organic metal complex are easily captured by a phenanthroline skeleton represented by the formula (10), so that the drive voltage is further decreased.

Moreover, the organic-EL-device material containing the compound represented by the formula (10) may be used for the organic EL device in the exemplary embodiment.

The compound represented by the formula (10) encompasses the compound represented by the formula (1). Specific examples of the compound represented by the formula (10) are the same as the above-described specific examples of the compound represented by the formula (1) and compounds shown below, but the invention is not limited to thereto.

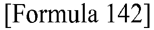

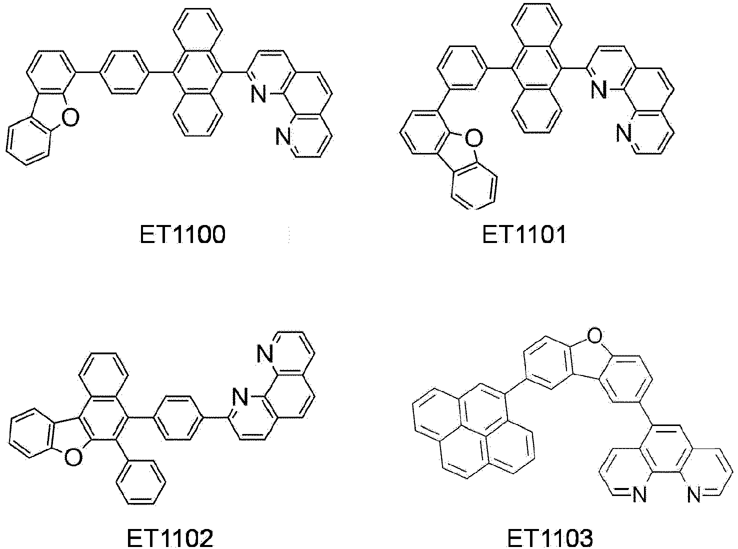

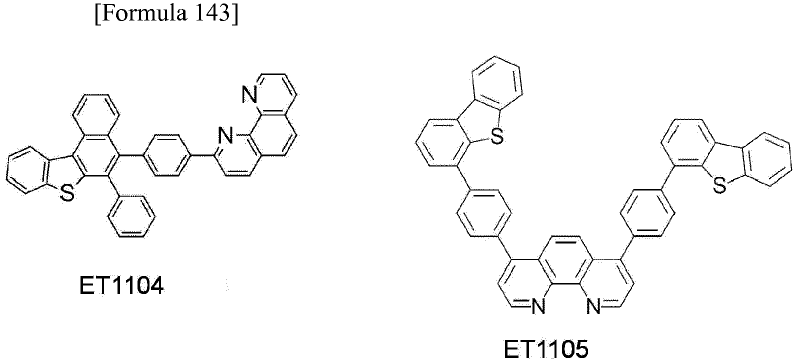

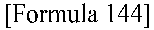

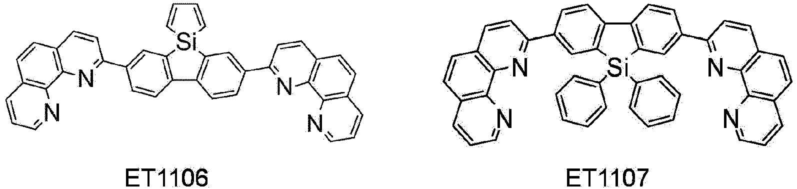

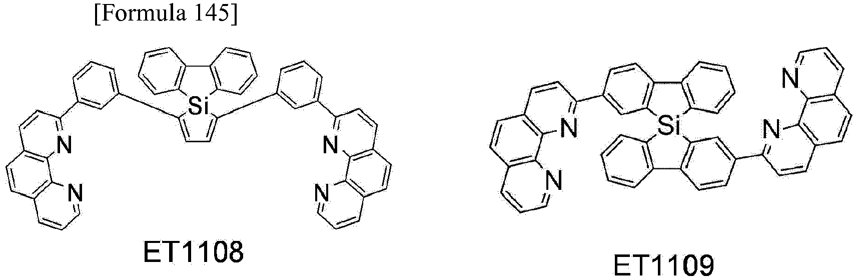

### Substrate

The organic EL device according to the exemplary embodiment is formed on a light-transmissive substrate. The light-transmissive plate, which supports the organic EL device, is preferably a smooth substrate that transmits 50% or more of light in a visible region of 400 nm to 700 nm. Specifically, examples of the substrate are a glass plate and a polymer plate.

### Anode and Cathode

The anode of the organic EL device is used for injecting holes into the hole injecting layer, the hole transporting layer or the emitting layer. It is effective that the anode has a work function of 4.5 eV or more. Specific examples of a material for the anode are alloys of indium-tin oxide (ITO), tin oxide (NESA), indium zinc oxide, gold, silver, platinum and copper.

The cathode is preferably formed of a material with smaller work function in order to inject electrons into the electron injecting layer, the electron transporting layer and the emitting layer. Although a material for the cathode is not particularly limited, examples of the material are indium, aluminium, magnesium, alloy of magnesium and indium, alloy of magnesium and aluminium, alloy of aluminium and lithium, alloy of aluminium, scandium and lithium, and alloy of magnesium and silver.

### Emitting Layer

The emitting layer of the organic EL device has a function for providing conditions for recombination of the electrons and the holes to emit light. The emitting layer is preferably a molecular deposit film. The molecular deposit film means a thin film formed by depositing a material compound in gas phase or a film formed by solidifying a material compound in a solution state or in liquid phase. The molecular deposit film is typically distinguished from a thin film formed by the LB (Langmuir Blodgett) method (i.e., molecular accumulation film) by differences in aggregation structures and higher order structures and functional differences arising therefrom.

### Dopant Material

The dopant material is selected from a known fluorescent material that emits fluorescence or a known phosphorescent material that emits phosphorescence.

### Host Material

As a host material, any host material applicable to the organic EL device is used. Examples of the host material include an amine derivative, azine derivative and fused polycyclic aromatic derivative.

Examples of the amine derivative are a monoamine compound, diamine compound, triamine compound, tetramine compound and amine compound substituted with a carbazole group.

Examples of the azine derivative include a monoazine derivative, diazine derivative and triazine derivative.

The fused polycyclic aromatic derivative is preferably a fused polycyclic aryl having no heterocyclic skeleton. Examples of the fused polycyclic aromatic derivative include the fused polycyclic aryl such as naphthalene, anthracene, phenanthrene, chrysene, fluoranthene and triphenylene, or derivatives thereof.

### Hole Injecting·Transporting Layer

The hole injecting·transporting layer helps injection of holes to the emitting layer and transports the holes to an emitting region. The hole injecting·transporting layer exhibits a large hole mobility and a small ionization energy.

A material for forming the hole injecting layer and the hole transporting layer is preferably a material for transporting the holes to the emitting layer at a lower electric field intensity. For instance, an aromatic amine compound is preferably used. A material for the hole injecting layer is preferably a porphyrin compound, an aromatic tertiary amine compound or a styryl amine compound, particularly preferably the aromatic tertiary amine compound such as hexacyanohexaazatriphenylene (HAT).

### Electron Injecting·Transporting Layer

The electron injection/transport layer helps injection of the electron to the luminescent layer and has a high electron mobility. The electron injecting layer is provided for adjusting energy level, by which, for instance, sudden changes of the energy level can be reduced.

In the exemplary embodiment, the electron injecting·transporting layer at least includes the electron transporting layer containing the compound represented by the formula (10). In addition to the electron transporting layer, the electron injecting·transporting layer may include an electron injecting layer or another electron transporting layer. The electron injecting·transporting layer may be formed by laminating a first electron transporting layer, second electron transporting layer and electron injecting layer in this order from the anode. In this arrangement, the compound represented by the formula (10) is preferably contained in the first electron transporting layer. When the organic EL device according to the exemplary embodiment includes a plurality of electron transporting layers, it is only required that the compound represented by the formula (10) is contained in at least one of the electron transporting layers. Preferably, the compound represented by the formula (10) is contained in the electron transporting layer closer to the emitting layer. Moreover, the electron transporting layer containing the compound represented by the formula (10) may contain an alkali metal as described above, or alternatively, may contain the following electron transrporting material in addition to the alkali metal.

The organic EL device according to the exemplary embodiment preferably includes the electron injecting layer between the electron transporting layer and the cathode, in which the electron injecting layer preferably contains a nitorogen-containing cyclic derivative as a main component. The electron injecting layer may serve as the electron transporting layer. Noted that "as a main component" means that the nitrogen-containing cyclic derivative is contained in the electron injecting layer at a content of 50 mass% or more.

The electron transporting material for forming the electron transporting layer or the electron injecting layer is preferably an aromatic heterocyclic compound having at least one heteroatom in the molecule, particularly preferably a nitrogen-containing cyclic derivative. The nitrogen-containing cyclic derivative is preferably an aromatic ring having a nitrogen-containing six-membered or five-membered ring skeleton, or a fused aromatic cyclic compound having a nitrogen-containing six-membered or five-membered ring skeleton.

### Electron-donating Dopant and Organic Metal Complex

It is also preferable that the electron transporting layer of the organic EL device according to the exemplary embodiment contains at least one of the electron-donating dopant and the organic metal complex. With this arrangement, a decrease in the voltage applied on the organic EL device is enhanced. The electron-donating dopant may be at least one selected from an alkali metal, an alkali metal compound, an alkaline-earth metal, an alkaline-earth metal compound, a rare-earth metal, a rare-earth metal compound and the like.

The organic metal complex may be at least one compound selected from an organic metal complex including an alkali metal, an organic metal complex including an alkali earth metal, and an organic metal complex including rare-earth metal.

Examples of the alkali metal are lithium (Li) (work function: 2.93 eV), sodium (Na) (work function: 2.36 eV), potassium (K) (work function: 2.28 eV), rubidium (Rb) (work function: 2.16 eV) and cesium (Cs) (work function: 1.95 eV), among which a substance having a work function of 2.9 eV or less is particularly preferable. Among the above, the alkali metal is preferably K, Rb or Cs, more preferably Rb or Cs, the most preferably Cs.

Examples of the alkali earth metal are calcium (Ca) (work function: 2.9 eV), strontium (Sr) (work function: 2.0 to 2.5 eV), and barium (Ba) (work function: 2.52 eV), among which a substance having a work function of 2.9 eV or less is particularly preferable.

Examples of the rare-earth metal are scandium (Sc), yttrium (Y), cerium (Ce), terbium (Tb) and ytterbium (Yb), among which a substance having a work function of 2.9 eV or less is particularly preferable.

Since the above preferred metals have particularly high reducibility, addition of a relatively small amount of the metals to an electron injecting zone can enhance luminance intensity and lifetime of the organic EL device.

Examples of the alkali metal compound are an alkali oxide such as lithium oxide (Li₂O), cesium oxide (Cs₂O) and potassium oxide (K₂O), and an alkali halogenide such as sodium fluoride (NaF), cesium fluoride (CsF) and potassium fluoride (KF), among which lithium fluoride (LiF), lithium oxide (Li₂O) and sodium fluoride (NaF) are preferable.

Examples of the alkali earth metal compound are barium oxide (BaO), strontium oxide (SrO), calcium oxide (CaO) and a mixture thereof, i.e., BaₓSr₁₋ₓO(O<x<1), BaₓCa₁₋ₓO(O<x<1), among which BaO, SrO and CaO are preferable.

Examples of the rare-earth metal compound are ytterbium fluoride (YbF₃), scandium fluoride (ScF₃), scandium oxide (ScO₃), yttrium oxide (Y₂O₃), cerium oxide (Ce₂O₃), gadolinium fluoride (GdF₃) and terbium fluoride (TbF₃), among which YbF₃, ScF₃ and TbF₃ are preferable.

The organic metal complex is not particularly limited, as long as the organic metal complex contains at least one of an alkali metal ion, alkali earth metal ion and rare-earth metal ion, as a metal ion. The ligand for each of the complexes is preferably quinolinol, benzoquinolinol, acridinol, phenanthridinol, hydroxyphenyl oxazole, hydroxyphenyl thiazole, hydroxydiaryl oxadiazole, hydroxydiaryl thiadiazole, hydroxyphenyl pyridine, hydroxyphenyl benzoimidazole, hydroxybenzo triazole, hydroxy fluborane, bipyridyl, phenanthroline, phthalocyanine, porphyrin, cyclopentadiene, β-diketones, azomethines, or a derivative thereof, but the ligand is not limited thereto.

A preferable method for adding the electron-donating dopant and the organic metal complex includes dispersing at least one of the electron-donating dopant and the organic metal complex in the electron transporting layer while co-depositing at least one of the electron-donating dopant and the organic metal complex with the compound represented by the formula (1) by resistance heating deposition. A dispersion concentration is shown by a film thickness ratio of the compound represented by the formula (1) to the electron-donating dopant and/or the organic metal complex being of 1000:1 to 1:1000, preferably 100:1 to 1:1.

When at least one of the electron-donating dopant and the organic metal complex forms a layer, after forming a layer of the compound represented by the formula (1), at least one of the electron-donating dopant and the organic metal complex is singularly deposited on the compound layer by resistance heating deposition to preferably form a 0.1 nm- to 15 nm-thick layer.

When at least one of the electron-donating dopant and the organic metal complex forms an island pattern, after forming an island pattern of the compound represented by the formula (1), at least one of the electron-donating dopant and the organic metal complex is singularly deposited on the compound layer by resistance heating deposition to preferably form a 0.05 nm- to 1 nm-thick island pattern.

A ratio of the compound represented by the formula (1) to at least one of the electron-donating dopant and the organic metal complex in the organic EL device according to the exemplary embodiment is preferably shown by a film thickness ratio of the main component to the electron-donating dopant and/or the organic metal complex being 100:1 to 1:1, more preferably 50:1 to 4:1.

### Blocking Layer

The organic EL device preferably includes a blocking layer such as an electron blocking layer, hole blocking layer or triplet blocking layer at a part adjacent to the emitting layer. Herein, the electron blocking layer prevents electrons from leaking from the emitting layer into the hole transporting layer while the hole blocking layer prevents holes from leaking from the emitting layer into the electron transporting layer. In the organic EL device according to the exemplary embodiment, the hole blocking layer is provided between the electron transporting layer and the emitting layer. The triplet blocking layer has a function of preventing triplet excitons generated in the emitting layer from diffusing into neighboring layers to trap the triplet excitons within the emitting layer, thereby suppressing energy deactivation of the triplet excitons on molecules other than the emitting dopant in the electron transporting layer.

### Manufacturing Method of Each Layer of Organic EL Device

A method of forming each of the layers in the organic EL device according to the exemplary embodiment is not particularly limited. Conventionally-known methods such as vacuum deposition and spin coating may be employed for forming the layers. The organic layer, which is used in the organic EL device of the exemplary embodiment, may be formed by a known method such as vacuum deposition, molecular beam epitaxy (MBE (Molecular Beam Epitaxy) method) or coating methods using a solution such as a dipping, spin coating, casting, bar coating, and roll coating.

### Film Thickness of Each Layer of Organic EL Device

A film thickness of the emitting layer is preferably in a range of 5 nm to 50 nm, more preferably in a range of 7 nm to 50 nm and most preferably in a range of 10 nm to 50 nm. By forming the emitting layer at the film thickness of 5 nm or more, the emitting layer is easily formable and chromaticity is easily adjustable. By forming the emitting layer at the film thickness of 50 nm or less, an increase in the drive voltage is suppressible.

Although the film thickness of each of other organic layers is not specifically limited, the film thickness is preferably in a typical range of several nm to 1 µm. When the film thickness is provided in the above range, defects such as pin holes caused by an excessively thinned film can be avoided while an increase in the drive voltage caused by an excessively thickened film can be suppressed.

### Modifications of Embodiment(s)

It should be noted that the invention is not limited to the above exemplary embodiment but may include any modification and improvement as long as such modification and improvement are compatible with the invention.

In the above exemplary embodiment, the organic EL device including the hole blocking layer between the emitting layer and the electron transporting layer containing the compound according to the exemplary embodiment is described as an example. However, the arrangement of the organic EL is not limited thereto.

For instance, such an organic EL device that includes: the emitting layer; and the electron transporting layer containing the compound according to the exemplary embodiment, in which the emitting layer is adjacent to the electron transporting layer, is also preferable.

The emitting layer is not limited to a single layer, but may be provided as a laminate by a plurality of emitting layers. When the organic EL device includes the plurality of emitting layers, the plurality of emitting layers may be each independently a fluorescent emitting layer or a phosphorescent emitting layer.

When the organic EL device includes the plurality of emitting layers, the plurality of emitting layers may be adjacent to each other, or may be laminated on each other via an intermediate layer to provide a so-called tandem-type organic EL device.

It is also preferable that the organic EL device contains at least one of the electron-donating dopant and the organic metal complex in an interfacial region between the cathode and the organic compound layer. With this arrangement, the organic EL device can emit light with enhanced luminance intensity and have a longer lifetime. Examples of the electron-donating dopant and the organic metal complex are the same as described above.

The electron-donating dopant and the organic metal complex are added to preferably form a layer or an island pattern in the interfacial region. The layer of the electron-donating dopant or the island pattern of the organic metal complex is preferably formed by: depositing at least one of the electron-donating dopant and the organic metal complex while simultaneously depositing an organic substance that is an emitting material or an electron-injecting material for forming the interfacial region, by resistance heating deposition; and dispersing at least one of the electron-donating dopant and an organic metal complex reduction-causing dopant in the organic substance.

In the invention, it is also preferable that the emitting layer contains an assistance material for assisting injection of charges. When the emitting layer is formed of a host material that exhibits a wide energy gap, a difference in ionization potential (Ip) between the host material and the hole injecting/transporting layer etc. becomes so large that injection of the holes into the emitting layer becomes difficult, which may cause a rise in a driving voltage required for providing sufficient luminance. In the above instance, since a hole-injectable or hole-transportable assistance substance for assisting injection of charges is contained in the emitting layer, holes can be easily inject-d into the emitting layer and the drive voltage can be decreased.

As the assistance substance for assisting the injection of charges, for instance, a typical hole injecting·transporting material is usable.

Specific examples of the material for assisting the injection of charges are a triazole derivative, oxadiazole derivative, imidazoles derivative, polyarylalkane derivative, pyrazoline derivative, pyrazolone derivative, phenylenediamine derivative, arylamine derivative, amino-substituted chalcone derivative, oxazole derivative, fluorenone derivative, hydrazone derivative, stilbene derivative, silazane derivative, polysilane copolymer, aniline copolymer, and conductive polymer oligomer (particularly, a thiophene oligomer).

The hole injecting material is exemplified by the above. The hole injecting material is preferably a porphyrin compound, aromatic tertiary amine compound and styryl amine compound, particularly preferably aromatic tertiary amine compound.

The organic EL device of the invention is suitably applicable to an electronic device such as: a display of a television, a mobile phone, a personal computer and the like; and an emitting unit of an illuminator or a vehicle light.

### Examples

Next, the invention will be described in further detail by exemplifying Example(s) and Comparative(s). However, the invention is not limited by the description of Example(s).

### Synthesis Examples

### (1) Synthesis Example 1: Synthesis of Compound 5

A synthesis scheme of a compound 5 is shown below.

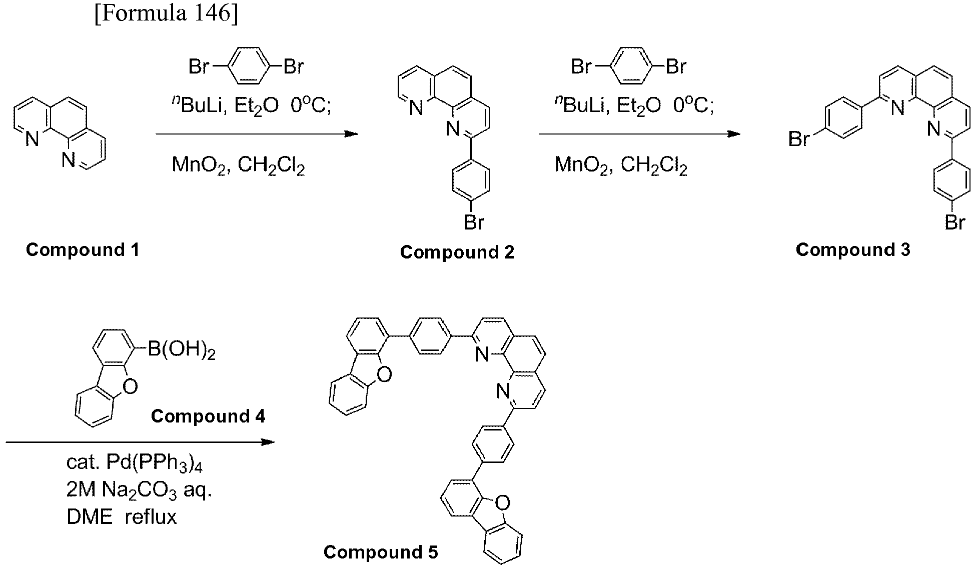

### (1-1) Synthesis of Compound 2

In synthesizing the compound 5, a compound 2 was initially synthesized.

Under an argon gas atmosphere, 1,4-dibromobenzene (50 g, 211 mmol) was dissolved in diethylether (350 mL). After the obtained solution was cooled to 0 degrees C, n-butyllithium (2.69 M hexane solution)(72 mL, 194 mmol) was dropped into the cooled solution for 30 minutes. The obtained mixture solution was further stirred for 30 minutes. The prepared p-bromophenyl lithium was dropped at 0 degrees C for 45 minutes in a suspension liquid in which 1,10-phenanthroline (15 g, 85 mmol) as a compound 1 was suspended in diethylether (350 mL). The obtained mixture solution was further stirred for five hours. After the completion of the reaction, water was dropped at 0 degrees C for 30 minutes in the obtained reaction solution. The reaction solution was extracted with dichloromethane. A solvent except for 200 mL of dichloromethane was distilled under reduced pressure. Manganese dioxide (150 g) was added to the obtained solution and stirred at the room temperature for 4.5 hours. Subsequently, magnesium sulfate was added to the reaction solution. The obtained deposit was subjected to filtration and a solvent was distilled under reduced pressure. A residue was purified by silica-gel column chromatography (dichloromethane/hexane/methanol). The obtained solid was washed with methanol and dried under reduced pressure, so that a compound 2 (23 g, a yield of 81%) was obtained as a white solid. By FD-MS (Field Desorption Mass Spectrometry) analysis, the obtained compound was identified to be the compound 2.

### (1-2) Synthesis of Compound 3

A compound 3 (18 g, a yield of 55%) was obtained as a white solid in the same manner as in the (1-1) synthesis of the compound 2, except for using the compound 2 (23 g, 68 mmol) in place of the compound 1. By FD-MS (Field Desorption Mass Spectrometry) analysis, the obtained compound was identified to be the compound 3.

### (1-3) Synthesis of Compound 5

The compound 3 (6.0 g, 12 mmol) and a compound 4 (5.7 g, 27 mmol) were suspended in 1,2-dimethoxyethane (200 mL), to which tetrakis(triphenylphosphine)palladium(0) (0.7 g, 0.61 mmol) and an aqueous solution of 2M sodium carbonate (37 mL) were added. The obtained solution was heated to reflux for seven hours. After the completion of the reaction, water was added to the reaction solution and the reaction solution was filtrated to obtain a solid. The obtained solid was washed with water and methanol and dried under reduced pressure. The obtained crude product was purified by silica-gel column chromatography (dichloromethane). The obtained solid was washed with methanol and dried under reduced pressure, so that a compound 5 (7.2 g, a yield of 88%) was obtained as a white solid. By FD-MS (Field Desorption Mass Spectrometry) analysis, the obtained compound was identified to be the compound 5.

### (2) Synthesis Example 2: Synthesis of Compound 9 (Reference Example, which is not according to our present invention)

A synthesis scheme of a compound 9 is shown below.

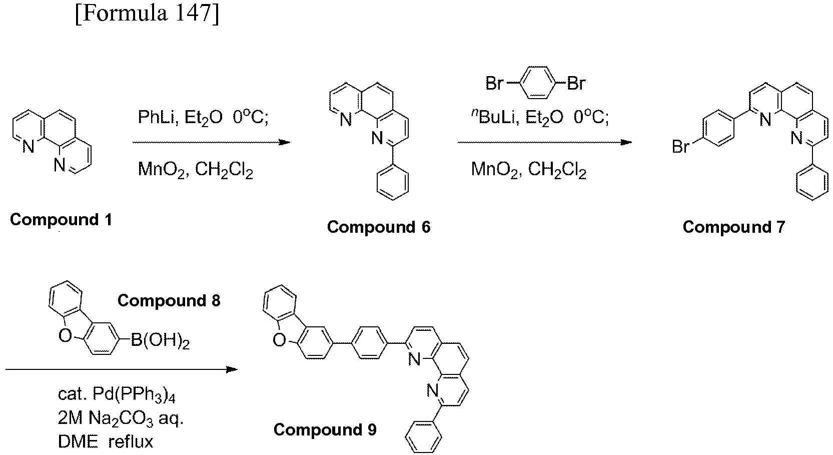

### (2-1) Synthesis of Compound 6

A compound 6 (24 g, a yield of 83%) was obtained as a yellow solid in the same manner as in the (1-1) synthesis of the compound 2, except for using phenyllithium (1.6M butylether solution)(139 mL, 222 mmol) in place of p-bromophenyllithium, the phenyllithium being used at 2 molar equivalent to 1,10-phenanthroline. By FD-MS (Field Desorption Mass Spectrometry) analysis, the obtained compound was identified to be the compound 6.

### (2-2) Synthesis of Compound 7

A compound 7 (12 g, a yield of 76%) was obtained as a yellow solid in the same manner as in the (1-1) synthesis of the compound 2, except for using the compound 6 (10 g, 39 mmol) in place of the compound 1. By FD-MS (Field Desorption Mass Spectrometry) analysis, the obtained compound was identified to be the compound 7.

### (2-3) Synthesis of Compound 9

A compound 9 (1.7g, a yield of 23%) was obtained as a white solid in the same manner as in (1-3) synthesis of the compound 5, except for using the compound 7(6.0g, 15 mmol) in place of the compound 3 and using the compound 8 (3.4 g, 16 mmol) in place of the compound 4. By FD-MS (Field Desorption Mass Spectrometry) analysis, the obtained compound was identified to be the compound 9.

### (3) Synthesis Example 3: Synthesis of Compound 10

A synthesis scheme of a compound 10 is shown below.

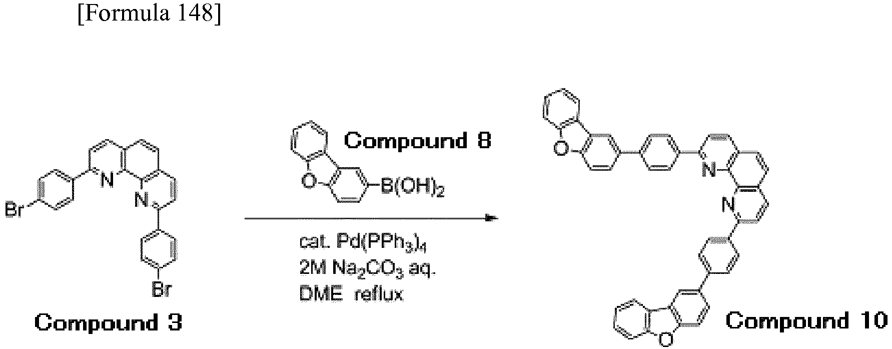

### (3-1) Synthesis of Compound 10

A compound 10 (0.25oz, a yield of 80%) was obtained as a white solid in the same manner as in the (1-3) synthesis of the compound 5, except for using the compound 8 (6.3 g, 30 mmol) in place of the compound 4. By FD-MS (Field Desorption Mass Spectrometry) analysis, the obtained compound was identified to be the compound 10.

### (4) Synthesis Example 4: Synthesis of Compound 13

A synthesis scheme of a compound 13 is shown below.

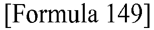

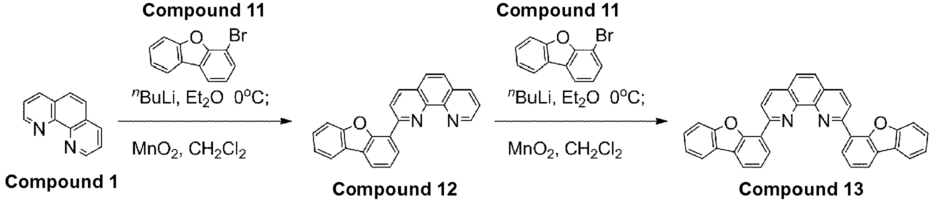

### (4-1) Synthesis of Compound 12

A compound 12 (6.5 g, a yield of 67%) was obtained as a yellow solid in the same manner as in the (1-1) synthesis of the compound 2, except for using the compound 11 (17 g, 69 mmol) in place of 1,4-dibromobenzene. By FD-MS (Field Desorption Mass Spectrometry) analysis, the obtained compound was identified to be the compound 12.

### (4-2) Synthesis of Compound 13

A compound 13 (2.1 g, a yield of 22%) was obtained as a light yellow solid in the same manner as in (1-1) synthesis of the compound 2, except for using the compound 12 (6.5 g, 19 mmol) in place of the compound 1 and using the compound 11 (12 g, 47 mmol) in place of 1,4-dibromobenzene. By FD-MS (Field Desorption Mass Spectrometry) analysis, the obtained compound was identified to be the compound 13.

### (4A) Synthesis Example 4A: Synthesis of Compound 1C

A synthesis scheme of a compound 1C is shown below.

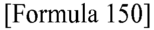

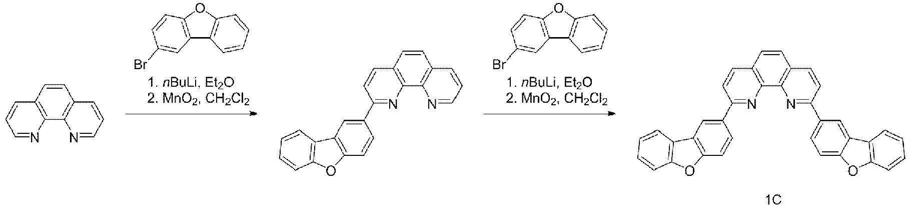

The compound 1C was synthesized according to the above scheme in the same manner as in the synthesis of the compound 13 except for using 2-bromodibenzofuran in place of the compound 11.

### (4B) Synthesis Example 4B: Synthesis of Compound 1D (Reference Example, which is not according to our present invention)

A synthesis scheme of a compound 1D is shown below.

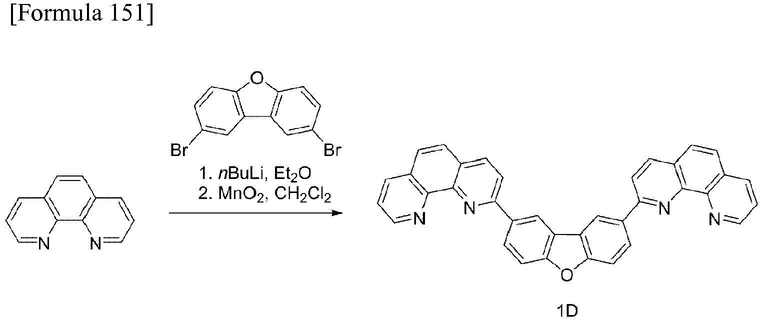

The compound 1D was synthesized according to the above scheme in the same manner as in the synthesis of the compound 13 except for using 2,8-bromodibenzofuran in place of the compound 11.

### (5) Synthesis Example 5: Synthesis of Compound 16

A synthesis scheme of a compound 16 is shown below.

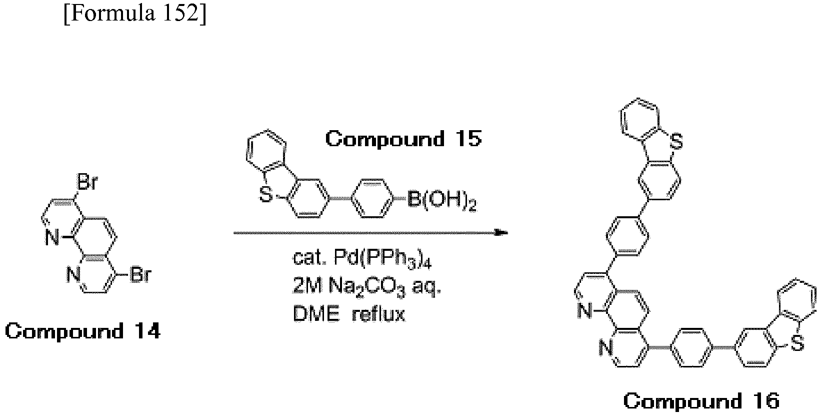

The compound 16 (8.6 g, a yield of 83%) was obtained as a white solid in the same manner as in (1-3) synthesis of the compound 5, except for using the compound 14 (5.0 g, 15 mmol) in place of the compound 3 and using the compound 15 (9.9 g, 33 mmol) in place of the compound 4. By FD-MS (Field Desorption Mass Spectrometry) analysis, the obtained compound was identified to be the compound 16.

### (6) Synthesis Example 6: Synthesis of Compound 19

A synthesis scheme of a compound 19 is shown below.

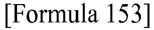

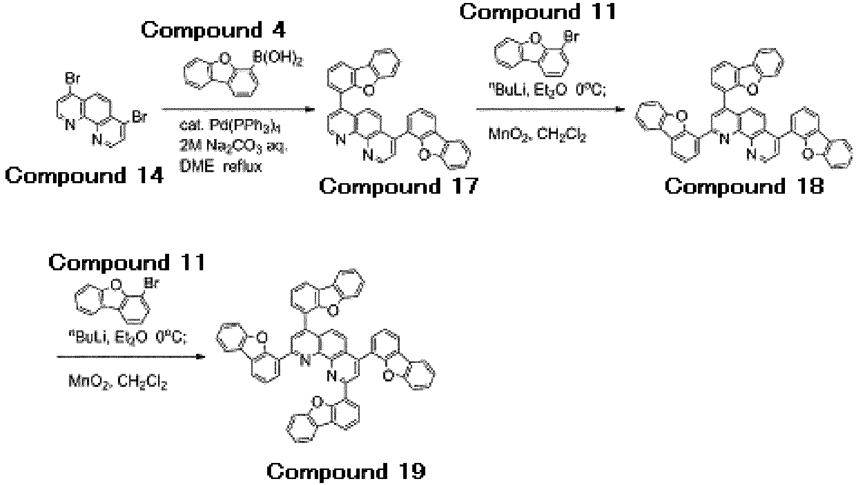

### (6-1) Synthesis of Compound 17

A compound 17 (13 g, a yield of 86%) was obtained as a white solid in the same manner as in the (1-3) synthesis of the compound 5, except for using the compound 14 (10 g, 30 mmol) in place of the compound 3. By FD-MS (Field Desorption Mass Spectrometry) analysis, the obtained compound was identified to be the compound 17.

### (6-2) Synthesis of Compound 18

A compound 18 (9.6 g, a yield of 56%) was obtained as a yellow solid in the same manner as in (1-1) synthesis of the compound 2, except for using the compound 17 (13 g, 25 mmol) in place of the compound 1 and using the compound 11 (16 g, 63 mmol) in place of 1,4-dibromobenzene. By FD-MS (Field Desorption Mass Spectrometry) analysis, the obtained compound was identified to be the compound 18.

### (6-3) Synthesis of Compound 19

A compound 19 (3.2 g, a yield of 27%) was obtained as a light yellow solid in the same manner as in (1-1) synthesis of the compound 2, except for using the compound 18 (9.6 g, 14 mmol) in place of the compound 1 and using the compound 11 (8.7 g, 35 mmol) in place of 1,4-dibromobenzene. By FD-MS (Field Desorption Mass Spectrometry) analysis, the obtained compound was identified to be the compound 19.

### (7) Synthesis Example 7: Synthesis of Compound 22

A synthesis scheme of a compound 22 is shown below.

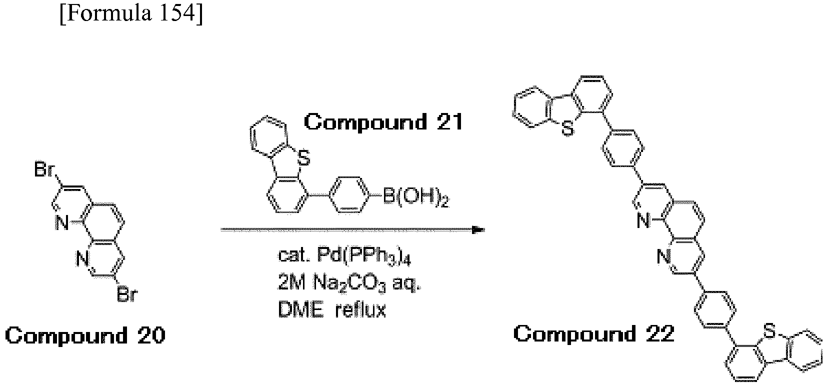

### (7-1) Synthesis of Compound 22

The compound 22 (6.5 g, a yield of 63%) was obtained as a white solid in the same manner as in (1-3) synthesis of the compound 5, except for using the compound 20 (5.0 g, 15 mmol) in place of the compound 3 and using the compound 21 (9.9 g, 33 mmol) in place of the compound 4. By FD-MS (Field Desorption Mass Spectrometry) analysis, the obtained compound was identified to be the compound 22.

### (8) Synthesis Example 8: Synthesis of Compound 26

A synthesis scheme of a compound 26 is shown below.

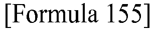

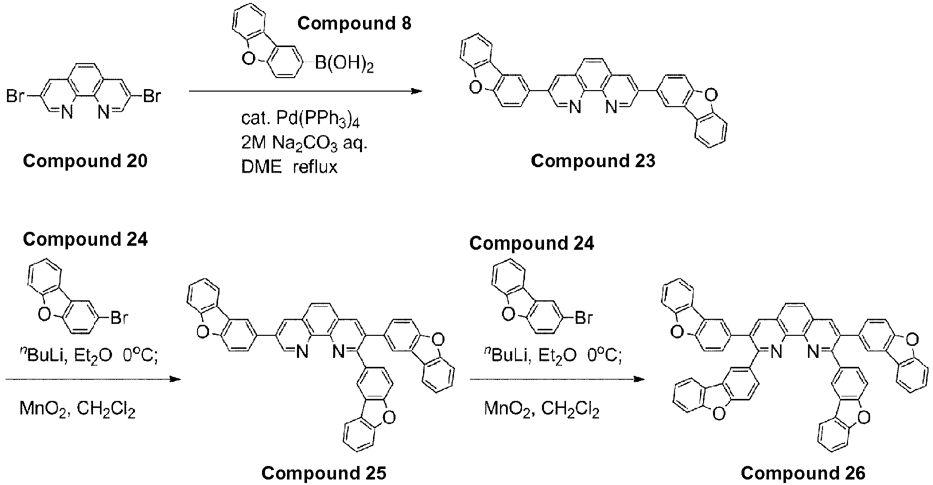

### (8-1) Synthesis of Compound 23

A compound 23 (13 g, a yield of 89%) was obtained as a white solid in the same manner as in (1-3) synthesis of the compound 5, except for using the compound 20 (10 g, 30 mmol) in place of the compound 3 and using the compound 8 (16 g, 65 mmol) in place of the compound 4. By FD-MS (Field Desorption Mass Spectrometry) analysis, the obtained compound was identified to be the compound 23.

### (8-2) Synthesis of Compound 25

A compound 25 (6.2 g, a yield of 36%) was obtained as a yellow solid in the same manner as in (1-1) synthesis of the compound 2, except for using the compound 23 (13 g, 25 mmol) in place of the compound 1 and using the compound 24 (16 g, 63 mmol) in place of 1,4-dibromobenzene. By FD-MS (Field Desorption Mass Spectrometry) analysis, the obtained compound was identified to be the compound 25.

### (8-3) Synthesis of Compound 26

A compound 26 (2.1 g, a yield of 27%) was obtained as a light yellow solid in the same manner as in (1-1) synthesis of the compound 2, except for using the compound 25 (6.2 g, 9.1 mmol) in place of the compound 1 and using the compound 24 (5.6 g, 23 mmol) in place of 1,4-dibromobenzene. By FD-MS (Field Desorption Mass Spectrometry) analysis, the obtained compound was identified to be the compound 26.

### (9) Synthesis Example 9: Synthesis of Compound 29 (Reference Example, which is not according to our present invention)

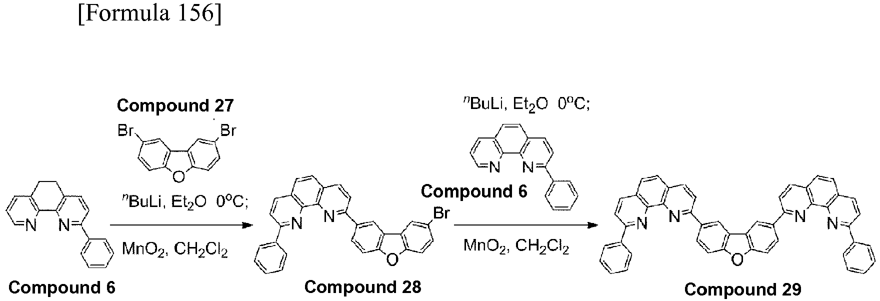

### (9-1) Synthesis of Compound 28

A compound 28 (7.6 g, a yield of 98%) was obtained as a brown oily substance in the same manner as in (1-1) synthesis of the compound 2, except for using the compound 6 (4.0 g, 16 mmol) in place of the compound 1, using the compound 27 (10 g, 31 mmol) in place of 1,4-dibromobenzene, and using n-butyllithium at 0.95 molar equivalent to the compound 1. By FD-MS (Field Desorption Mass Spectrometry) analysis, the obtained compound was identified to be the compound 28.

### (9-2) Synthesis of Compound 29

A compound 29 (4.9 g, a yield of 48%) was obtained as a light yellow solid in the same manner as in (1-1) synthesis of the compound 2, except for using the compound 6 (4.7 g, 18 mmol) in place of the compound 1 and using the compound 28 (7.6 g, 15 mmol) in place of 1,4-dibromobenzene. By FD-MS (Field Desorption Mass Spectrometry) analysis, the obtained compound was identified to be the compound 29.

### (9A) Synthesis Example 9A: Synthesis of Compound 1A (Reference Example, which is not according to our present invention)

A synthesis scheme of a compound 1A is shown below.

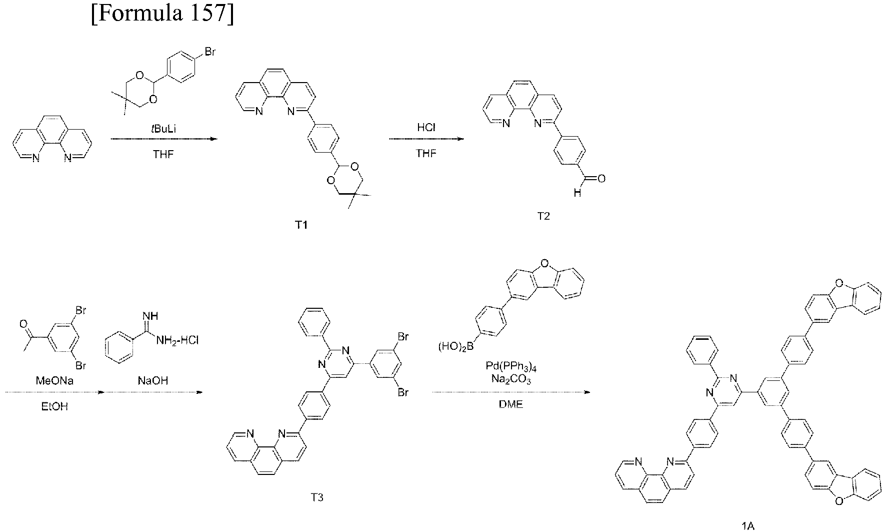

### (9A-1) Synthesis of Compound T1

Under an argon gas atmosphere, p-(5,5-dimethyl-1,3-dioxane-2-yl)bromobenzene (15.4 g, 56.8 mmol) was dissolved in tetrahydrofuran (100 mL). After the obtained solution was cooled to minus 78 degrees C, t-butyllithium (1.3 M hexane solution) (45.9 mL, 59.6 mmol) was dropped in the cooled solution for 20 minutes. The obtained mixture solution was further stirred for two hours. Subsequently, the mixture solution was gradually heated up to 0 degrees C. To the obtained solution, a solution in which 1,10-phenanthroline (10.2 g, 56.8 mmol) was dissolved in tetrahydrofuran (50 mL) was added. The obtained mixture solution was stirred for eight hours at the room temperature. After the completion of the reaction, water (80 mL) was added at 0 degrees C to the obtained solution. The solution was extracted with dichloromethane, to which manganese dioxide (60 g) was added. After being stirred for four hours at the room temperature, the obtained solution was dried with magnesium sulfate and a solvent was distilled under reduced pressure. A residue was purified by silica-gel column chromatography, so that a compound T1 (8.41 g, a yield of 40%) was obtained.

### (9A-2) Synthesis of Compound T2

The compound T1 (8.30 g, 22.4 mmol) was dissolved in tetrahydrofuran (500 mL), to which hydrochloric acid (5% aqueous solution)(125 mL, 200 mmol) was added. The obtained solution was stirred at 50 degrees C for 15 hours. Subsequently, a solid obtained after the reaction was dissolved in a mixture of water and dichloromethane. The obtained solution was neutralized with aqueous solution of sodium hydrogencarbonate. After being extracted with dichloromethane, the obtained solution was dried with magnesium sulfate and a solvent was distilled under reduced pressure to obtain a compound T2 (5.99 g, a yield of 94%).

### (1-3) Synthesis of Compound T3

Under an argon gas atmosphere, the compound T2 (5.80 g, 20.4 mmol), 3,5-dibromo-4-hydroxyacetophenone (5.67 g, 20.4 mmol) and sodium methoxide (110 mg, 2.04 mmol) were added to ethanol (50 mL). The obtained solution was stirred at the room temperature for two hours and at the reflux temperature for three hours. Subsequently, benzamidine hydrochloride (3.29 g, 21.0 mmol) and sodium hydroxide (1.63 g, 40.8 mmol) were added to the reaction solution. The obtained solution was stirred at the reflux temperature for three hours. After the completion of the reaction, a deposit was separated by filtration. Then, the obtained solid was washed with water and methanol to obtain a compound T3 (7.75 g, a yield of 38%).

### (1-4) Synthesis of Compound 1A

Under an argon gas atmosphere, 4-(2-dibenzofuranyl)phenylboronic acid (4.92 g, 17.1 mmol), the compound T3 (5.00 g, 7.76 mmol), tetrakistriphenylphosphine palladium (896 mg, 0.776 mmol), 1,2-dimethoxyethane (100 mL) and a sodium carbonate aqueous solution (2M, 100 mL) were mixed and stirred while being heated to reflux for six hours. After the reaction solution was cooled down to the room temperature, the reaction solution was extracted with toluene. After an aqueous phase was removed, an organic phase was washed with saturated saline. After the organic phase was drided with magnesium sulfate and concentrated, the obtained residue was purified by silica-gel column chromatography, so that the compound 1A (3.77 g, a yield of 50%) was obtained. As a result of mass analysis, this compound was a target object and m/e was equal to 970 while a calculated molecular weight was 971.13.

### (2) Synthesis of Compound 1B

A synthesis scheme of a compound 1B is shown below.

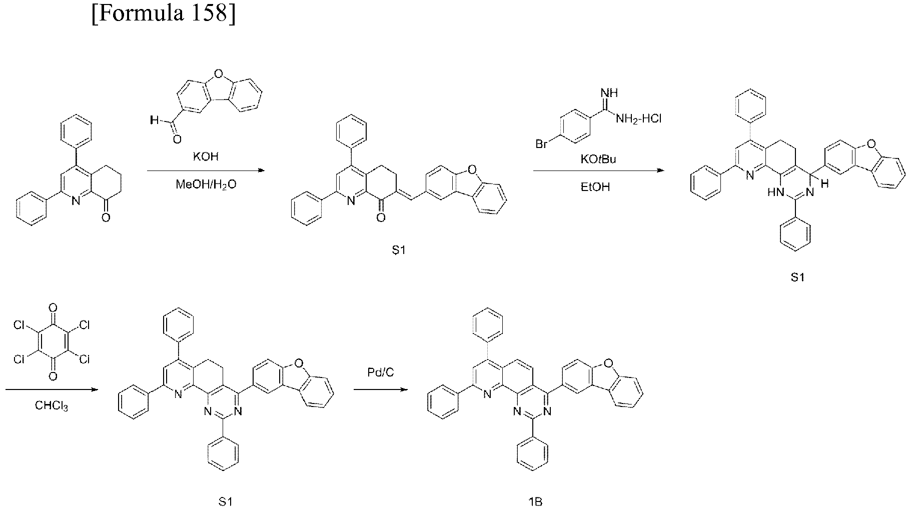

A synthesis according to this scheme was conducted with reference to Examples described in Japanese Patent No. 5113571. As a result of mass analysis, this compound was a target object and m/e was equal to 575 while a calculated molecular weight was 575.67.

### Example 1

### (1) Manufacturing of Organic EL Device

A glass substrate (size: 25 mm×75 mm×0.7 mm thick, manufactured by Geomatec Co., Ltd.) having an ITO transparent electrode (anode) was ultrasonic-cleaned in isopropyl alcohol for five minutes, and then UV/ozone-cleaned for 30 minutes.

After the glass substrate having the transparent electrode line was cleaned, the glass substrate was attached to a substrate holder of a vacuum evaporation apparatus. Initially, a compound HT-1 was deposited on a surface of the glass substrate so as to cover the transparent electrode line, thereby forming a 50 nm thick film of the compound HT1. The HT1 film serves as a hole injecting layer. A compound HT-2 was deposited on the HT-1 film to form a 45 nm thick HT-2 film. The HT2 film serves as a hole transporting layer.

Then, a compound BH-1 (host material) and a compound BD-1 (dopant material) were co-deposited on the HT-2 film at a film thickness ratio at which the compound BD-1 was at 3 mass%, so that a 20 nm thick organic layer was formed. The organic layer serves as the emitting layer. The compound 5 and lithium (Li) were deposited on the emitting layer at a film thickness ratio at which Li was 2 mass%, so that a 30 nm thick electron transporting layer was formed on the emitting layer. Metal (Al) was deposited on the electron transporting layer to form an 80 nm thick metal cathode, so that the organic electroluminescence device was manufactured.

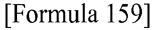

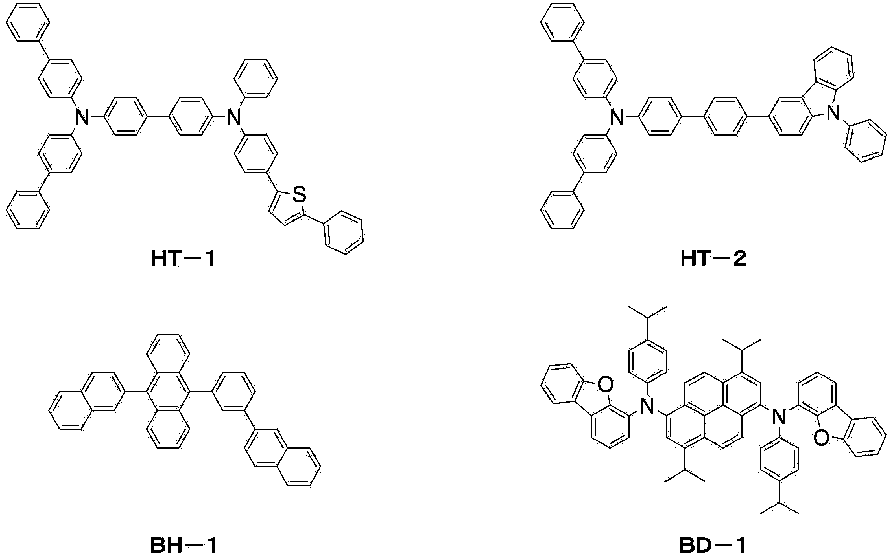

### (2) Evaluation of Organic EL Device

Voltage was applied on the manufactured organic EL devices such that a current density was 10 mA/cm², where a value (V) of the voltage was measured. The results are shown in Table 1.

### Examples 2 to 3 and Comparative 1

The organic EL devices according to Examples 2 to 3 and Comparative 1 were manufactured in the same manner as in Example 1 except that the compound 9 (Example 2 as a Reference Example, which is not according to our present invention), the compound 10 (Example 3) and a compound ET-1 below (Comparative 1) were used in place of the compound 5 to form the electron transporting layer. The results are shown in Table 1.

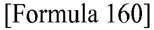

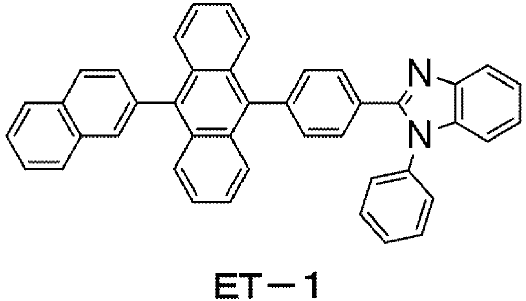

**Table 1**

| | Electron Transporting Layer | Drive Voltage (V) |
|---|---|---|
| Example 1 | Compound 5 | 3.4 |
| Example 2 | Compound 9 | 3.2 |
| Example 3 | Compound 10 | 3.5 |
| Comparative 1 | Compound ET-1 | 3.7 |

### Examples 4 to 8 and Comparative 2

The organic EL device in Example 4 was manufactured in the same manner as in Example 1 except that the amount of lithium doped for forming the electron transporting layer in Example 1 was changed to 5 mass%.

The organic EL devices in Examples 5 to 8 and Comparative 2 were manufactured in the same manner as in Example 1 except that the amount of lithium doped for forming the electron transporting layer in Example 1 was changed to 5 mass% and that the compound 5 to be used for the electron transporting layer was replaced by the compound 9 (Example 5 as a Reference Example, which is not according to our present invention), the compound 13 (Example 6), the compound 1C (Example 7), the compound 1D (Example 8 as a Reference Example, which is not according to our present invention) and a compound ET-2 below (Comparative 2).

The manufactured organic EL devices were driven by DC constant current (50 mA/cm²) at the room temperature to emit light. Time elapsed until a luminance intensity was decreased to 80% was measured and a lifetime (80%LT) was evaluated. The results are shown in Table 2.

Voltage was applied on each of the manufactured organic EL devices such that the current density was 10 mA/cm², where spectral radiance spectrum was measured using a spectroradiometer CS-1000 (manufactured by Konica Minolta, Inc.). The external quantum efficiency EQE (unit: %) was calculated based on the obtained spectral-radiance spectra, assuming that the spectra was provided under a Lambertian radiation. The results are shown in Table 2.

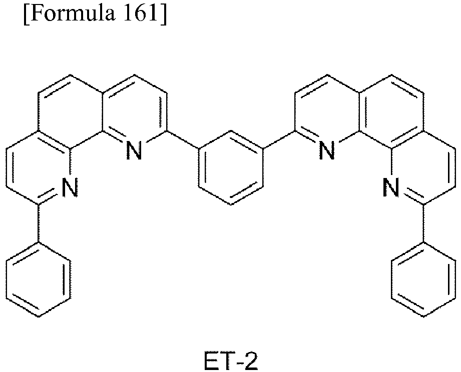

**Table 2**

| | Electron Transporting Layer | Lifetime (80%LT) (hr) | External Quantum Efficiency (%) | Emission Color |
|---|---|---|---|---|
| Example 4 | Compound 5 | 350 | 7.41 | Blue |
| Example 5 | Compound 9 | 290 | 7.10 | Blue |
| Example 6 | Compound 13 | 230 | 7.51 | Blue |
| Example 7 | Compound 1C | 320 | 7.09 | Blue |
| Example 8 | Compound 1D | 310 | 7.52 | Blue |
| Comparative 2 | Compound ET-2 | 220 | 7.21 | Blue |

Table 2 shows that the lifetime of the organic EL device can be prolonged by using the compound of the invention. The organic EL device using a combined derivative of phenanthroline and one of dibenzofuran and dibenzothiophene exhibited a longer lifetime than the compound (ET-2) used in Comparative 2.
Moreover, in comparison between the organic EL devices of Example 8 and Comparative 2, both of which have particularly similar structures, the organic EL device of Example 8 using a combined derivative of phenanthroline and dibenzofuran exhibited a longer lifetime and a higher efficiency than the compound (ET2) used in Comparative 2.

Further, in comparison between the organic EL devices of Examples 6 and 7, the organic EL device using phenanthroline substituted by dibenzofurans at the respective 4 positions (i.e., the compound 13) exhibited a higher efficiency while the organic EL device using phenanthroline substituted by dibenzofurans at the respective 2 positions (i.e., the compound 1C) exhibited a longer lifetime.

## Claims

1. A compound represented by a formula (1) below, 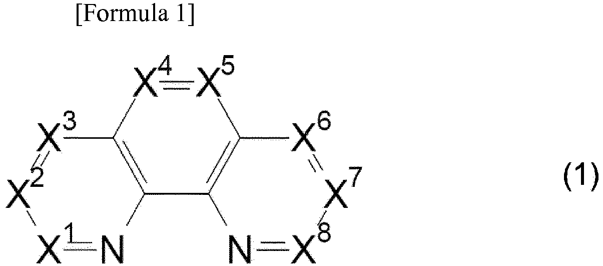
where: X¹ to X⁸ each independently represent
a carbon atom to be bonded to a group represented by a formula (2) below,
CR^{X},
or a nitrogen atom;
at least two of X¹ to X⁸ are carbon atoms to be each bonded to the group represented by the formula (2);
R^{x} is independently selected from the group consisting of a hydrogen atom, a halogen atom, a cyano group, a nitro group, a substituted or unsubstituted hydroxyl group, a substituted or unsubstituted carboxyl group, a substituted or unsubstituted sulfonyl group, a substituted or unsubstituted boryl group, a substituted or unsubstituted phosphino group, a substituted or unsubstituted mercapto group, a substituted or unsubstituted acyl group, a substituted or unsubstituted amino group, a substituted or unsubstituted silyl group, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted aralkyl group having 6 to 30 carbon atoms, a substituted or unsubstituted aryl group having 6 to 40 ring carbon atoms, and a substituted or unsubstituted heteroaryl group having 5 to 40 ring atoms; and
R^{x} of adjacent ones of CR^{X} in X¹ to X⁸ are optionally bonded to each other to form a cyclic structure or are not optionally bonded, 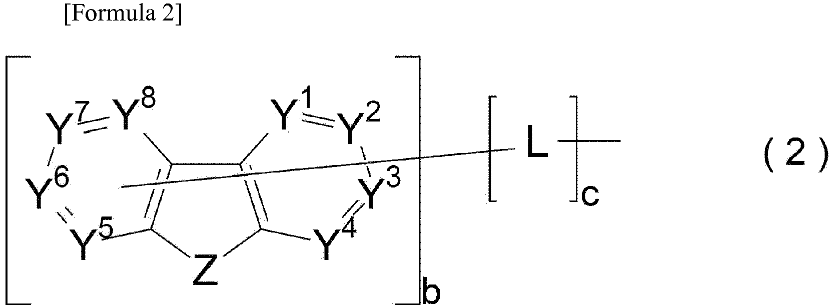
where: b is an integer of 1 to 5; c is an integer of 1 to 8;
Z represents an oxygen atom or sulfur atom;
when b is from 2 to 5, a plurality of Z are mutually the same or different;
L is selected from a single bond and a linking group, wherein
the linking group represents a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms;
the polyvalent heteroaryl group having 5 to 40 ring atoms in L of the formula (2) comprises a substituted or unsubstituted polyvalent group derived from a phenanthroline ring represented by the formula (1);
when c is from 2 to 8, a plurality of L are mutually the same or different;
Y¹ to Y⁸ each independently represent a nitrogen atom, CR^{Y} or a carbon atom to be bonded to L;
R^{Y} represents the same as R^{X} in the formula (1);
the heteroaryl group having 5 to 40 ring atoms in R^{Y} comprises a substituted or unsubstituted phenanthrolyl group derived from the phenanthroline ring represented by the formula (1);
adjacent ones of R^{Y} are optionally bonded to each other to form a cyclic structure or are not optionally bonded;
when X¹ or X⁸ is a carbon atom to be bonded to the group represented by the formula (2) in which b is 1, Z is an oxygen atom, Y⁴ or Y⁵ is a carbon atom to be bonded to L, and c is 2, one of two L, which is near the phenanthroline ring represented by the formula (1), is a divalent group other than an anthracene group;
when two of X¹ to X⁸ are carbon atoms to be each bonded to the group represented by the formula (2) in which b and c are 1, Z is a sulfur atom, Y⁴ or Y⁵ is a carbon atom to be bonded to L, and L is a p-phenylene group, L is bonded to one of X¹, X², X⁴, X⁵, X⁷ and X⁸;
when X¹ or X⁸ is a carbon atom to be bonded to the group represented by the formula (2) in which b and c are 1, Z is an oxygen atom or sulfur atom, Y³ is a carbon atom to be bonded to L, and L is a p-phenylene group, R^{Y} in Y⁴ is a group other than a phenyl group;
when X¹ or X⁸ is a carbon atom to be bonded to the group represented by the formula (2) in which b and c are 1, Z is an oxygen atom or sulfur atom, Y⁶ is a carbon atom to be bonded to L, and L is a p-phenylene group, R^{Y} in Y⁵ is a group other than a phenyl group;
when X⁴ or X⁵ is a carbon atom to be bonded to the group represented by the formula (2) in which Y² is a carbon atom to be bonded to L, and L is a single bond and Z is an oxygen atom, R^{Y} in Y⁷ is a group other than a pyrenyl group;
when X⁴ or X⁵ is a carbon atom to be bonded to the group represented by the formula (2) in which Y⁷ is a carbon atom to be bonded to L, and L is a single bond and Z is an oxygen atom, R^{Y} in Y² is a group other than a pyrenyl group; and
a substituent for the above substituted or unsubstituted groups is selected from the group consisting of an aryl group, heteroaryl group, alkyl group, halogen atom, alkylsilyl group, arylsilyl group and cyano group.

2. The compound according to claim 1, wherein
L is selected from a single bond and a linking group, wherein
the linking group represents a substituted or unsubstituted polyvalent linear, branched or cyclic aliphatic hydrocarbon group having 1 to 30 carbon atoms, or a substituted or unsubstituted polyvalent aryl group having 6 to 40 ring carbon atoms.

3. The compound according to claim 1 or 2, wherein
X¹ or X⁸ in the formula (1) is a carbon atom to be bonded to the group represented by the formula (2).

4. The compound according to claim 1 or 2, wherein
X¹ and X⁸ in the formula (1) are carbon atoms to be each bonded to the group represented by the formula (2).

5. The compound according to claim 1 or 2, wherein
X³ and X⁶ in the formula (1) are carbon atoms to be each bonded to the group represented by the formula (2).

6. The compound according to claim 1 or 2, wherein
X² or X⁷ in the formula (1) is a carbon atom to be bonded to the group represented by the formula (2).

7. The compound according to claim 1 or 2, wherein
X³ or X⁶ in the formula (1) is a carbon atom to be bonded to the group represented by the formula (2).

8. The compound according to claim 1 or 2, wherein
X⁴ or X⁵ in the formula (1) is a carbon atom to be bonded to the group represented by the formula (2).

9. The compound according to claim 1 or 2, wherein
X¹ and X⁷ in the formula (1) are carbon atoms to be each bonded to the group represented by the formula (2).

10. The compound according to claim 1 or 2, wherein
X² and X⁷ in the formula (1) are carbon atoms to be each bonded to the group represented by the formula (2).

11. The compound according to claim 1 or 2, wherein
X¹, X², X⁷ and X⁸ in the formula (1) are carbon atoms to be each bonded to the group represented by the formula (2).

12. The compound according to claim 1 or 2, wherein
X¹, X³, X⁶ and X⁸ in the formula (1) are carbon atoms to be each bonded to the group represented by the formula (2).

13. The compound according to any one of claims 1 to 12, wherein the compound represented by the formula (1) is represented by a formula (1-1) below, 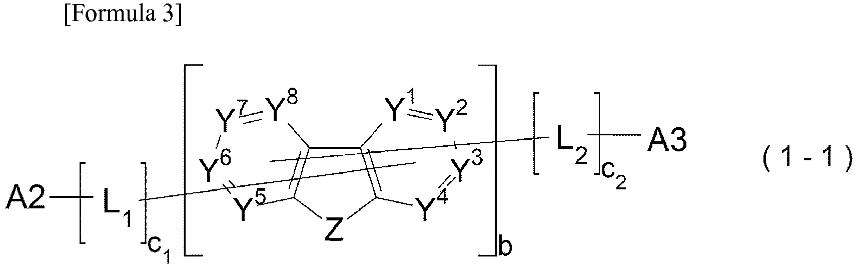 where:
A2 and A3 each independently represent a structure represented by the formula (1);
c₁ is an integer of 1 to 8 and c₂ is an integer of 1 to 8; L₁ and L₂ each independently represent the same as L in the formula (2); and X¹ to X⁸ in A2 and A3, Y¹ to Y⁸, Z and b respectively represent the same as X¹ to X⁸, Y¹ to Y⁸, Z and b in the formulae (1) and (2).

14. The compound according to any one of claims 1 to 13, wherein
the rest of X¹ to X⁸ in the formula (1) except for the carbon atom to be bonded to the group represented by the formula (2) are CR^{X}.

15. The compound according to any one of claims 1 to 14, wherein
Z in the formula (2) is an oxygen atom.

16. The compound according to any one of claims 1 to 15, wherein
c in the formula (2) is an integer of 1 to 5.

17. Use of the compound according to any one of claims 1 to 16 in an organic compound layer of an organic electroluminescence device comprising an anode, a cathode, and the organic compound layer disposed between the anode and the cathode.

18. Use of the compound according to any one of claims 1 to 16 in an electron transporting layer of an organic electroluminescence device comprising an anode, a cathode, and an emitting layer and the electron transporting layer between the anode and the cathode.

19. A material for an organic electroluminescence device, comprising: the compound according to any one of claims 1 to 16.

20. An organic electroluminescence device comprising:
an anode;
a cathode opposite the anode; and
an organic compound layer disposed between the anode and the cathode,
the organic compound layer comprising an emitting layer and an electron transporting layer provided on a side of the emitting layer near the cathode,
the electron transporting layer comprising a compound represented by a formula (10) below, 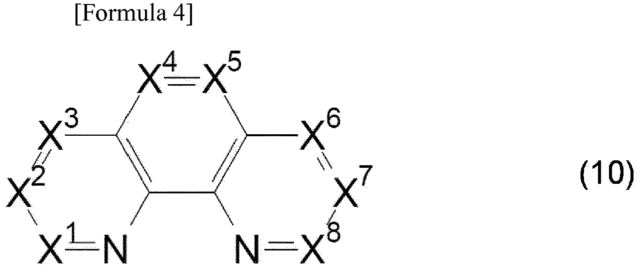
where: X¹ to X⁸ each independently represent
a carbon atom to be bonded to a group represented by a formula (20) below,
CR^{X}, or
a nitrogen atom;
at least two of X¹ to X⁸ are carbon atoms to be each bonded to the group represented by the formula (20);
R^{x} is independently selected from the group consisting of a hydrogen atom, a halogen atom, a cyano group, a nitro group, a substituted or unsubstituted hydroxyl group, a substituted or unsubstituted carboxyl group, a substituted or unsubstituted sulfonyl group, a substituted or unsubstituted boryl group, a substituted or unsubstituted phosphino group, a substituted or unsubstituted mercapto group, a substituted or unsubstituted acyl group, a substituted or unsubstituted amino group, a substituted or unsubstituted silyl group, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted aralkyl group having 6 to 30 carbon atoms, a substituted or unsubstituted aryl group having 6 to 40 ring carbon atoms, and a substituted or unsubstituted heteroaryl group having 5 to 40 ring atoms; and
R^{X} of adjacent ones of CR^{X} in X¹ to X⁸ are optionally bonded to each other to form a cyclic structure or are not optionally bonded, 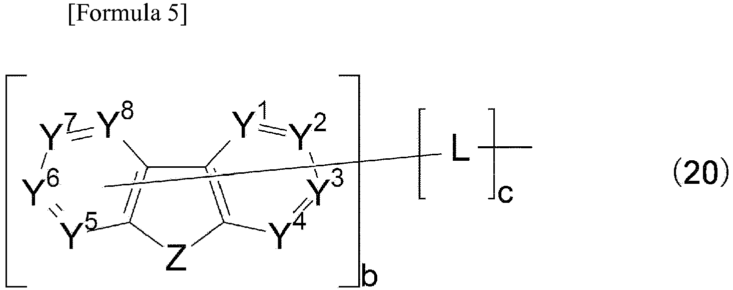
where: b is an integer of 1 to 5;
c is an integer of 1 to 8;
Z represents an oxygen atom or sulfur atom;
when b is from 2 to 5, a plurality of Z are mutually the same or different;
L is selected from a single bond and a linking group, wherein
the linking group represents a substituted or unsubstituted polyvalent linear, branched or cyclic aliphatic hydrocarbon group having 1 to 30 carbon atoms, a substituted or unsubstituted polyvalent aryl group having 6 to 40 ring carbon atoms, or a substituted or unsubstituted polyvalent heteroaryl group having 5 to 40 ring atoms;
the polyvalent heteroaryl group having 5 to 40 ring atoms in L of the formula (20) comprises a substituted or unsubstituted polyvalent group derived from a phenanthroline ring represented by the formula (10);
when c is from 2 to 8, a plurality of L are mutually the same or different;
Y¹ to Y⁸ each independently represent a nitrogen atom, CR^{Y} or a carbon atom to be bonded to L;
R^{Y} represents the same as R^{X} in the formula (10);
the heteroaryl group having 5 to 40 ring atoms in R^{Y} comprises a substituted or unsubstituted phenanthrolyl group derived from the phenanthroline ring represented by the formula (10); and
adjacent ones of R^{Y} are optionally bonded to each other to form a cyclic structure or are not optionally bonded.

21. The organic electroluminescence device according to claim 20, wherein
L is selected from a single bond and a linking group, wherein
the linking group represents a substituted or unsubstituted polyvalent linear, branched or cyclic aliphatic hydrocarbon group having 1 to 30 carbon atoms, or a substituted or unsubstituted polyvalent aryl group having 6 to 40 ring carbon atoms.

22. The organic electroluminescence device according to claim 20 or 21, wherein
X¹ or X⁸ in the formula (10) is a carbon atom to be bonded to the group represented by the formula (20).

23. The organic electroluminescence device according to claim 20 or 21, wherein
X¹ and X⁸ in the formula (10) are carbon atoms to be each bonded to the group represented by the formula (20).

24. The organic electroluminescence device according to claim 20 or 21, wherein
X³ and X⁶ in the formula (10) are carbon atoms to be each bonded to the group represented by the formula (20).

25. The organic electroluminescence device according to claim 20 or 21, wherein
X² or X⁷ in the formula (10) is a carbon atom to be bonded to the group represented by the formula (20).

26. The organic electroluminescence device according to claim 20 or 21, wherein
X³ or X⁶ in the formula (10) is a carbon atom to be bonded to the group represented by the formula (20).

27. The organic electroluminescence device according to claim 20 or 21, wherein
X⁴ or X⁵ in the formula (10) is a carbon atom to be bonded to the group represented by the formula (20).

28. The organic electroluminescence device according to claim 20 or 21, wherein
X¹ and X⁷ in the formula (10) are carbon atoms to be each bonded to the group represented by the formula (20).

29. The organic electroluminescence device according to claim 20 or 21, wherein
X² and X⁷ in the formula (10) are carbon atoms to be each bonded to the group represented by the formula (20).

30. The organic electroluminescence device according to claim 20 or 21, wherein
X¹, X², X⁷ and X⁸ in the formula (10) are carbon atoms to be each bonded to the group represented by the formula (20).

31. The organic electroluminescence device according to claim 20 or 21, wherein
X¹, X³, X⁶ and X⁸ in the formula (10) are carbon atoms to be each bonded to the group represented by the formula (20).

32. The organic electroluminescence device according to any one of claims 20 to 31, wherein
the compound represented by the formula (10) is represented by a formula (10-1) below, 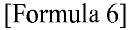 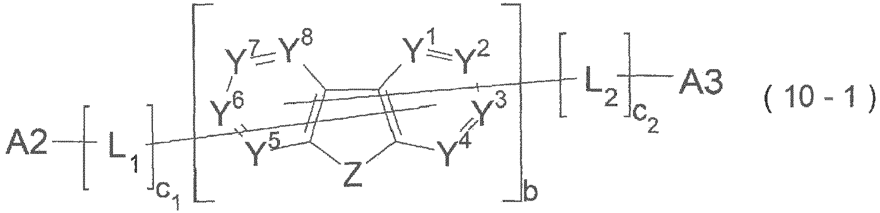
where: A2 and A3 each independently represent a structure represented by the formula (10);
c₁ is an integer of 1 to 8 and c₂ is an integer of 1 to 8;
L₁ and L₂ each independently represent the same as L in the formula (20); and
X¹ to X⁸ in A2 and A3, Y¹ to Y⁸, Z and b respectively represent the same as X¹ to X⁸, Y¹ to Y⁸, Z and b in the formulae (10) and (20).

33. The organic electroluminescence device according to any one of claims 20 to 32, wherein
the rest of X¹ to X⁸ in the formula (10) except for the carbon atom to be bonded to the group represented by the formula (20) are CR^{X}.

34. The organic electroluminescence device according to any one of claims 20 to 33, wherein
Z in the formula (20) is an oxygen atom.

35. The organic electroluminescence device according to any one of claims 20 to 34, further comprising a blocking layer between the electron transporting layer and the emitting layer.

36. The organic electroluminescence device according to any one of claims 20 to 34, wherein
the electron transporting layer is adjacent to the emitting layer.

37. The organic electroluminescence device according to any one of claims 20 to 36, wherein
the electron transporting layer comprises at least one of an electron-donating dopant and an organic metal complex.

38. An electronic device comprising the organic electroluminescence device according to any one of claims 20 to 37.

## Patentansprüche

1. Eine Verbindung der nachstehenden Formel (1) 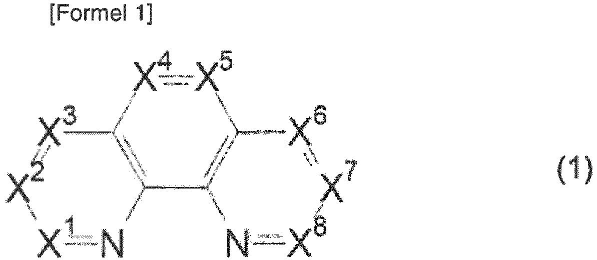
worin: X¹ bis X⁸ jeweils unabhängig voneinander darstellen
ein Kohlenstoffatom, das an eine durch die nachstehende Formel (2) dargestellte Gruppe gebunden ist,
CR^{X},
oder ein Stickstoffatom;
mindestens zwei von X¹ bis X⁸ sind Kohlenstoffatome, die jeweils an die durch die Formel (2) dargestellte Gruppe gebunden sind;
R^{x} ist jeweils unabhängig voneinander ausgewählt aus der Gruppe bestehend aus einem Wasserstoffatom, einem Halogenatom, einer Cyanogruppe, einer Nitrogruppe, einer substituierten oder unsubstituierten Hydroxylgruppe, einer substituierten oder unsubstituierten Carboxylgruppe, einer substituierten oder unsubstituierten Sulfonylgruppe, einer substituierten oder unsubstituierten Borylgruppe, einer substituierten oder unsubstituierten Phosphinogruppe, einer substituierten oder unsubstituierten Mercaptogruppe, einer substituierten oder unsubstituierten Acylgruppe, einer substituierten oder unsubstituierten Aminogruppe, einer substituierten oder unsubstituierten Silylgruppe, einer substituierten oder unsubstituierten Alkylgruppe mit 1 bis 30 Kohlenstoffatomen, einer substituierten oder unsubstituierten Alkenylgruppe mit 2 bis 30 Kohlenstoffatomen, einer substituierten oder unsubstituierten Alkinylgruppe mit 2 bis 30 Kohlenstoffatomen, einer substituierten oder unsubstituierten Aralkylgruppe mit 6 bis 30 Kohlenstoffatomen, einer substituierten oder unsubstituierten Arylgruppe mit 6 bis 40 Ringkohlenstoffatomen und einer substituierten oder unsubstituierten Heteroarylgruppe mit 5 bis 40 Ringatomen; und
R^{X} von benachbarten CR^{X} in X¹ bis X⁸ sind gegebenenfalls miteinander verbunden, unter Bildung einer cyclischen Struktur, oder sind nicht gegebenenfalls verbunden, 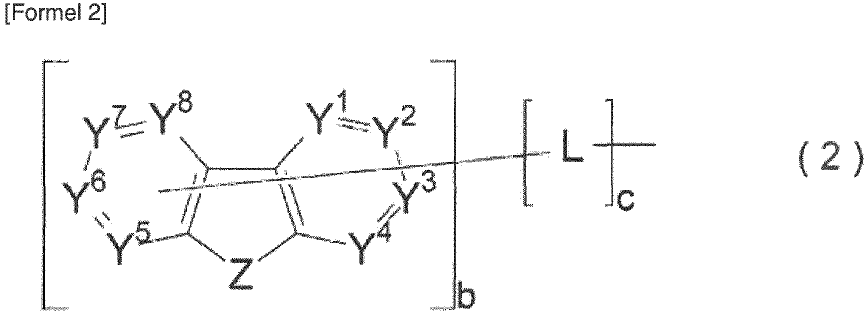
worin: b eine ganze Zahl von 1 bis 5 ist; c eine ganze Zahl von 1 bis 8 ist;
Z stellt ein Sauerstoffatom oder ein Schwefelatom dar;
wenn b 2 bis 5 ist, sind mehrere Z gleich oder verschieden;
L ist ausgewählt aus einer Einfachbindung und einer verbindenden Gruppe, worin
die verbindende Gruppe eine substituierte oder unsubstituierte mehrwertige lineare, verzweigte oder cyclische aliphatische Kohlenwasserstoffgruppe mit 1 bis 30 Kohlenstoffatomen, eine substituierte oder unsubstituierte mehrwertige Arylgruppe mit 6 bis 40 Ringkohlenstoffatomen oder eine substituierte oder unsubstituierte mehrwertige Heteroarylgruppe mit 5 bis 40 Ringatome darstellt;
die mehrwertige Heteroarylgruppe mit 5 bis 40 Ringatomen in L der Formel (2) umfasst eine substituierte oder unsubstituierte mehrwertige Gruppe, die von einem Phenanthrolinring der Formel (1) abgeleitet ist;
wenn c 2 bis 8 ist, sind mehrere L gleich oder verschieden;
Y¹ bis Y⁸ stellen jeweils unabhängig voneinander ein Stickstoffatom, CR^{Y} oder ein an L gebundenes Kohlenstoffatom dar;
R^{Y} stellt dasselbe wie R^{X} in der Formel (1) dar;
die Heteroarylgruppe mit 5 bis 40 Ringatomen in R^{Y} umfasst eine substituierte oder unsubstituierte Phenanthrolylgruppe, die von dem Phenanthrolinring der Formel (1) abgeleitet ist;
benachbarte R^{Y} sind gegebenenfalls miteinander verbunden, unter Bildung einer cyclischen Struktur, oder sind nicht gegebenenfalls verbunden;
wenn X¹ oder X⁸ ein Kohlenstoffatom ist, das an die durch die Formel (2) dargestellte Gruppe gebunden ist, worin b 1 ist, Z ein Sauerstoffatom ist, Y⁴ oder Y⁵ ein an L gebundenes Kohlenstoffatom ist, und c 2 ist, dann ist eines von zwei L, das nahe dem durch die Formel (1) dargestellten Phenanthrolinring ist, eine andere zweiwertige Gruppe als eine Anthracengruppe;
wenn zwei von X¹ bis X⁸ Kohlenstoffatome sind, die an die durch die Formel (2) dargestellte Gruppe gebunden sind, worin b und c 1 sind, Z ein Schwefelatom ist, Y⁴ oder Y⁵ ein an L gebundenes Kohlenstoffatom ist, und L eine p-Phenylengruppe ist, dann ist L an eines von X¹, X², X⁴, X⁵, X⁷ und X⁸ gebunden;
wenn X¹ oder X⁸ ein Kohlenstoffatom ist, das an die durch die Formel (2) dargestellte Gruppe gebunden ist, worin b und c 1 sind, Z ein Sauerstoffatom oder Schwefelatom ist, Y³ ein an L gebundenes Kohlenstoffatom ist, und L eine p-Phenylengruppe ist, dann ist R^{Y} in Y⁴ eine andere Gruppe als eine Phenylgruppe;
wenn X¹ oder X⁸ ein Kohlenstoffatom ist, das an die durch die Formel (2) dargestellte Gruppe gebunden ist, worin b und c 1 sind, Z ein Sauerstoffatom oder Schwefelatom ist, Y⁶ ein an L gebundenes Kohlenstoffatom ist, und L eine p-Phenylengruppe ist, dann ist R^{Y} in Y⁵ eine andere Gruppe als eine Phenylgruppe;
wenn X⁴ oder X⁵ ein Kohlenstoffatom ist, das an die durch die Formel (2) dargestellte Gruppe gebunden ist, worin Y² ein an L gebundenes Kohlenstoffatom ist, und L eine Einfachbindung ist, und Z ein Sauerstoffatom ist, dann ist R^{Y} in Y⁷ eine andere Gruppe als eine Pyrenylgruppe;
wenn X⁴ oder X⁵ ein Kohlenstoffatom ist, das an die durch die Formel (2) dargestellte Gruppe gebunden ist, worin Y⁷ ein an L gebundenes Kohlenstoffatom ist, und L eine Einfachbindung ist, und Z ein Sauerstoffatom ist, dann ist R^{Y} in Y² eine andere Gruppe als eine Pyrenylgruppe; und
ein Substituent der vorstehenden substituierten oder unsubstituierten Gruppen ist ausgewählt aus der Gruppe bestehend aus einer Arylgruppe, einer Heteroarylgruppe, einer Alkylgruppe, einem Halogenatom, einer Alkylsilylgruppe, einer Arylsilylgruppe und einer Cyanogruppe.

2. Verbindung gemäß Anspruch 1, worin
L ausgewählt ist aus einer Einfachbindung und einer verbindenden Gruppe, worin
die verbindende Gruppe eine substituierte oder unsubstituierte mehrwertige lineare, verzweigte oder cyclische aliphatische Kohlenwasserstoffgruppe mit 1 bis 30 Kohlenstoffatomen, oder eine substituierte oder unsubstituierte mehrwertige Arylgrupe mit 6 bis 40 Ringatomen darstellt.

3. Verbindung gemäß Anspruch 1 oder 2, worin
X¹ oder X⁸ in der Formel (1) ein an eine durch die Formel (2) dargestellte Gruppe gebundenes Kohlenstoffatom ist.

4. Verbindung gemäß Anspruch 1 oder 2, worin
X¹ und X⁸ in der Formel (1) jeweils an die durch die Formel (2) dargestellte Gruppe gebundene Kohlenstoffatome sind.

5. Verbindung gemäß Anspruch 1 oder 2, worin
X³ und X⁶ in der Formel (1) jeweils an die durch die Formel (2) dargestellte Gruppe gebundene Kohlenstoffatome sind.

6. Verbindung gemäß Anspruch 1 oder 2, worin
X² oder X⁷ in der Formel (1) jeweils ein an die durch die Formel (2) dargestellte Gruppe gebundenes Kohlenstoffatom ist.

7. Verbindung gemäß Anspruch 1 oder 2, worin
X³ oder X⁶ in der Formel (1) jeweils ein an die durch die Formel (2) dargestellte Gruppe gebundenes Kohlenstoffatom ist.

8. Verbindung gemäß Anspruch 1 oder 2, worin
X⁴ oder X⁵ in der Formel (1) jeweils ein an die durch die Formel (2) dargestellte Gruppe gebundenes Kohlenstoffatom ist.

9. Verbindung gemäß Anspruch 1 oder 2, worin
X¹ und X⁷ in der Formel (1) jeweils an die durch die Formel (2) dargestellte Gruppe gebundene Kohlenstoffatome sind.

10. Verbindung gemäß Anspruch 1 oder 2, worin
X² und X⁷ in der Formel (1) jeweils an die durch die Formel (2) dargestellte Gruppe gebundene Kohlenstoffatome sind.

11. Verbindung gemäß Anspruch 1 oder 2, worin
X¹, X², X⁷ und X⁸ in der Formel (1) jeweils an die durch die Formel (2) dargestellte Gruppe gebundene Kohlenstoffatome sind.

12. Verbindung gemäß Anspruch 1 oder 2, worin
X¹, X³, X⁶ und X⁸ in der Formel (1) jeweils an die durch die Formel (2) dargestellte Gruppe gebundene Kohlenstoffatome sind.

13. Verbindung gemäß einem der Ansprüche 1 bis 12, worin die durch die Formel (1) dargestellte Verbindung durch die nachstehende Formel (1-1) dargestellt ist 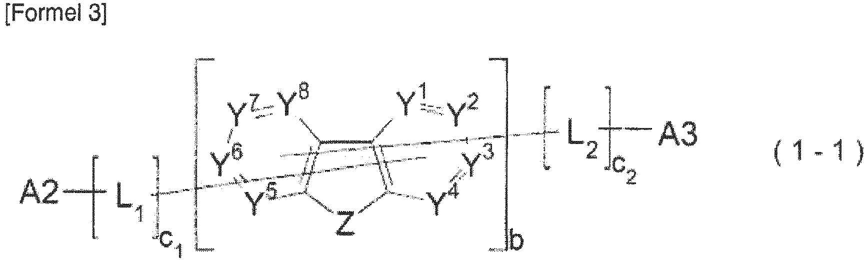
worin: A2 ujd A3 jeweils unabhängig voneinander eine durch die Formel (1) dargestellte Struktur darstellen;
c₁ ist eine ganze Zahl von 1 bis 8 und c₂ ist eine ganze Zahl von 1 bis 8; L₁ und L₂ stellen jeweils unabhängig voneinander dasselbe dar wie L in der Formel (2); und X¹ bis X⁸ in A2 und A3, Y¹ bis Y⁸, Z und b stellen jeweils das gleiche dar wie X¹ bis X⁸, Y¹ bis Y⁸, Z und b in den Formeln (1) und (2).

14. Verbindung gemäß einem der Ansprüche 1 bis 13, worin
die übrigen X¹ bis X⁸ in der Formel (1) CR^{x} darstellen, mit Ausnahme des Kohlenstoffatoms, das an die durch die Formel (2) dargestellte Gruppe gebunden ist.

15. Verbindung gemäß einem der Ansprüche 1 bis 14, worin
Z in der Formel (2) ein Sauerstoffatom ist.

16. Verbindung gemäß einem der Ansprüche 1 bis 15, worin
c in der Formel (2) eine ganze Zahl von 1 bis 5 ist.

17. Verwendung der Verbindung gemäß einem der Ansprüche 1 bis 16, in einer Schicht einer organischen Verbindung einer organischen Elektrolumineszenzvorrichtung, umfassend eine Anode, eine Kathode und die Schicht der organischen Verbindung angeordnet zwischen der Anode und der Kathode.

18. Verwendung der Verbindung gemäß einem der Ansprüche 1 bis 16, in einer Elektronen transportierenden Schicht einer organischen Elektrolumineszenzvorrichtung, umfassend eine Anode, eine Kathode, eine emittierende Schicht und die Elektronen transportierende Schicht angeordnet zwischen der Anode und der Kathode.

19. Ein Material für eine Elektrolumineszenzvorrichtung, umfassend: die Verbindung gemäßen einem der Ansprüche 1 bis 16.

20. Eine organische Elektrolumineszenzvorrichtung umfassend:
eine Anode;
eine Kathode gegenüber der Anode; und
eine Schicht einer organischen Verbindung angeordnet zwischen der Anode und der Kathode,
wobei die Schicht der organischen Verbindung eine emittierende Schicht und eine Elektronen transportierende Schicht, die auf der Seite der emittierenden Schicht nahe der Kathode vorgesehen ist, umfasst,
wobei die Elektronen transportierende Schicht eine durch die nachstehende Formel (10) dargestellte Verbindung umfasst 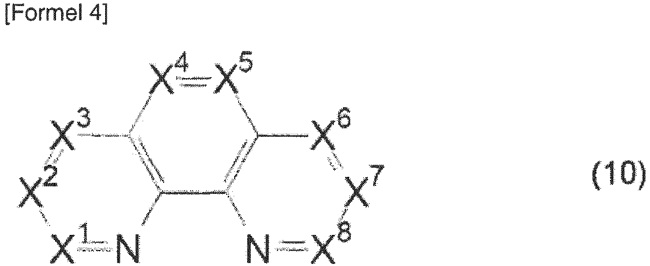
worin: X¹ bis X⁸ jeweils unabhängig voneinander darstellen
ein Kohlenstoffatom, das an eine durch die nachstehende Formel (20) dargestellte Gruppe gebunden ist,
CR^{x}, oder
ein Stickstoffatom;
mindestens zwei von X¹ bis X⁸ sind Kohlenstoffatome, die jeweils an die durch die Formel (20) dargestellte Gruppe gebunden sind;
R^{x} ist jeweils unabhängig voneinander ausgewählt aus der Gruppe bestehend aus einem Wasserstoffatom, einem Halogenatom, einer Cyanogruppe, einer Nitrogruppe, einer substituierten oder unsubstituierten Hydroxylgruppe, einer substituierten oder unsubstituierten Carboxylgruppe, einer substituierten oder unsubstituierten Sulfonylgruppe, einer substituierten oder unsubstituierten Borylgruppe, einer substituierten oder unsubstituierten Phosphinogruppe, einer substituierten oder unsubstituierten Mercaptogruppe, einer substituierten oder unsubstituierten Acylgruppe, einer substituierten oder unsubstituierten Aminogruppe, einer substituierten oder unsubstituierten Silylgruppe, einer substituierten oder unsubstituierten Alkylgruppe mit 1 bis 30 Kohlenstoffatomen, einer substituierten oder unsubstituierten Alkenylgruppe mit 2 bis 30 Kohlenstoffatomen, einer substituierten oder unsubstituierten Alkinylgruppe mit 2 bis 30 Kohlenstoffatomen, einer substituierten oder unsubstituierten Aralkylgruppe mit 6 bis 30 Kohlenstoffatomen, einer substituierten oder unsubstituierten Arylgruppe mit 6 bis 40 Ringkohlenstoffatomen und einer substituierten oder unsubstituierten Heteroarylgruppe mit 5 bis 40 Ringatomen; und
R^{x} von benachbarten CR^{x} in X¹ bis X⁸ sind gegebenenfalls miteinander verbunden, unter Bildung einer cyclischen Struktur, oder sind nicht gegebenenfalls verbunden, 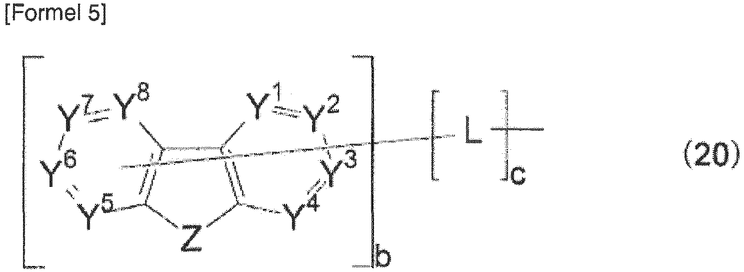
worin: b eine ganze Zahl von 1 bis 5 ist;
c eine ganze Zahl von 1 bis 8 ist;
Z stellt ein Sauerstoffatom oder ein Schwefelatom dar;
wenn b 2 bis 5 ist, sind mehrere Z gleich oder verschieden;
L ist ausgewählt aus einer Einfachbindung und einer verbindenden Gruppe, worin
die verbindende Gruppe eine substituierte oder unsubstituierte mehrwertige lineare, verzweigte oder cyclische aliphatische Kohlenwasserstoffgruppe mit 1 bis 30 Kohlenstoffatomen, eine substituierte oder unsubstituierte mehrwertige Arylgruppe mit 6 bis 40 Ringkohlenstoffatomen oder eine substituierte oder unsubstituierte mehrwertige Heteroarylgruppe mit 5 bis 40 Ringatome darstellt;
die mehrwertige Heteroarylgruppe mit 5 bis 40 Ringatomen in L der Formel (20) umfasst eine substituierte oder unsubstituierte mehrwertige Gruppe, die von einem Phenanthrolinring der Formel (10) abgeleitet ist;
wenn c 2 bis 8 ist, sind mehrere L gleich oder verschieden;
Y¹ bis Y⁸ stellen jeweils unabhängig voneinander ein Stickstoffatom, CR^{Y} oder ein an L gebundenes Kohlenstoffatom dar;
R^{Y} stellt dasselbe wie R^{X} in der Formel (10) dar;
die Heteroarylgruppe mit 5 bis 40 Ringatomen in R^{Y} umfasst eine substituierte oder unsubstituierte Phenanthrolylgruppe, die von dem Phenanthrolinring der Formel (10) abgeleitet ist;
benachbarte R^{Y} sind gegebenenfalls miteinander verbunden, unter Bildung einer cyclischen Struktur, oder sind nicht gegebenenfalls verbunden.

21. Organische Elektrolumineszenzvorrichtung gemäß Anspruch 20, worin:
L ausgewählt ist aus einer Einfachbindung und einer verbindenden Gruppe, worin die verbindende Gruppe eine substituierte oder unsubstituierte mehrwertige lineare,
verzweigte oder cyclische aliphatische Kohlenwasserstoffgruppe mit 1 bis 30 Kohlenstoffatomen, oder eine substituierte oder unsubstituierte mehrwertige Arylgrupe mit 6 bis 40 Ringatomen darstellt.

22. Organische Elektrolumineszenzvorrichtung gemäß Anspruch 20 oder 21, worin
X¹ oder X⁸ in der Formel (10) ein an eine durch die Formel (20) dargestellte Gruppe gebundenes Kohlenstoffatom ist.

23. Organische Elektrolumineszenzvorrichtung gemäß Anspruch 20 oder 21, worin
X¹ und X⁸ in der Formel (10) jeweils an die durch die Formel (20) dargestellte Gruppe gebundene Kohlenstoffatome sind.

24. Organische Elektrolumineszenzvorrichtung gemäß Anspruch 20 oder 21, worin
X³ und X⁶ in der Formel (10) jeweils an die durch die Formel (20) dargestellte Gruppe gebundene Kohlenstoffatome sind.

25. Organische Elektrolumineszenzvorrichtung gemäß Anspruch 20 oder 21, worin
X² oder X⁷ in der Formel (10) jeweils ein an die durch die Formel (20) dargestellte Gruppe gebundenes Kohlenstoffatom ist.

26. Organische Elektrolumineszenzvorrichtung gemäß Anspruch 20 oder 21, worin
X³ oder X⁶ in der Formel (10) jeweils ein an die durch die Formel (20) dargestellte Gruppe gebundenes Kohlenstoffatom ist.

27. Organische Elektrolumineszenzvorrichtung gemäß Anspruch 20 oder 21, worin
X⁴ oder X⁵ in der Formel (10) jeweils ein an die durch die Formel (20) dargestellte Gruppe gebundenes Kohlenstoffatom ist.

28. Organische Elektrolumineszenzvorrichtung gemäß Anspruch 20 oder 21, worin
X¹ und X⁷ in der Formel (10) jeweils an die durch die Formel (20) dargestellte Gruppe gebundene Kohlenstoffatome sind.

29. Organische Elektrolumineszenzvorrichtung gemäß Anspruch 20 oder 21, worin
X² und X⁷ in der Formel (10) jeweils an die durch die Formel (20) dargestellte Gruppe gebundene Kohlenstoffatome sind.

30. Organische Elektrolumineszenzvorrichtung gemäß Anspruch 20 oder 21, worin
X¹, X², X⁷ und X⁸ in der Formel (10) jeweils an die durch die Formel (20) dargestellte Gruppe gebundene Kohlenstoffatome sind.

31. Organische Elektrolumineszenzvorrichtung gemäß Anspruch 20 oder 21, worin
X¹, X³, X⁶ und X⁸ in der Formel (10) jeweils an die durch die Formel (20) dargestellte Gruppe gebundene Kohlenstoffatome sind.

32. Organische Elektrolumineszenzvorrichtung gemäß einem der Ansprüche 20 bis 31, worin
die durch die Formel (10) dargestellte Verbindung durch die nachstehende Formel (10-1) dargestellt ist 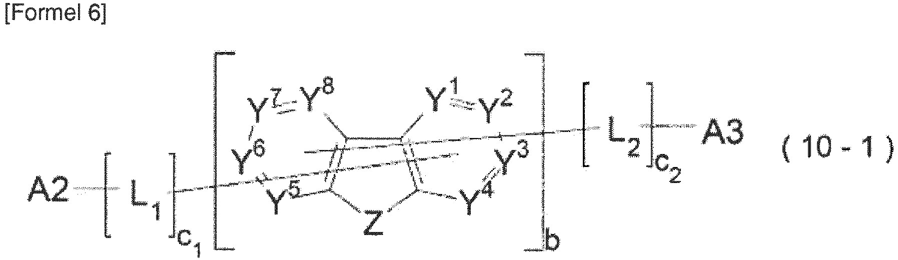
worin: A2 ujd A3 jeweils unabhängig voneinander eine durch die Formel (10) dargestellte Struktur darstellen;
c₁ ist eine ganze Zahl von 1 bis 8 und c₂ ist eine ganze Zahl von 1 bis 8;
L₁ und L₂ stellen jeweils unabhängig voneinander dasselbe dar wie L in der Formel (20); und X¹ bis X⁸ in A2 und A3, Y¹ bis Y⁸, Z und b stellen jeweils das gleiche dar wie X¹ bis X⁸, Y¹ bis Y⁸, Z und b in den Formein (i0) und (20).

33. Organische Elektrolumineszenzvorrichtung gemäß einem der Ansprüche 20 bis 32, worin
die übrigen X¹ bis X⁸ in der Formel (10) CR^{x} darstellen, mit Ausnahme des Kohlenstoffatoms, das an die durch die Formel (20) dargestellte Gruppe gebunden ist.

34. Organische Elektrolumineszenzvorrichtung gemäß einem der Ansprüche 20 bis 33, worin
Z in der Formel (20) ein Sauerstoffatom ist.

35. Organische Elektrolumineszenzvorrichtung gemäß einem der Ansprüche 20 bis 34, welche weiterhin zwischen der Elektronen transportierenden Schicht und der emittierenden Schicht eine Sperrschicht umfasst.

36. Organische Elektrolumineszenzvorrichtung gemäß einem der Ansprüche 20 bis 34, worin
die Elektronen transportierende Schicht angrenzend zu der emittierenden Schicht angeordnet ist.

37. Organische Elektrolumineszenzvorrichtung gemäß einem der Ansprüche 20 bis 36, worin
die Elektronen transportierende Schicht mindestens eines von einem Elektronenspendenden Dotanden und einem organischen Metallkomplex umfasst.

38. Elektronische Vorrichtung, umfassend die organische Elektrolumineszenzvorrichtung gemäß einem der Ansprüche 20 bis 37.

## Revendications

1. Composé représenté par la formule (1) ci-dessous, 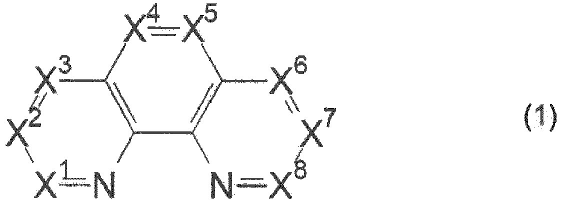
où : X¹ à X⁸ représentent chacun indépendamment
un atome de carbone devant se lier à un groupe représenté par la formule (2) ci-dessous,
CR^{X},
ou un atome d'azote ;
au moins deux de X¹ à X⁸ représentent des atomes de carbone devant se lier chacun au groupe représenté par la formule (2) ;
R^{X} est indépendamment choisi dans le groupe constitué par un atome d'hydrogène, un atome d'halogène, un groupe cyano, un groupe nitro, un groupe hydroxyle substitué ou non substitué, un groupe carboxyle substitué ou non substitué, un groupe sulfonyle substitué ou non substitué, un groupe boryle substitué ou non substitué, un groupe phosphino substitué ou non substitué, un groupe mercapto substitué ou non substitué, un groupe acyle substitué ou non substitué, un groupe amino substitué ou non substitué, un groupe silyle substitué ou non substitué, un groupe alkyle substitué ou non substitué ayant 1 à 30 atome(s) de carbone, un groupe alcényle substitué ou non substitué ayant 2 à 30 atomes de carbone, un groupe alcynyle substitué ou non substitué ayant 2 à 30 atomes de carbone, un groupe aralkyle substitué ou non substitué ayant 6 à 30 atomes de carbone, un groupe aryle substitué ou non substitué ayant 6 à 40 atomes de carbone de noyau, et un groupe hétéroaryle substitué ou non substitué ayant 5 à 40 atomes de noyau ; et
R^{X} de groupes adjacents de CR^{X} dans X¹ à X⁸ sont facultativement liés les uns aux autres pour former une structure cyclique ou ne sont pas facultativement liés, 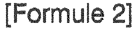 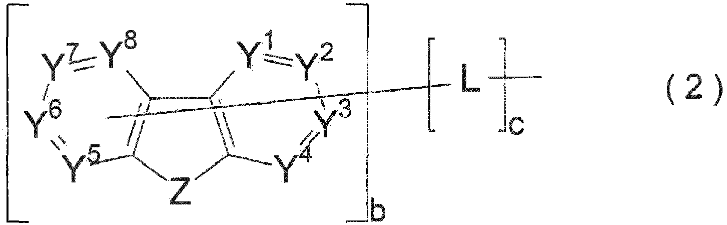
où : b représente un nombre entier compris entre 1 et 5 ; c représente un nombre entier compris entre 1 et 8 ;
Z représente un atome d'oxygène ou un atome de soufre ;
lorsque b est compris entre 2 et 5, une pluralité de Z sont mutuellement identiques ou différents ;
L est choisi parmi une simple liaison et un groupe de liaison, où
le groupe de liaison représente un groupe hydrocarboné aliphatique linéaire, ramifié ou cyclique polyvalent substitué ou non substitué ayant 1 à 30 atome(s) de carbone, un groupe aryle polyvalent substitué ou non substitué ayant 6 à 40 atomes de carbone de noyau, ou un groupe hétéroaryle polyvalent substitué ou non substitué ayant 5 à 40 atomes de noyau ;
le groupe hétéroaryle polyvalent ayant 5 à 40 atomes de noyau dans L de la formule (2) comprend un groupe polyvalent substitué ou non substitué dérivé d'un noyau phénanthroline représenté par la formule (1) ;
lorsque c est compris entre 2 et 8, une pluralité de L sont mutuellement identiques ou différents ;
Y¹ à Y⁸ représentent chacun indépendamment un atome d'azote, CR^{Y} ou un atome de carbone devant se lier à L ;
R^{Y} représente le même élément que R^{X} dans la formule (1) ;
le groupe hétéroaryle ayant 5 à 40 atomes de noyau dans R^{Y} comprend un groupe phénanthrolyle substitué ou non substitué dérivé du noyau phénanthroline représenté par la formule (1) ;
des groupes adjacents de R^{Y} sont facultativement liés les uns aux autres pour former une structure cyclique ou ne sont pas facultativement liés ;
lorsque X¹ ou X⁸ représente un atome de carbone devant se lier au groupe représenté par la formule (2) dans laquelle b vaut 1, Z représente un atome d'oxygène, Y⁴ ou Y⁵ représente un atome de carbone devant se lier à L, et c vaut 2, l'un de deux L, qui est proche du noyau phénanthroline représenté par la formule (1), représente un groupe divalent autre qu'un groupe anthracène ;
lorsque deux de X¹ à X⁸ représentent des atomes de carbone devant se lier chacun au groupe représenté par la formule (2) dans laquelle b et c valent 1, Z représente un atome de soufre, Y⁴ ou Y⁵ représente un atome de carbone devant se lier à L, et L représente un groupe p-phénylène, L est lié à l'un de X¹, X², X⁴, X⁵, X⁷, et X⁸ ;
lorsque X¹ ou X⁸ représente un atome de carbone devant se lier au groupe représenté par la formule (2) dans laquelle b et c valent 1, Z représente un atome d'oxygène ou un atome de soufre, Y³ représente un atome de carbone devant se lier à L, et L représente un groupe p-phénylène, R^{Y} dans Y⁴ représente un groupe autre qu'un groupe phényle ;
lorsque X¹ ou X⁸ représente un atome de carbone devant se lier au groupe représenté par la formule (2) dans laquelle b et c valent 1, Z représente un atome d'oxygène ou un atome de soufre, Y⁶ représente un atome de carbone devant se lier à L, et L représente un groupe p-phénylène, R^{Y} dans Y⁵ représente un groupe autre qu'un groupe phényle ;
lorsque X⁴ ou X⁵ représente un atome de carbone devant se lier au groupe représenté par la formule (2) dans laquelle Y² représente un atome de carbone devant se lier à L, et L représente une simple liaison et Z représente un atome d'oxygène, R^{Y} dans Y⁷ représente un groupe autre qu'un groupe pyrényle ;
lorsque X⁴ ou X⁵ représente un atome de carbone devant se lier au groupe représenté par la formule (2) dans laquelle Y⁷ représente un atome de carbone devant se lier à L, et L représente une simple liaison et Z représente un atome d'oxygène, R^{Y} dans Y² représente un groupe autre qu'un groupe pyrényle ; et
un substituant des groupes substitués ou non substitués ci-dessus est choisi dans le groupe constitué par un groupe aryle, un groupe hétéroaryle, un groupe alkyle, un atome d'halogène, un groupe alkylsilyle, un groupe arylsilyle et un groupe cyano.

2. Composé selon la revendication 1, dans lequel
L est choisi parmi une simple liaison et un groupe de liaison, où
le groupe de liaison représente un groupe hydrocarboné aliphatique linéaire, ramifié ou cyclique polyvalent substitué ou non substitué ayant 1 à 30 atome(s) de carbone, ou un groupe aryle polyvalent substitué ou non substitué ayant 6 à 40 atomes de carbone de noyau.

3. Composé selon la revendication 1 ou 2, dans lequel
X¹ ou X⁸ dans la formule (1) représente un atome de carbone devant se lier au groupe représenté par la formule (2).

4. Composé selon la revendication 1 ou 2, dans lequel
X¹ et X⁸ dans la formule (1) représentent des atomes de carbone devant se lier chacun au groupe représenté par la formule (2).

5. Composé selon la revendication 1 ou 2, dans lequel
X³ et X⁶ dans la formule (1) représentent des atomes de carbone devant se lier chacun au groupe représenté par la formule (2).

6. Composé selon la revendication 1 ou 2, dans lequel
X² ou X⁷ dans la formule (1) représente un atome de carbone devant se lier au groupe représenté par la formule (2).

7. Composé selon la revendication 1 ou 2, dans lequel
X³ ou X⁶ dans la formule (1) représente un atome de carbone devant se lier au groupe représenté par la formule (2).

8. Composé selon la revendication 1 ou 2, dans lequel
X⁴ ou X⁵ dans la formule (1) représente un atome de carbone devant se lier au groupe représenté par la formule (2).

9. Composé selon la revendication 1 ou 2, dans lequel
X¹ et X⁷ dans la formule (1) représentent des atomes de carbone devant se lier chacun au groupe représenté par la formule (2).

10. Composé selon la revendication 1 ou 2, dans lequel
X² et X⁷ dans la formule (1) représentent des atomes de carbone devant se lier chacun au groupe représenté par la formule (2).

11. Composé selon la revendication 1 ou 2, dans lequel
X¹, X², X⁷ et X⁸ dans la formule (1) représentent des atomes de carbone devant se lier chacun au groupe représenté par la formule (2).

12. Composé selon la revendication 1 ou 2, dans lequel
X¹, X³, X⁶ et X⁸ dans la formule (1) représentent des atomes de carbone devant se lier chacun au groupe représenté par la formule (2).

13. Composé selon l'une quelconque des revendications 1 à 12, dans lequel le composé représenté par la formule (1) est représenté par une formule (1-1) ci-dessous, 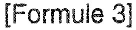 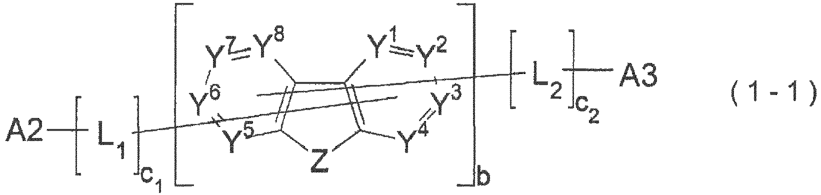
où : A2 et A3 représentent chacun indépendamment une structure représentée par la formule (1) ;
c₁ représente un nombre entier compris entre 1 et 8 et c₂ représente un nombre entier compris entre 1 et 8 ; L₁ et L₂ représentent chacun indépendamment le même élément que L dans la formule (2) ; et X¹ à X⁸ dans A2 et A3, Y¹ à Y⁸, Z et b représentent respectivement les mêmes éléments que X¹ à X⁸, Y¹ à Y⁸, Z et b dans les formules (1) et (2).

14. Composé selon l'une quelconque des revendications 1 à 13, dans lequel
le reste de X¹ à X⁸ dans la formule (1), à l'exception de l'atome de carbone devant se lier au groupe représenté par la formule (2), représentent CR^{X}.

15. Composé selon l'une quelconque des revendications 1 à 14, dans lequel
Z dans la formule (2) représente un atome d'oxygène.

16. Composé selon l'une quelconque des revendications 1 à 15, dans lequel
c dans la formule (2) représente un nombre entier compris entre 1 et 5.

17. Utilisation du composé selon l'une quelconque des revendications 1 à 16 dans une couche de composé organique d'un dispositif électroluminescent organique comprenant une anode, une cathode et la couche de composé organique disposée entre l'anode et la cathode.

18. Utilisation du composé selon l'une quelconque des revendications 1 à 16 dans une couche de transport d'électrons d'un dispositif électroluminescent organique comprenant une anode, une cathode et une couche émettrice et la couche de transport d'électrons entre l'anode et la cathode.

19. Matériau pour un dispositif électroluminescent organique, comprenant : le composé selon l'une quelconque des revendications 1 à 16.

20. Dispositif électroluminescent organique comprenant :
une anode ;
une cathode opposée à l'anode ; et
une couche de composé organique disposée entre l'anode et la cathode,
la couche de composé organique comprenant une couche émettrice et une couche de transport d'électrons prévue sur un côté de la couche émettrice près de la cathode,
la couche de transport d'électrons comprenant un composé représenté par la formule (10) ci-dessous, 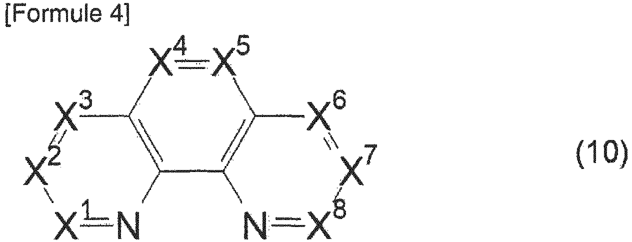
où : X¹ à X⁸ représentent chacun indépendamment
un atome de carbone devant se lier à un groupe représenté par la formule (20) ci-dessous,
CR^{X}, ou
un atome d'azote ;
au moins deux de X¹ à X⁸ représentent des atomes de carbone devant se lier chacun au groupe représenté par la formule (20) ;
R^{X} est indépendamment choisi dans le groupe constitué par un atome d'hydrogène, un atome d'halogène, un groupe cyano, un groupe nitro, un groupe hydroxyle substitué ou non substitué, un groupe carboxyle substitué ou non substitué, un groupe sulfonyle substitué ou non substitué, un groupe boryle substitué ou non substitué, un groupe phosphino substitué ou non substitué, un groupe mercapto substitué ou non substitué, un groupe acyle substitué ou non substitué, un groupe amino substitué ou non substitué, un groupe silyle substitué ou non substitué, un groupe alkyle substitué ou non substitué ayant 1 à 30 atome(s) de carbone, un groupe alcényle substitué ou non substitué ayant 2 à 30 atomes de carbone, un groupe alcynyle substitué ou non substitué ayant 2 à 30 atomes de carbone, un groupe aralkyle substitué ou non substitué ayant 6 à 30 atomes de carbone, un groupe aryle substitué ou non substitué ayant 6 à 40 atomes de carbone de noyau, et un groupe hétéroaryle substitué ou non substitué ayant 5 à 40 atomes de noyau ; et
R^{X} de groupes adjacents de CR^{X} dans X¹ à X⁸ sont facultativement liés les uns aux autres pour former une structure cyclique ou ne sont pas facultativement liés, 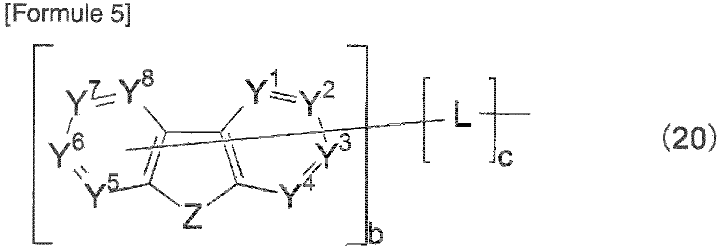
où : b représente un nombre entier compris entre 1 et 5 ;
c représente un nombre entier compris entre 1 et 8 ;
Z représente un atome d'oxygène ou un atome de soufre ;
lorsque b est compris entre 2 et 5, une pluralité de Z sont mutuellement identiques ou différents ;
L est choisi parmi une simple liaison et un groupe de liaison, où
le groupe de liaison représente un groupe hydrocarboné aliphatique linéaire, ramifié ou cyclique polyvalent substitué ou non substitué ayant 1 à 30 atome(s) de carbone, un groupe aryle polyvalent substitué ou non substitué ayant 6 à 40 atomes de carbone de noyau, ou un groupe hétéroaryle polyvalent substitué ou non substitué ayant 5 à 40 atomes de noyau ;
le groupe hétéroaryle polyvalent ayant 5 à 40 atomes de noyau dans L de la formule (20) comprend un groupe polyvalent substitué ou non substitué dérivé d'un noyau phénanthroline représenté par la formule (10) ;
lorsque c est compris entre 2 et 8, une pluralité de L sont mutuellement identiques ou différents ;
Y¹ à Y⁸ représentent chacun indépendamment un atome d'azote, CR^{Y} ou un atome de carbone devant se lier à L ;
R^{Y} représente le même élément que R^{X} dans la formule (10) ;
le groupe hétéroaryle ayant 5 à 40 atomes de noyau dans R^{Y} comprend un groupe phénanthrolyle substitué ou non substitué dérivé du noyau phénanthroline représenté par la formule (10) ; et
des groupes adjacents de R^{Y} sont facultativement liés les uns aux autres pour former une structure cyclique ou ne sont pas facultativement liés.

21. Dispositif électroluminescent organique selon la revendication 20, dans lequel
L est choisi parmi une simple liaison et un groupe de liaison, où
le groupe de liaison représente un groupe hydrocarboné aliphatique linéaire, ramifié ou cyclique polyvalent substitué ou non substitué ayant 1 à 30 atome(s) de carbone, ou un groupe aryle polyvalent substitué ou non substitué ayant 6 à 40 atomes de carbone de noyau.

22. Dispositif électroluminescent organique selon la revendication 20 ou 21, dans lequel
X¹ ou X⁸ dans la formule (10) représente un atome de carbone devant se lier au groupe représenté par la formule (20).

23. Dispositif électroluminescent organique selon la revendication 20 ou 21, dans lequel
X¹ et X⁸ dans la formule (10) représentent des atomes de carbone devant se lier chacun au groupe représenté par la formule (20).

24. Dispositif électroluminescent organique selon la revendication 20 ou 21, dans lequel
X³ et X⁶ dans la formule (10) représentent des atomes de carbone devant se lier chacun au groupe représenté par la formule (20).

25. Dispositif électroluminescent organique selon la revendication 20 ou 21, dans lequel
X² ou X⁷ dans la formule (10) représente un atome de carbone devant se lier au groupe représenté par la formule (20).

26. Dispositif électroluminescent organique selon la revendication 20 ou 21, dans lequel
X³ ou X⁶ dans la formule (10) représente un atome de carbone devant se lier au groupe représenté par la formule (20).

27. Dispositif électroluminescent organique selon la revendication 20 ou 21, dans lequel
X⁴ ou X⁵ dans la formule (10) représente un atome de carbone devant se lier au groupe représenté par la formule (20).

28. Dispositif électroluminescent organique selon la revendication 20 ou 21, dans lequel
X¹ et X⁷ dans la formule (10) représentent des atomes de carbone devant se lier chacun au groupe représenté par la formule (20).

29. Dispositif électroluminescent organique selon la revendication 20 ou 21, dans lequel
X² et X⁷ dans la formule (10) représentent des atomes de carbone devant se lier chacun au groupe représenté par la formule (20).

30. Dispositif électroluminescent organique selon la revendication 20 ou 21, dans lequel
X¹, X², X⁷ et X⁸ dans la formule (10) représentent des atomes de carbone devant se lier chacun au groupe représenté par la formule (20).

31. Dispositif électroluminescent organique selon la revendication 20 ou 21, dans lequel
X¹, X³, X⁶ et X⁸ dans la formule (10) représentent des atomes de carbone devant se lier chacun au groupe représenté par la formule (20).

32. Dispositif électroluminescent organique selon l'une quelconque des revendications 20 à 31, dans lequel
le composé représenté par la formule (10) est représenté par une formule (10-1) ci-dessous, 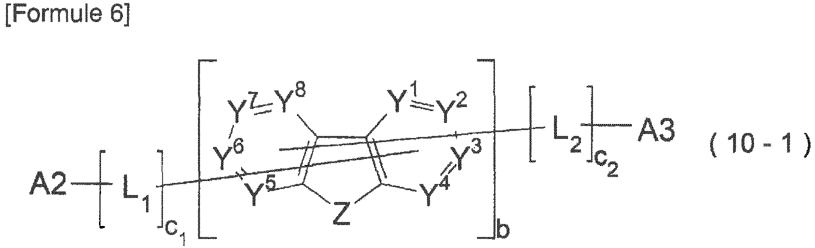
où : A2 et A3 représentent chacun indépendamment une structure représentée par la formule (10) ;
c₁ représente un nombre entier compris entre 1 et 8 et c₂ représente un nombre entier compris entre 1 et 8 ;
L₁ et L₂ représentent chacun indépendamment le même élément que L dans la formule (20) ; et
X¹ à X⁸ dans A2 et A3, Y¹ à Y⁸, Z et b représentent respectivement les mêmes éléments que X¹ à X⁸, Y¹ à Y⁸, Z et b dans les formules (10) et (20).

33. Dispositif électroluminescent organique selon l'une quelconque des revendications 20 à 32, dans lequel
le reste de X¹ à X⁸ dans la formule (10), à l'exception de l'atome de carbone devant se lier au groupe représenté par la formule (20), représentent CR^{X}.

34. Dispositif électroluminescent organique selon l'une quelconque des revendications 20 à 33, dans lequel
Z dans la formule (20) représente un atome d'oxygène.

35. Dispositif électroluminescent organique selon l'une quelconque des revendications 20 à 34, comprenant en outre une couche de blocage entre la couche de transport d'électrons et la couche émettrice.

36. Dispositif électroluminescent organique selon l'une quelconque des revendications 20 à 34, dans lequel
la couche de transport d'électrons est adjacente à la couche émettrice.

37. Dispositif électroluminescent organique selon l'une quelconque des revendications 20 à 36, dans lequel
la couche de transport d'électrons comprend au moins l'un parmi un dopant donneur d'électrons et un complexe métallique organique.

38. Dispositif électronique comprenant le dispositif électroluminescent organique selon l'une quelconque des revendications 20 à 37.
